(19) Europäisches Patentamt
European Patent Office
Office européen des brevets

(11) EP 3 353 163 B1

(12) EUROPEAN PATENT SPECIFICATION

(45) Date of publication and mention of the grant of the patent:
**05.02.2020 Bulletin 2020/06**

(21) Application number: **16770283.6**

(22) Date of filing: **23.09.2016**

(51) Int Cl.:
***C07D 401/12*** *(2006.01)* ***A61K 31/4418*** *(2006.01)*
***A61P 25/16*** *(2006.01)* ***A61P 25/28*** *(2006.01)*

(86) International application number:
**PCT/EP2016/072710**

(87) International publication number:
**WO 2017/050978 (30.03.2017 Gazette 2017/13)**

(54) **2,3,4,5-TETRAHYDROPYRIDIN-6-AMINE DERIVATIVES**

2,3,4,5-TETRAHYDROPYRIDIN-6-AMIN-DERIVATE

DÉRIVÉS DE 2,3,4,5-TETRAHYDROPYRIDIN-6-AMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.09.2015 EP 15186394**
**30.05.2016 EP 16172005**

(43) Date of publication of application:
**01.08.2018 Bulletin 2018/31**

(73) Proprietor: **Janssen Pharmaceutica, N.V.**
**2340 Beerse (BE)**

(72) Inventors:
- **ROMBOUTS, Frederik, Jan, Rita**
**2340 Beerse (BE)**
- **GIJSEN, Henricus, Jacobus, Maria**
**2340 Beerse (BE)**
- **VAN BRANDT, Sven, Franciscus, Anna**
**2340 Beerse (BE)**
- **TRABANCO-SUÁREZ, Andrés, Avelino**
**28042 Madrid (ES)**

(74) Representative: **Garcia Prieto, Maria**
**Johnson & Johnson**
**Patent Law Department**
**Turnhoutseweg 30**
**2340 Beerse (BE)**

(56) References cited:
**WO-A1-2011/009943**
**WO-A1-2014/059185**
**WO-A1-2015/124576**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 353 163 B1

## Description

FIELD OF THE INVENTION

The present invention relates to 2,3,4,5-tetrahydropyridin-6-amine compound inhibitors of beta-secretase having the structure shown in Formula (I)

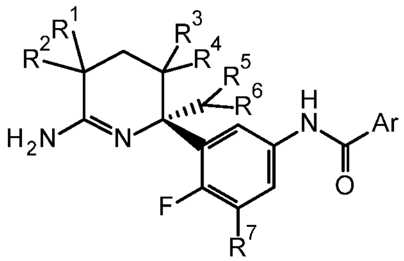

(I)

wherein the radicals are as defined in the specification. The invention is also directed to pharmaceutical compositions comprising such compounds, to processes for preparing such compounds and compositions, and to the use of such compounds and compositions for the prevention and treatment of disorders in which beta-secretase is involved, such as Alzheimer's disease (AD), mild cognitive impairment, senility, dementia, dementia with Lewy bodies, Down's syndrome, dementia associated with stroke, dementia associated with Parkinson's disease, and dementia associated with beta-amyloid.

BACKGROUND OF THE INVENTION

Alzheimer's Disease (AD) is a neurodegenerative disease associated with aging. AD patients suffer from cognition deficits and memory loss as well as behavioral problems such as anxiety. Over 90% of those afflicted with AD have a sporadic form of the disorder while less than 10% of the cases are familial or hereditary. In the United States, about one in ten people at age 65 have AD while at age 85, one out of every two individuals are afflicted by AD. The average life expectancy from the initial diagnosis is 7-10 years, and AD patients require extensive care either in an assisted living facility or by family members. With the increasing number of elderly in the population, AD is a growing medical concern. Currently available therapies for AD merely treat the symptoms of the disease and include acetylcholinesterase inhibitors to improve cognitive properties as well as anxiolytics and antipsychotics to control the behavioral problems associated with this ailment.

The hallmark pathological features in the brain of AD patients are neurofibrillary tangles which are generated by hyperphosphorylation of tau protein and amyloid plaques which form by aggregation of beta-amyloid 1-42 (Abeta 1-42) peptide. Abeta 1-42 forms oligomers and then fibrils, and ultimately amyloid plaques. The oligomers and fibrils are believed to be especially neurotoxic and may cause most of the neurological damage associated with AD. Agents that prevent the formation of Abeta 1-42 have the potential to be disease-modifying agents for the treatment of AD. Abeta 1-42 is generated from the amyloid precursor protein (APP), comprised of 770 amino acids. The N-terminus of Abeta 1-42 is cleaved by beta-secretase (BACE1), and then gamma-secretase cleaves the C-terminal end. In addition to Abeta 1-42, gamma-secretase also liberates Abeta 1-40 which is the predominant cleavage product as well as Abeta 1-38 and Abeta 1-43. These Abeta forms can also aggregate to form oligomers and fibrils. Thus, inhibitors of BACE1 would be expected to prevent the formation of Abeta 1-42 as well as Abeta 1-40, Abeta 1-38 and Abeta 1-43 and would be potential therapeutic agents in the treatment of AD.

WO-2015/124576 (Lundbeck & Co AS H) discloses 2-amino-3,5,5-trifluoro-3,4,5,6-tetrahydropyridine derivatives and their use as BACE inhibitors for the treatment of neurodegenerative disorders. WO-2011/009943 (Novartis AG) discloses dihydrooxazine derivatives having BACE inhibitory properties. WO-2014/059185 (Amgen Inc) discloses discloses dihydrothiazine derivatives as BACE inhibitors.

SUMMARY OF THE INVENTION

The present invention is directed to compounds of Formula (I)

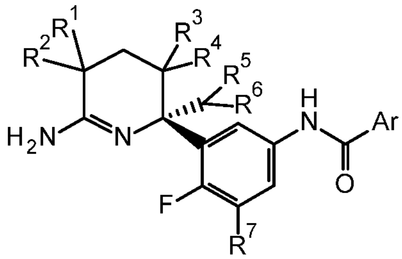

(I)

and the tautomers and the stereoisomeric forms thereof, wherein

$R^1$ is selected from the group consisting of $-C_{1-3}$alkyl, $-C_{1-3}$alkyl-F and fluoro;

$R^2$ is selected from the group consisting of $-SO_2C_{1-3}$alkyl, $-SO_2$cyclopropyl, -CN, $-OC_{1-3}$alkyl, $CF_3$, and $-SO(NCH_3)CH_3$;

Ar is homoaryl or heteroaryl;

wherein homoaryl is phenyl or phenyl substituted with one, two or three substituents each independently selected from the group consisting of halo, cyano, $C_{1-3}$alkyl, cyclopropyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and $HC{\equiv}CCH_2O$;

heteroaryl is selected from the group consisting of pyridyl, pyrimidinyl, pyrazinyl, pyridazinyl, furanyl, thienyl, pyrrolyl, pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents each independently selected from the group consisting of halo, cyano, $C_{1-3}$alkyl, cyclopropyl, $C_{2-3}$alkynyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and $HC{\equiv}CCH_2O$;

$R^3$, $R^4$, $R^5$, and $R^6$ are each independently selected from the group consisting of H, fluoro, methyl and methoxy; and

$R^7$ is hydrogen or fluoro;

and the pharmaceutically acceptable addition salts and the solvates thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Exemplifying the invention are compounds for use in methods of treating a disorder mediated by the beta-secretase enzyme, comprising administering to a subject in need thereof a therapeutically effective amount of any of the compounds or pharmaceutical compositions described above.

Further exemplifying the invention are compounds and compositions for use in methods of inhibiting the beta-secretase enzyme, comprising administering to a subject in need thereof a therapeutically effective amount of any of the compounds or pharmaceutical compositions described above.

An example of the invention is a compound for use in a method of treating a disorder selected from the group consisting of Alzheimer's disease, mild cognitive impairment, senility, dementia, dementia with Lewy bodies, Down's syndrome, dementia associated with stroke, dementia associated with Parkinson's disease, and dementia associated with beta-amyloid, preferably Alzheimer's disease, comprising administering to a subject in need thereof, a therapeutically effective amount of any of the compounds or pharmaceutical compositions described above.

Another example of the invention is any of the compounds described above for use in treating: (a) Alzheimer's Disease, (b) mild cognitive impairment, (c) senility, (d) dementia, (e) dementia with Lewy bodies, (f) Down's syndrome, (g) dementia associated with stroke, (h) dementia associated with Parkinson's disease, or (i) dementia associated with beta-amyloid in a subject in need thereof.

DETAILED DESCRIPTION OF THE INVENTION

The present invention is directed to compounds of formula (I) as defined hereinbefore, and pharmaceutically acceptable addition salts and solvates thereof. The compounds of formula (I) are inhibitors of the beta-secretase enzyme (also known as beta-site cleaving enzyme, BACE, BACE1, Asp2 or memapsin 2, or BACE2), and may be useful in the

treatment of Alzheimer's disease, mild cognitive impairment, senility, dementia, dementia associated with stroke, dementia with Lewy bodies, Down's syndrome, dementia associated with Parkinson's disease, and dementia associated with beta-amyloid, preferably Alzheimer's disease, mild cognitive impairment or dementia, more preferably Alzheimer's disease.

In a particular embodiment, the invention is directed to compounds of Formula (I), wherein
$R^1$ is selected from the group consisting of -$C_{1-3}$alkyl, -$C_{1-3}$alkyl-F and fluoro;
$R^2$ is selected from the group consisting of -$SO_2C_{1-3}$alkyl, -$SO_2$cyclopropyl, -CN, -$OC_{1-3}$alkyl, $CF_3$, and -$SO(NCH_3)CH_3$;
Ar is homoaryl or heteroaryl;
wherein homoaryl is phenyl or phenyl substituted with one, two or three substituents each independently selected from the group consisting of halo, cyano, $C_{1-3}$alkyl, cyclopropyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and HC≡$CCH_2O$;
heteroaryl is selected from the group consisting of pyridyl, pyrimidinyl, pyrazinyl, pyridazinyl, furanyl, thienyl, pyrrolyl, pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents each independently selected from the group consisting of halo, cyano, $C_{1-3}$alkyl, cyclopropyl, $C_{2-3}$alkynyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and HC≡$CCH_2O$;
$R^3$, $R^4$, $R^5$, and $R^6$ are each independently selected from the group consisting of H, fluoro and methyl; and
$R^7$ is hydrogen or fluoro;
and the pharmaceutically acceptable addition salts and the solvates thereof.

In an additional embodiment, the invention is directed to compounds of Formula (I) wherein $R^1$ is selected from the group consisting of $C_{1-3}$alkyl and fluoro, and $R^7$ is hydrogen or fluoro.
In a further particular embodiment, the invention is directed to compounds of Formula (I) wherein $R^1$ is selected from the group consisting of $C_{1-3}$alkyl and fluoro, and $R^7$ is hydrogen.

In a particular embodiment, the invention is directed to compounds of Formula (I) having Formula (I-a)

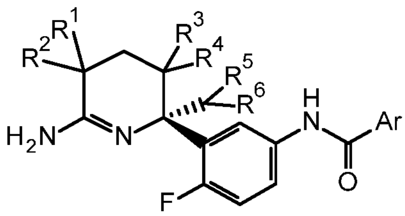

(I-a)

and the tautomers and the stereoisomeric forms thereof, wherein

$R^1$ is $C_{1-2}$alkyl or fluoro;

$R^2$ is -$SO_2C_{1-3}$alkyl, -$SO_2$cyclopropyl, -CN, -$OC_{1-3}$alkyl, $CF_3$, or -$SO(NCH_3)CH_3$;

Ar is homoaryl or heteroaryl;

wherein homoaryl is phenyl or phenyl substituted with one, two or three substituents each independently selected from the group consisting of halo, cyano, $C_{1-3}$alkyl, cyclopropyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy-, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and HC≡$CCH_2O$-;

heteroaryl is selected from the group consisting of pyridyl, pyrimidinyl, pyrazinyl, pyridazinyl, furanyl, thienyl, pyrrolyl, pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents each independently selected from the group consisting of halo, cyano, $C_{1-3}$alkyl, cyclopropyl, $C_{2-3}$alkynyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropy-

loxy)$C_{1-3}$alkyloxy, and HC≡$CCH_2O$-;

$R^3$, $R^4$, $R^5$, and $R^6$ are each independently selected from H, fluoro and methyl;

and the pharmaceutically acceptable addition salts and the solvates thereof.

In an embodiment $R^1$ is $C_{1-2}$alkyl and all other variables are as described in Formula (I) or (I-a) herein. In an embodiment $R^1$ is $CH_3$ and all other variables are as described in Formula (I) or (I-a) herein.

In an embodiment $R^2$ is -$SO_2C_{1-3}$alkyl, -$SO_2$cyclopropyl, or -CN and all other variables are as described in Formula (I) or (I-a) herein.

In an embodiment $R^2$ is -$SO_2C_{1-3}$alkyl or -CN and all other variables are as described in Formula (I) or (I-a) herein.

In an embodiment $R^2$ is -$SO_2C_{1-3}$alkyl, in particular -$SO_2CH_3$, -$SO_2CH_2CH_3$, -$SO_2CH(CH_3)_2$, and all other variables are as described in Formula (I) or (I-a) herein.

In an embodiment $R^2$ is -$SO_2C_{1-3}$alkyl, in particular -$SO_2CH_3$, and all other variables are as described in Formula (I) or (I-a) herein.

In an embodiment $R^2$ is -CN and all other variables are as described in Formula (I) or (I-a) herein.

In an embodiment, heteroaryl is selected from the group consisting of pyridyl, pyrimidinyl, pyrazinyl, and pyridazinyl, each optionally substituted with one, two or three substituents each independently selected from the group consisting of halo, cyano, $C_{1-3}$alkyl, cyclopropyl, $C_{2-3}$alkynyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and HC≡$CCH_2O$-, and all other variables are as described in Formula (I) or (I-a) herein.

In an embodiment, heteroaryl is selected from the group consisting of furanyl, thienyl, pyrrolyl, pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents each independently selected from the group consisting of halo, cyano, $C_{1-3}$alkyl, cyclopropyl, $C_{2-3}$alkynyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and HC≡$CCH_2O$-, and all other variables are as described in Formula (I) or (I-a) herein.

In an embodiment, heteroaryl is selected from the group consisting of pyridyl, pyrimidinyl, pyrazinyl, pyridazinyl, and oxazolyl, each optionally substituted with one, two or three substituents each independently selected from the group consisting of halo, cyano, $C_{1-3}$alkyl, cyclopropyl, monohalo-$C_{1-3}$alkyl, polyhalo-$C_{1-3}$alkyl, monohalo-$C_{1-3}$alkyloxy, polyhalo-$C_{1-3}$alkyloxy, and $C_{1-3}$alkyloxy; and all other variables are as described in Formula (I) or (I-a) herein.

In an embodiment, heteroaryl is selected from the group consisting of pyridyl, pyrimidinyl, pyrazinyl, and pyridazinyl, each optionally substituted with one, two or three substituents each independently selected from the group consisting of halo, cyano, $C_{1-3}$alkyl, cyclopropyl, monohalo-$C_{1-3}$alkyl, polyhalo-$C_{1-3}$alkyl, monohalo-$C_{1-3}$alkyloxy, polyhalo-$C_{1-3}$alkyloxy, and $C_{1-3}$alkyloxy; and all other variables are as described in Formula (I) or (I-a) herein.

In an embodiment, heteroaryl is selected from the group consisting of pyridyl, pyrimidinyl, pyrazinyl, and pyridazinyl, each optionally substituted with one, two or three substituents each independently selected from the group consisting of halo, cyano, $C_{1-3}$alkyl, cyclopropyl, and $C_{1-3}$alkyloxy; and all other variables are as described in Formula (I) or (I-a) herein.

In an embodiment Ar is pyridyl substituted with one or two substituents each independently selected from the group consisting of halo, cyano, $C_{1-3}$alkyl and $C_{1-3}$alkyloxy; and all other variables are as described in Formula (I) or (I-a) herein.

In an embodiment Ar is pyridyl substituted with one or two substituents, each independently selected from halo, cyano and $C_{1-3}$alkyl; and all other variables are as described in Formula (I) or (I-a) herein.

In an embodiment Ar is pyridyl substituted with one or two substituents each independently selected from the group consisting of halo, $C_{1-3}$alkyl and $C_{1-3}$alkyloxy, and all other variables are as described in Formula (I) or (I-a) herein.

In an embodiment Ar is pyridyl substituted with one or two independently selected halo substituents, and all other variables are as described in Formula (I) or (I-a) herein.

In an embodiment Ar is pyrazinyl substituted with one or two substituents each independently selected from the group consisting of halo, $C_{1-3}$alkyl, $C_{1-3}$alkyloxy, monohalo-$C_{1-3}$alkyl, polyhalo-$C_{1-3}$alkyl, monohalo-$C_{1-3}$alkyloxy, and polyhalo-$C_{1-3}$alkyloxy; and all other variables are as described in Formula (I) or (I-a) herein.

In an embodiment Ar is pyrazinyl substituted with one or two substituents each independently selected from the group consisting of halo, $C_{1-3}$alkyl and $C_{1-3}$alkyloxy, and all other variables are as described in Formula (I) or (I-a) herein.

In an embodiment Ar is pyrazinyl substituted with one or two substituents each independently selected from halo or $C_{1-3}$alkyloxy, and all other variables are as described in Formula (I) or (I-a) herein.

In an embodiment Ar is pyrazinyl substituted with one or two substituents each independently selected from the group consisting of monohalo-$C_{1-3}$alkyl, polyhalo-$C_{1-3}$alkyl, monohalo-$C_{1-3}$alkyloxy, and polyhalo-$C_{1-3}$alkyloxy, and all other variables are as described in Formula (I) or (I-a) herein.

In an embodiment, $>CR^3R^4$ is selected from the group consisting of $>CH_2$, $>CHF$, $>CF_2$, $>C(CH_3)F$, and $>C(CH_3)(OCH_3)$ and all other variables are as described in Formula (I) herein.

In an embodiment, $>CR^3R^4$ is selected from the group consisting of $>CH_2$, $>CHF$, $>C(CH_3)F$, and $>C(CH_3)(OCH_3)$ and all other variables are as described in Formula (I) herein.

In an embodiment, $>CR^3R^4$ is selected from the group consisting of $>CH_2$, $>CHF$, $>CF_2$, and $>C(CH_3)F$, and $-CHR^5R^6$ is $-CH_3$, $-CH_2F$ or $-CHF_2$ and all other variables are as described in Formula (I) or (I-a) herein.

In an embodiment, $>CR^3R^4$ is selected from the group consisting of $>CH_2$, $>CHF$, and $>C(CH_3)F$, and $-CHR^5R^6$ is $-CH_3$, $-CH_2F$ or $-CHF_2$ and all other variables are as described in Formula (I) or (I-a) herein.

In an embodiment, $>CR^3R^4$ is $>CH_2$ or $>CHF$, and $-CHR^5R^6$ is $-CH_3$, $-CH_2F$ or $-CHF_2$ and all other variables are as described in Formula (I) or (I-a) herein.

In an embodiment, $>CR^3R^4$ is $>CH_2$, and $-CHR^5R^6$ is $-CH_2F$ and all other variables are as described in Formula (I) or (I-a) herein.

In an embodiment, $R^7$ is hydrogen or fluoro and all other variables are as described in Formula (I) herein.

In an embodiment, $R^7$ is fluoro and all other variables are as described in Formula (I) herein.

In an embodiment $R^1$ is $CH_3$, $R^2$ is $-SO_2C_{1-3}$alkyl, in particular $-SO_2CH_3$, $-SO_2CH_2CH_3$, or $-SO_2CH(CH_3)_2$, and Ar is pyridyl or pyrazinyl, each optionally substituted with one or two substituents each independently selected from the group consisting of halo, cyano, $C_{1-3}$alkyl, cyclopropyl, monohalo-$C_{1-3}$alkyl, polyhalo-$C_{1-3}$alkyl, monohalo-$C_{1-3}$alkyloxy, polyhalo-$C_{1-3}$alkyloxy, and $C_{1-3}$alkyloxy; and all other variables are as described in Formula (I) or (I-a) herein.

In an embodiment $R^1$ is $CH_3$, $R^2$ is $-SO_2C_{1-3}$alkyl, in particular $-SO_2CH_3$, and Ar is pyrazinyl substituted with one or two substituents each independently selected from halo or $C_{1-3}$alkyloxy and all other variables are as described in Formula (I) or (I-a) herein.

In an embodiment $R^1$ is $CH_3$, $R^2$ is $-SO_2C_{1-3}$alkyl, in particular $-SO_2CH_3$, and Ar is pyridyl substituted with one or two substituents each independently selected from halo or $C_{1-3}$alkyloxy and all other variables are as described in Formula (I) or (I-a) herein.

In an embodiment $R^1$ is $CH_3$, $R^2$ is $-SO_2C_{1-3}$alkyl, in particular $-SO_2CH_3$, $-SO_2CH_2CH_3$, or $-SO_2CH(CH_3)_2$, and Ar is pyridyl substituted with one or two substituents each independently selected from halo, cyano and $C_{1-3}$alkyl; and all other variables are as described in Formula (I) or (I-a) herein.

In an embodiment $R^1$ is $CH_3$, $R^2$ is $-SO_2C_{1-3}$alkyl, in particular $-SO_2CH_3$, $-SO_2CH_2CH_3$, $-SO_2CH(CH_3)_2$, and Ar is pyrazinyl optionally substituted with one or two substituents each independently selected from the group consisting of monohalo-$C_{1-3}$alkyl, polyhalo-$C_{1-3}$alkyl, monohalo-$C_{1-3}$alkyloxy, and polyhalo-$C_{1-3}$alkyloxy; and all other variables are as described in Formula (I) or (I-a) herein.

In an embodiment $R^1$ is $CH_3$, $R^2$ is -CN and Ar is pyrazinyl substituted with one or two substituents each independently selected from halo or $C_{1-3}$alkyloxy and all other variables are as described in Formula (I) or (I-a) herein.

In an embodiment $R^1$ is $CH_3$, $R^2$ is $-SO_2CH_3$ or -CN, and Ar is 5-methoxypyrazin-2-yl and all other variables are as described in Formula (I) or (I-a) herein.

In an embodiment $R^1$ is $CH_3$, $R^2$ is $-SO_2CH_3$, Ar is 5-methoxypyrazin-2-yl, $>CR^3R^4$ is $>CH_2$, and $-CHR^5R^6$ is $-CH_3$ or $-CH_2F$ and all other variables are as described in Formula (I) or (I-a) herein.

In an embodiment $R^1$ is $CH_3$, $R^2$ is -CN, Ar is 5-methoxypyrazin-2-yl, $>CR^3R^4$ is $>CH_2$ or $>CHF$, and $-CHR^5R^6$ is $-CH_3$ or $-CHF_2$ and all other variables are as described in Formula (I) or (I-a) herein.

In the compounds of Formula (I) or (I-a) as defined herein, the quaternary carbon atom substituted with $-CHR^5R^6$ and the

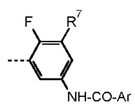

moiety (herein referred to as C-2) has a configuration as depicted in the structure (I) or (I-a) herein, wherein the 2,3,4,5-tetrahydropyridin-6-amine core is in the plane of the drawing, $-CHR^5R^6$ is projected below the plane of the drawing (with the bond shown with a wedge of parallel lines 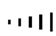 ) and the

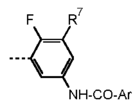

moiety is projected above the plane of the drawing (with the bond shown with a bold wedge 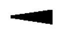).

In a further embodiment, in the compound of Formula (I) or (I-a) the quaternary carbon atom substituted with $R^1$ and $R^2$ (*i.e.* $CR^1R^2$, herein referred to as C-5) has a configuration as depicted in the structures ($I^{I}$) and ($I^{II}$) below, wherein for example, $-SO_2C_{1-3}$alkyl and -CN are respectively, projected below the plane of the drawing. In ($I^{I}$) and ($I^{II}$), all variables are as defined herein.

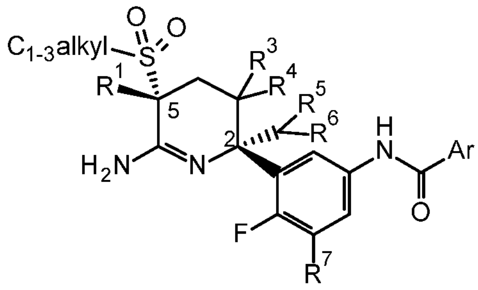

($I^{I}$)

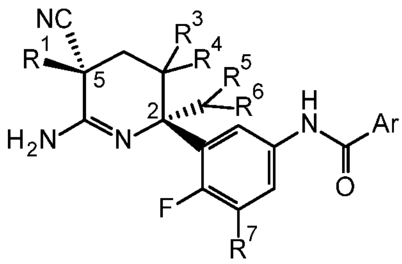

($I^{II}$)

In particular, said compounds of Formulae ($I^{I}$) and ($I^{II}$) have the Formulae (I') and (Ia'), and (I") and (Ia"), respectively. In (I'), (I"), (Ia') and (Ia"), all variables are as defined herein.

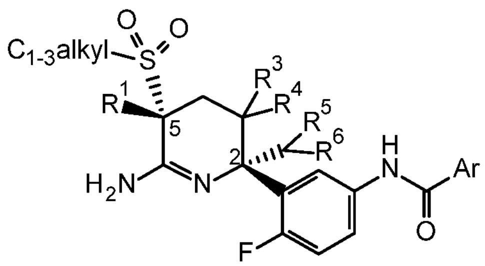

(I')

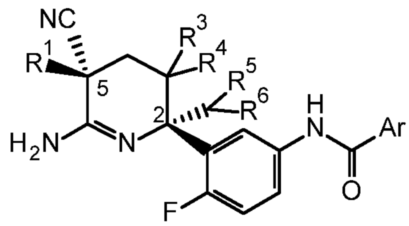

(I")

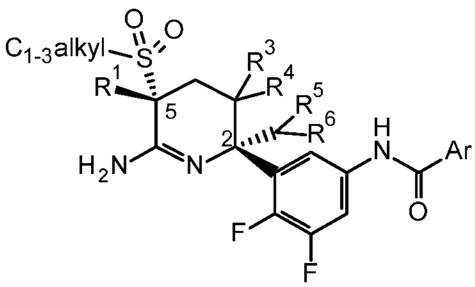

(Ia')

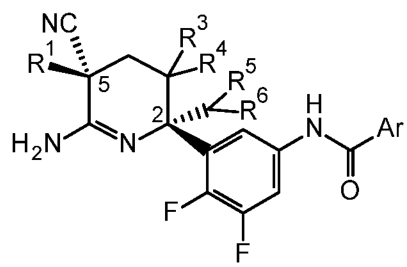

(Ia")

DEFINITIONS

"Halo" shall denote fluoro, chloro and bromo; "$C_{1-2}$alkyl" and"$C_{1-3}$alkyl" shall denote a straight or branched saturated alkyl group having 1 or 2, or 1, 2 or 3 carbon atoms, respectively e.g. methyl, ethyl, 1-propyl and 2-propyl; "$C_{1-3}$alkyloxy" shall denote an ether radical wherein $C_{1-3}$alkyl is as defined before; "$C_{2-3}$alkynyl" shall denote an acyclic

straight or branched hydrocarbon of 2 or 3 carbon atoms and having a carbon-carbon triple bond; "mono- and polyhalo$C_{1-3}$alkyl" and "mono- and polyhalo-cyclopropyl" shall denote $C_{1-3}$alkyl as defined before or cyclopropyl, respectively, substituted with 1, 2, 3 or where possible with more halo atoms as defined before; "mono- and polyhalo$C_{1-3}$alkyloxy" and "mono- and polyhalo-cyclopropyloxy" shall denote an ether radical wherein mono- and polyhalo$C_{1-3}$alkyl and mono- and polyhalo-cyclopropyl are as defined before.

The term "subject" as used herein, refers to an animal, preferably a mammal, most preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

Hereinbefore and hereinafter, the term "compound of formula (I) or (I-a)" is meant to include the addition salts, the solvates and the stereoisomers thereof.

The terms "stereoisomers" or "stereochemically isomeric forms" hereinbefore or hereinafter are used interchangeably.

The invention includes all stereoisomers of the compound of Formula (I) or (I-a) either as a pure stereoisomer or as a mixture of two or more stereoisomers.

Enantiomers are stereoisomers that are non-superimposable mirror images of each other. A 1:1 mixture of a pair of enantiomers is a racemate or racemic mixture. Diastereomers (or diastereoisomers) are stereoisomers that are not enantiomers, i.e. they are not related as mirror images. If a compound contains a double bond, the substituents may be in the E or the Z configuration. If a compound contains a disubstituted cycloalkyl group, the substituents may be in the cis or trans configuration. Therefore, the invention includes enantiomers, diastereomers, racemates, E isomers, Z isomers, cis isomers, trans isomers and mixtures thereof.

The absolute configuration is specified according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) or (I-a) is for instance specified as (R), this means that the compound is substantially free of the (S) isomer; when a compound of formula (I) or (I-a) is for instance specified as E, this means that the compound is substantially free of the Z isomer; when a compound of formula (I) or (I-a) is for instance specified as cis, this means that the compound is substantially free of the trans isomer.

For use in medicine, the addition salts of the compounds of this invention refer to non-toxic "pharmaceutically acceptable addition salts". Other salts may, however, be useful in the preparation of compounds according to this invention or of their pharmaceutically acceptable addition salts. Suitable pharmaceutically acceptable addition salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable addition salts thereof may include alkali metal salts, e.g., sodium or potassium salts; alkaline earth metal salts, e.g., calcium or magnesium salts; and salts formed with suitable organic ligands, e.g., quaternary ammonium salts.

Representative acids which may be used in the preparation of pharmaceutically acceptable addition salts include, but are not limited to, the following: acetic acid, 2,2-dichloroactic acid, acylated amino acids, adipic acid, alginic acid, ascorbic acid, L-aspartic acid, benzenesulfonic acid, benzoic acid, 4- acetamidobenzoic acid, (+)-camphoric acid, camphorsulfonic acid, capric acid, caproic acid, caprylic acid, cinnamic acid, citric acid, cyclamic acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, beta-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5- disulfonic acid, 1-hydroxy-2-naphthoic acid, nicotinic acid, nitric acid, oleic acid, orotic acid, oxalic acid, palmitic acid, pamoic acid, phosphoric acid, L- pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, trifluoromethylsulfonic acid, and undecylenic acid. Representative bases which may be used in the preparation of pharmaceutically acceptable addition salts include, but are not limited to, the following: ammonia, L-arginine, benethamine, benzathine, calcium hydroxide,

choline, dimethylethanolamine, diethanolamine, diethylamine, 2-(diethylamino)-ethanol, ethanolamine, ethylene-diamine, *N*-methyl-glucamine, hydrabamine, 1*H*-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The names of compounds were generated according to the nomenclature rules agreed upon by the Chemical Abstracts Service (CAS) or according to the nomenclature rules agreed upon by the International Union of Pure and Applied Chemistry (IUPAC).

The compounds according to formula (I), in particular (I-a), may be in dynamic equilibrium with their tautomeric form (I*) and form an inseparable mixture. Such tautomeric forms although not explicitly indicated in the above formula are intended to be included within the scope of the present invention.

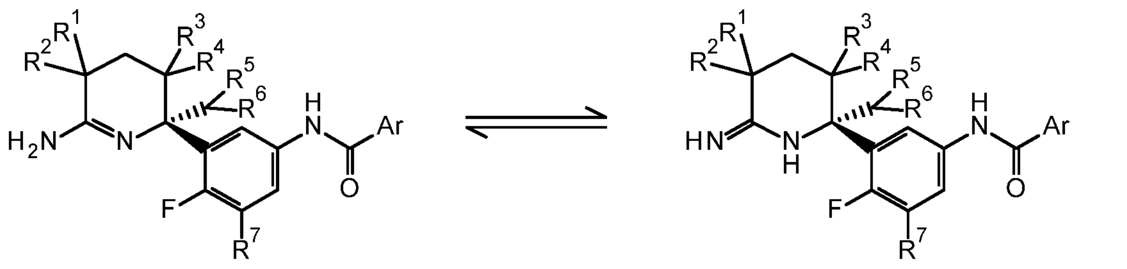

(I) (I*)

PREPARATION OF THE COMPOUNDS

EXPERIMENTAL PROCEDURE 1

The final compounds according to Formula (I) can be prepared by reacting an intermediate compound of Formula (II-a) with a compound of Formula (XXIV) according to reaction scheme (1). The reaction is performed in a suitable reaction-inert solvent, such as, for example, dioxane, in the presence of a suitable base, such as, for example, potassium phosphate ($K_3PO_4$), a copper catalyst such as, for example, copper(I) iodide (CuI) and a diamine such as, for example, (1*R*,2*R*)-(-)-1,2-diaminocyclohexane or *N*,*N*'-dimethylethylenediamine, under thermal conditions such as, for example, heating the reaction mixture at 100 °C, for example for 16 hours. In reaction scheme (1) all variables are defined as in Formula (I) and Z is halo.

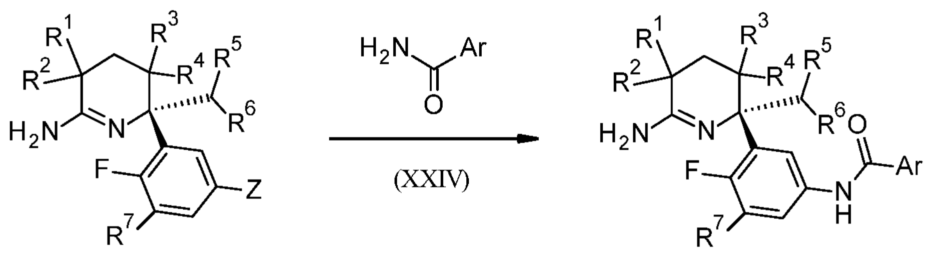


(II-a) (I)

Reaction Scheme 1

EXPERIMENTAL PROCEDURE 2

Additionally, the final compounds according to Formula (I) can be prepared by reacting an intermediate compound of Formula (II-b) with a compound of Formula (XXV) according to reaction scheme (2). The reaction is performed in a suitable reaction-inert solvent, such as, for example, methanol (MeOH), in the presence of an acid, such as, for example, hydrochloric acid (HCl), and of a carboxyl activating agent such as, for example, 1-ethyl-3-(3-dimethylaminopropyl)carbodiimide [EDCI, CAS 1892-57-5], under suitable conditions such as, for example, stirring the reaction mixture at 25 °C, for example for 10 minutes. In reaction scheme (2) all variables are defined as in Formula (I).

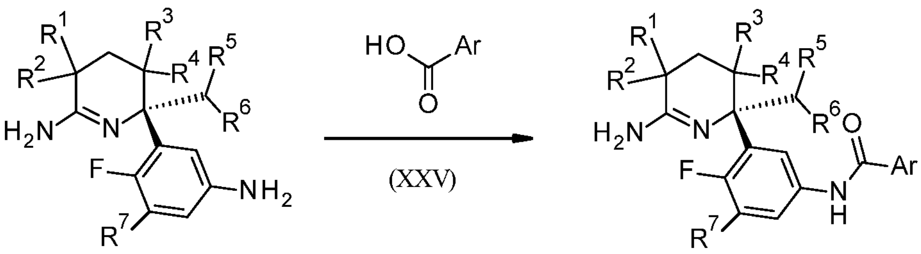


(II-b) (I)

Reaction Scheme 2

EXPERIMENTAL PROCEDURE 3

Intermediate compounds according to Formula (II-b) can be prepared by subjecting an intermediate compound of Formula (III) to reducing conditions according to reaction scheme (3). Typical examples are reduction by a suitable catalyst, such as, for example, palladium on carbon, under hydrogen atmosphere, or the use of a reducing agent such as, for example, tin(II) chloride. The reactions are typically performed in a suitable solvent, such as, for example, MeOH, or in a solvent mixture, such as tetrahydrofuran(THF)/ethanol(EtOH). Thermal conditions such as, for example, heating the mixture, may improve the reaction outcome. In reaction scheme (3) all variables are defined as in Formula (I).

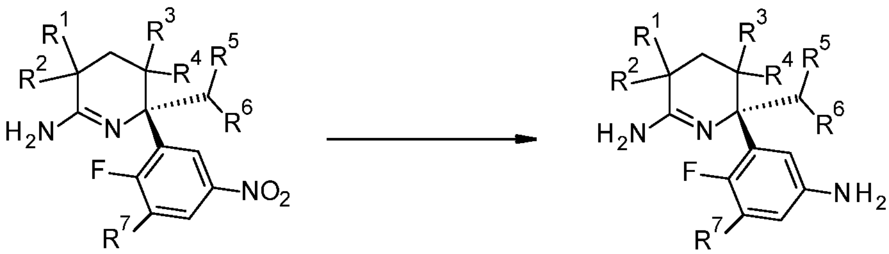

(III) (II-b)

Reaction Scheme 3

EXPERIMENTAL PROCEDURE 4

Intermediate compound of Formula (II-b) can alternatively be prepared from intermediate of Formula (II-a) according to reaction scheme (4). In a typical procedure, a compound of Formula (II-a) in which Z is a halo, for example bromo, is reacted with sodium azide ($NaN_3$) to an intermediate compound of Formula (II-b). The reaction is performed in a suitable reaction-inert solvent, such as, for example acetonitrile, in the presence of a suitable base, such as, for example, sodium carbonate ($Na_2CO_3$), a copper catalyst such as, for example, copper(I) iodide (CuI) and a diamine such as, for example, *N*,*N'*-dimethylethylenediamine, under thermal conditions such as, for example, heating the reaction mixture at 100 °C, for example for 16 hours. In reaction scheme (4) all variables are defined as in Formula (I).

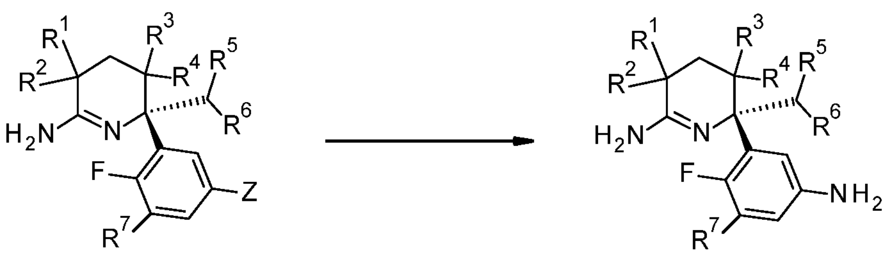

(II-a) (II-b)

Reaction scheme 4

EXPERIMENTAL PROCEDURE 5

Intermediate compounds according to Formula (III) can be prepared by nitration of an intermediate compound of Formula (II) according to reaction scheme (5). A typical procedure involves the treatment of intermediate (II), dissolved in sulphuric acid, with a source of nitronium ion, such as, for example, potassium nitrate, at low temperature, such as, for example, 0 °C. In reaction scheme (5) $R^7$ is hydrogen, and all other variables are defined as in Formula (I).

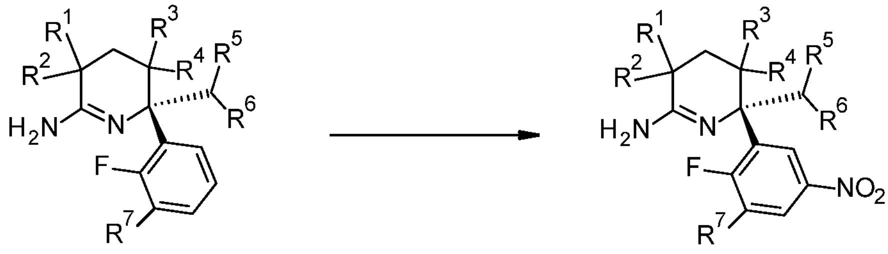

(II) (III)

Reaction Scheme 5

EXPERIMENTAL PROCEDURE 6

Intermediate compounds according to Formulas (II) and (II-a) can be prepared by means of one-step or two-step procedures, according to reaction scheme (6), starting from a suitable compound of Formula (IV), where PG is a suitable protecting group, such as, for example, *tert*-butoxycarbonyl (BOC), trifluoroacetyl or tert-butylsulphinyl. In the two-step procedure the amino group in intermediate (IV) is first deprotected to give intermediate (V) by means of methods known to the person skilled in the art, such as, for example, by treating intermediate (IV) with an acid such as, for example, formic acid. Heating the reaction mixture, for example at 80 °C for about 4 hours, may improve the reaction outcome. Isolated intermediate (V) can then be dissolved in a suitable solvent, such as, for example, dichloromethane (DCM), and cyclised into the corresponding intermediate (II) or (II-a) in the presence of a Lewis acid, such as, for example, trimethyl aluminium. Alternatively, intermediate (IV) can be stirred in the presence of an acid, such as in-situ generated HCl in methanolic solution or pure formic acid, under thermal conditions, such as, for example, heating the reaction mixture at about 120 °C for a period of time sufficient to drive the reaction to completion, to obtain corresponding intermediate (II) or (II-a) in one pot. In reaction scheme (6) all variables are defined as in Formula (I), Z is hydrogen or halo and PG is a protecting group.

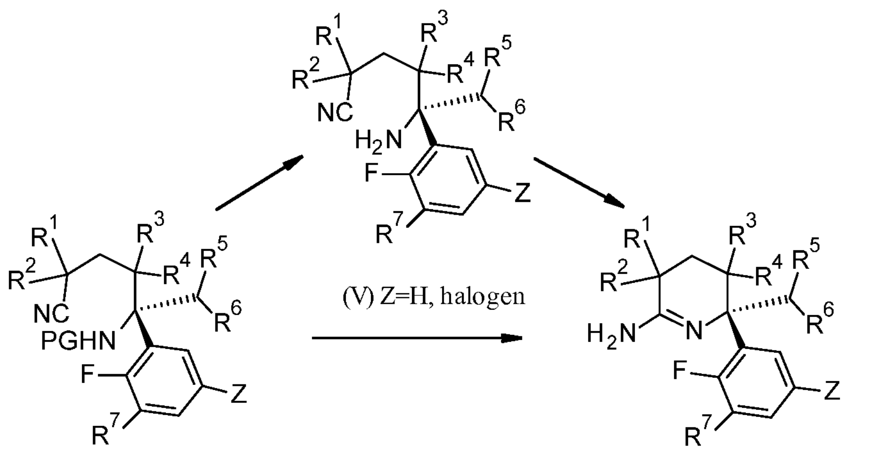


(IV) Z=H, halogen
PG=protecting group

(II) Z=H
(II-a) Z=halogen

Reaction Scheme 6

EXPERIMENTAL PROCEDURE 7

Intermediate compounds according to Formula (IV) can be obtained by a two-step procedure starting from intermediate (VII) according to reaction scheme (7). Intermediate (VII) can be converted into intermediate (VI) by treatment with a reducing agent, such as, for example, sodium borohydride, in a suitable solvent, such as, for example, THF. Low temperature, such as, for example, 0 °C, may improve the reaction outcome. Intermediate (VI) can then be converted into intermediate (IV) by means of standard methylation reactions, such as, for example, by treating the compound, dissolved in a suitable solvent, such as, for example, THF, with a base, such as, for example, sodium hydride, and quenching the resulting anion with a methylating agent, such as, for example, methyl iodide, at low temperature, such as, for example, at 0 °C. In reaction scheme (7) all variables are defined as in Formula (I), Z is hydrogen or halo and PG is a protecting group for example, *tert*-butoxycarbonyl (BOC), trifluoroacetyl or *tert*-butylsulphinyl.

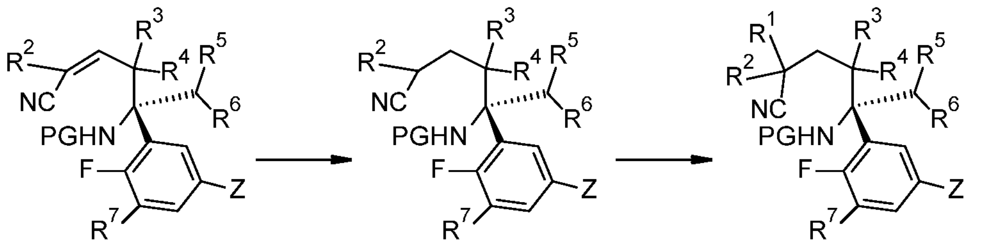

(VII) Z=H, halogen PG=protecting group

(VI) Z=H, halogen PG=protecting group

(IV) Z=H, halogen PG=protecting group

Reaction Scheme 7

EXPERIMENTAL PROCEDURE 8

Intermediate compounds according to Formula (VII) can be prepared in six steps starting from intermediate (XIII) according to reaction scheme (8). Intermediate (XIII) can be converted into intermediate (XII) by means of the nucleophilic addition of an appropriate anion. The anion can be generated by means of methods known to the person skilled in the art: Typical examples are treating the desired acetate, such as, for example, tert-butyl acetate, with an appropriate base, such as, for example, lithium diisopropylamide, in an inert solvent, such as, for example, THF, at a low temperature, such as, for example, at -78 °C, or treating the corresponding $\alpha$-bromoacetate with zinc in the presence of Cu(I) in an inert solvent, such as, for example, THF, at a temperature high enough to promote the insertion of the zinc into the carbon-bromine bond, such as, for example, at 40 °C. The solution of the anion can then be reacted with a solution of intermediate (XIII) in an appropriate solvent, such as THF, at a temperature which allows smooth reaction, such as, for example, -78 °C or 0 °C, to afford intermediate (XII). The chirality in which the

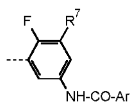

moiety is projected above the plane of the drawing (with the bond shown with a bold wedge ) can be enriched by using an intermediate (XIII) in which the tert-butylsulfinyl group has the R-configuration. By choosing a suitable acid, such as, for example, hydrochloric acid, intermediate (XII) can then undergo hydrolysis of the ester and removal of the nitrogen protecting group in one pot to afford intermediate (XI). Stirring the reaction under thermal conditions, such as, for example, at 80 °C for 5 hours, may improve the reaction outcome. Intermediate (XI) can subsequently be reduced into the corresponding alcohol by treatment with a standard reducing agent, such as, for example, borane in THF, to afford intermediate (X). The amino group of intermediate (X) can be protected by means of methods known to the person skilled in the art, such as, for example, by treating intermediate (X), dissolved in a suitable solvent, such as, for example, DCM or THF, with an appropriate anhydride, such as, for example, trifluoroacetic anhydride or tert-butoxycarbonyl anhydride (BOC-anhydride), in the presence of a base, such as, for example, triethylamine or sodium hydrogenocarbonate. Protected intermediate (IX) can be subsequently oxidised to aldehyde (VIII) by means of standard oxidising agents, such as, for example, Dess-Martin periodinane in an inert solvent, such as, for example, DCM. Intermediate (VIII) can be finally converted into intermediate (VII) by means of a Knoevenagel condensation with a suitable active

hydrogen component, such as, for example, malononitrile or 2-(methylsulfonyl)acetonitrile, in the presence of a catalyst, such as, for example, magnesium oxide, in an inert solvent, such as, for example, MeOH. In reaction scheme (8) all variables are defined as in Formula (I), Z is hydrogen or halo, PG is a protecting group and Alk is a suitable alkyl chain, e.g. ethyl.

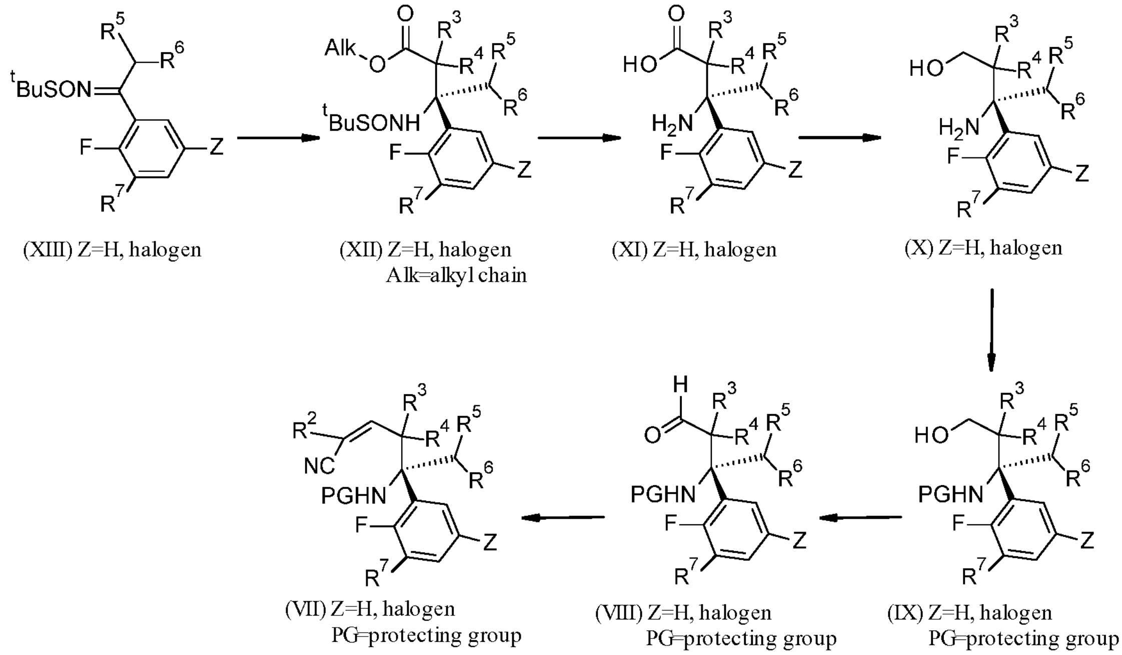

(XIII) Z=H, halogen

(XII) Z=H, halogen
Alk=alkyl chain

(XI) Z=H, halogen

(X) Z=H, halogen

(VII) Z=H, halogen
PG=protecting group

(VIII) Z=H, halogen
PG=protecting group

(IX) Z=H, halogen
PG=protecting group

Reaction Scheme 8

EXPERIMENTAL PROCEDURE 8-A

Alternatively, intermediate compounds according to Formula (X) can be obtained in two steps starting from intermediate (XII-a), where $Alk^1$ is a suitable alkyl chain, such as, for example, ethyl, according to reaction scheme (8-a). Treatment of intermediate (XII-a) with an ester reducing agent, such as, for example, lithium borohydride, in an inert solvent, such as, for example, THF, at a temperature which allows smooth reaction, such as, for example, at 0 °C, yields intermediate (XXII), which may be further deprotected into intermediate (X) by treatment with an appropriate acid, such as, for example, HCl, in an inert solvent, such as, for example, MeOH. In reaction scheme (8-a) all variables are defined as in Formula (I), Z is hydrogen or halo and $Alk^1$ is a suitable alkyl chain, such as ethyl.

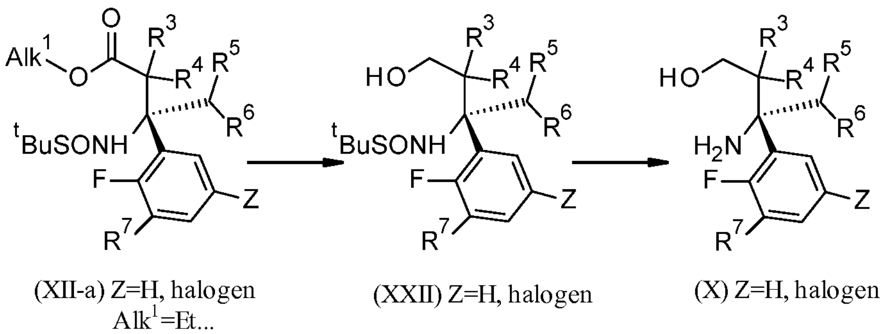

(XII-a) Z=H, halogen
$Alk^1$=Et...

(XXII) Z=H, halogen

(X) Z=H, halogen

Reaction Scheme 8-a

EXPERIMENTAL PROCEDURE 9

Intermediate compounds according to Formula (VII-a) can be prepared in three steps starting from intermediate (XIII), according to reaction scheme (9). Intermediate (XIII), dissolved in a suitable solvent, such as, for example, DCM, can be reacted with a suitable nucleophile, such as, for example, allylmagnesium bromide, at low temperature, such as,

for example, at -50 °C, to give intermediate (XXI). The chirality in which the

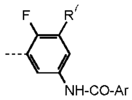

moiety is projected above the plane of the drawing (with the bond shown with a bold wedge ) can be enriched by using an intermediate (XIII) in which the tert-butylsulfinyl group has the R-configuration. Oxidative cleavage of the newly installed double bond by means of standard methods, such as, for example, ozonolysis at low temperature, such as, for example, at 0 °C, affords intermediate (XX), which can be finally converted into intermediate (VII-a) by means of a Knoevenagel condensation with a suitable active hydrogen component, such as, for example, malononitrile or 2-(methylsulfonyl)acetonitrile, in the presence of a catalyst, such as, for example, magnesium oxide, in an inert solvent, such as, for example, MeOH. In reaction scheme (9) all variables are defined as in Formula (I) and Z is hydrogen or halo.

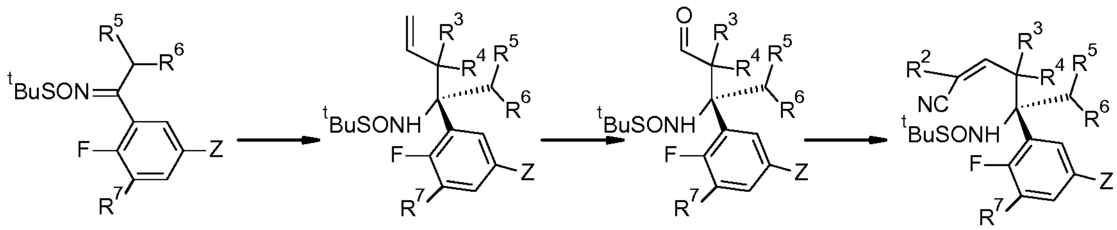


Reaction Scheme 9

EXPERIMENTAL PROCEDURE 10

Intermediate compounds according to Formula (VII-b) can be prepared in four steps starting from intermediate (XII-a), according to reaction scheme (10). Intermediate (XII-a) can be deprotected to give free-amino intermediate (XVIII) by means of standard deprotection techniques, such as by treating intermediate (XII-a), dissolved in a suitable solvent, such as MeOH, with an acid, such as, for example, HCl. Conversion to the mono-BOC derivative intermediate (XVII) can be achieved by submitting intermediate (XVIII) to conditions known to the person skilled in the art, such as, for example, by treating intermediate (XVIII), dissolved in an appropriate solvent, such as, for example, MeOH, with a BOC source, such as, for example, BOC-anhydride. Raising the temperature, for example to 60 °C, for example for 7 hours, may improve the reaction outcome. Intermediate (XVII), dissolved in a suitable solvent, such as, for example, DCM or THF, can be reduced to the corresponding aldehyde (XVI) by means of selective reducing agents, such as, for example, diisobutylaluminium hydride, at low temperature, such as, for example, at -78 °C, or lithium borohydride at low temperature, such as, for example, at 0 °C. Possible overreduced alcohol side-products can be converted back into intermediate (XVI) by means of standard oxidation reagents, such as, for example, by using Dess-Martin periodinane in DCM. Intermediate (XVI) can be finally converted into intermediate (VII-b) by means of a Knoevenagel condensation with a suitable active hydrogen component, such as, for example, malononitrile or 2-(methylsulfonyl)acetonitrile, in the presence of a catalyst, such as, for example, magnesium oxide, in a suitable solvent, such as, for example, MeOH. In reaction scheme (10), all variables are defined as in Formula (I), Z is hydrogen or halo and $Alk^1$ is a suitable alkyl chain.

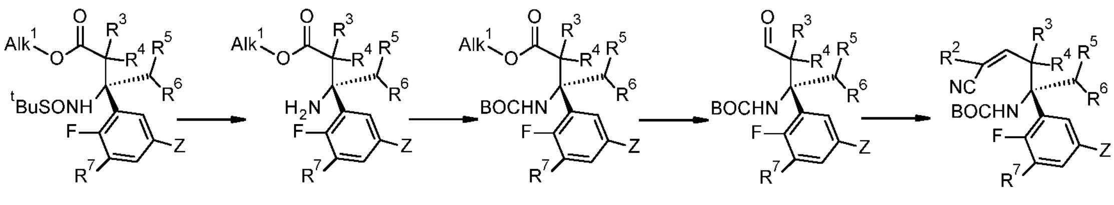


Reaction Scheme 10

PHARMACOLOGY

The compounds of the present invention and the pharmaceutically acceptable compositions thereof inhibit BACE and therefore may be useful in the treatment or prevention of Alzheimer's Disease (AD), mild cognitive impairment (MCI), senility, dementia, dementia with Lewy bodies, cerebral amyloid angiopathy, multi-infarct dementia, Down's syndrome, dementia associated with Parkinson's disease, dementia of the Alzheimer's type, vascular dementia, dementia due to HIV disease, dementia due to head trauma, dementia due to Huntington's disease, dementia due to Pick's disease, dementia due to Creutzfeldt-Jakob disease, frontotemporal dementia, dementia pugilistica, and dementia associated with beta-amyloid.

As used herein, the term "treatment" is intended to refer to all processes, wherein there may be a slowing, interrupting, arresting or stopping of the progression of a disease or an alleviation of symptoms, but does not necessarily indicate a total elimination of all symptoms.

The invention also relates to a compound according to the general Formula (I), a stereoisomeric form thereof or a pharmaceutically acceptable acid or base addition salt thereof, for use in the treatment or prevention of diseases or conditions selected from the group consisting of AD, MCI, senility, dementia, dementia with Lewy bodies, cerebral amyloid angiopathy, multi-infarct dementia, Down's syndrome, dementia associated with Parkinson's disease, dementia of the Alzheimer's type, and dementia associated with beta-amyloid.

The invention also relates to a compound according to the general Formula (I), a stereoisomeric form thereof or a pharmaceutically acceptable acid or base addition salt thereof, for use in the treatment, prevention, amelioration, control or reduction of the risk of diseases or conditions selected from the group consisting of AD, MCI, senility, dementia, dementia with Lewy bodies, cerebral amyloid angiopathy, multi-infarct dementia, Down's syndrome, dementia associated with Parkinson's disease, dementia of the Alzheimer's type, and dementia associated with beta-amyloid.

As already mentioned hereinabove, the term "treatment" does not necessarily indicate a total elimination of all symptoms, but may also refer to symptomatic treatment in any of the disorders mentioned above. In view of the utility of the compound of Formula (I), there is provided a compound for use in a method of treating subjects such as warm-blooded animals, including humans, suffering from or a method of preventing subjects such as warm-blooded animals, including humans, suffering from any one of the diseases mentioned hereinbefore.

Said methods comprise the administration, i.e. the systemic or topical administration, preferably oral administration, of a therapeutically effective amount of a compound of Formula (I), a stereoisomeric form thereof, a pharmaceutically acceptable addition salt or solvate thereof, to a subject such as a warm-blooded animal, including a human.

A method of treatment may also include administering the active ingredient on a regimen of between one and four intakes per day. In these methods of treatment the compounds according to the invention are preferably formulated prior to administration. As described herein below, suitable pharmaceutical formulations are prepared by known procedures using well known and readily available ingredients.

The compounds of the present invention, that can be suitable to treat or prevent Alzheimer's disease or the symptoms thereof, may be administered alone or in combination with one or more additional therapeutic agents. Combination therapy includes administration of a single pharmaceutical dosage formulation which contains a compound of Formula (I) and one or more additional therapeutic agents, as well as administration of the compound of Formula (I) and each additional therapeutic agent in its own separate pharmaceutical dosage formulation. For example, a compound of Formula (I) and a therapeutic agent may be administered to the patient together in a single oral dosage composition such as a tablet or capsule, or each agent may be administered in separate oral dosage formulations.

A skilled person will be familiar with alternative nomenclatures, nosologies, and classification systems for the diseases or conditions referred to herein. For example, the fifth edition of the Diagnostic & Statistical Manual of Mental Disorders (DSM-5™) of the American Psychiatric Association utilizes terms such as neurocognitive disorders (NCDs) (both major and mild), in particular, neurocognitive disorders due to Alzheimer's disease, due to traumatic brain injury (TBI), due to Lewy body disease, due to Parkinson's disease or to vascular NCD (such as vascular NCD present with multiple infarctions). Such terms may be used as an alternative nomenclature for some of the diseases or conditions referred to herein by the skilled person.

PHARMACEUTICAL COMPOSITIONS

The present invention also provides compositions for preventing or treating diseases in which inhibition of beta-secretase is beneficial, such as Alzheimer's disease (AD), mild cognitive impairment, senility, dementia, dementia with Lewy bodies, Down's syndrome, dementia associated with stroke, dementia associated with Parkinson's disease and dementia associated with beta-amyloid. Said compositions comprising a therapeutically effective amount of a compound according to formula (I) and a pharmaceutically acceptable carrier or diluent.

While it is possible for the active ingredient to be administered alone, it is preferable to present it as a pharmaceutical composition. Accordingly, the present invention further provides a pharmaceutical composition comprising a

compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy. A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The exact dosage and frequency of administration depends on the particular compound of Formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

Depending on the mode of administration, the pharmaceutical composition will comprise from 0.05 to 99% by weight, preferably from 0.1 to 70% by weight, more preferably from 0.1 to 50% by weight of the active ingredient, and, from 1 to 99.95% by weight, preferably from 30 to 99.9% by weight, more preferably from 50 to 99.9% by weight of a pharmaceutically acceptable carrier, all percentages being based on the total weight of the composition.

The present compounds can be used for systemic administration such as oral, percutaneous or parenteral administration; or topical administration such as via inhalation, a nose spray, eye drops or via a cream, gel, shampoo or the like. The compounds are preferably orally administered. The exact dosage and frequency of administration depends on the particular compound according to Formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The amount of a compound of Formula (I) that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300 mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being

treated; the time and route of administration; the rate of excretion; other drugs that have previously been administered; and the severity of the particular disease undergoing therapy, as is well understood by those of skill in the area.
A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

For the compositions, methods and kits provided above, one of skill in the art will understand that preferred compounds for use in each are those compounds that are noted as preferred above. Still further preferred compounds for the compositions, methods and kits are those compounds provided in the non-limiting Examples below.

## EXPERIMENTAL PART

Hereinafter, the term "m.p." means melting point, "min" means minutes, "aq." means aqueous, "r.m." or "RM" means reaction mixture, "r.t." or "RT" means room temperature, "rac" or "RS" means racemic, "sat." means saturated, "SFC" means supercritical fluid chromatography, "SFC-MS" means supercritical fluid chromatography/mass spectrometry, "LC-MS" means liquid chromatography/mass spectrometry, "HPLC" means high-performance liquid chromatography, "RP" means reversed phase, "UPLC" means ultra-performance liquid chromatography, "DAD" "means Diode Array Detector, "DSC" means differential scanning calorimetry, "SQD" means Single Quadrupole Detector, "QTOF" means Quadrupole-Time of Flight, "BEH" means bridged ethylsiloxane/silica hybrid, "CSH" means charged surface hybrid "$R_t$" means retention time (in minutes), "$[M+H]^+$" means the protonated mass of the free base of the compound, "wt" means weight, "LiHMDS" means lithium bis(trimethylsilyl)amide, "M" means molar, "THF" means tetrahydrofuran, "TMSCl" means trimethylsilyl chloride, "EtOAc" means ethyl acetate, "MeCN" means acetonitrile, "BuLi" means butyl lithium, "h" means hours, "$Et_2O$" means diethyl ether, "DCM" means dichloromethane, "KF" means potassium fluoride, "MTBE: means methy tert-butyl ether, "$BH_3$·THF" means borane-tetrahydrofuran complex, "MeOH" means methanol, "$Et_3N$" means triethylamine, "org." means organic, "OL" means organic layer, "N" means normal, "MeI" means iodomethane, "AcCl" means acetyl chloride, "$O_3/O_2$" means ozone/oxygen mixture, "sol." means solution, "BOC" means tert-butoxycarbonyl, "TLC" means thin layer chromatography, "Pd/C" means palladium on carbon, "$AlMe_3$" means trimethyl aluminium, "EtOH" means ethanol, "$iPrNH_2$"means isopropylamine, "DIPE" means diisopropyl ether, "$NH_4Ac$" means ammonium acetate, "iPrOH" means isopropanol, and "EDCI" means 1-ethyl-3-(3-dimethylaminopropyl)carbodiimide, "Sudan III" means 1-(4-(phenyldiazenyl)phenyl) azonaphthalen-2-ol, "DIBAL" means diisobutylaluminium hydride, "TFA" means trifluoroacetic acid, "ACN" means acetonitrile, "NP" means normal phase.

Whenever the notation "RS" is indicated herein, it denotes that the compound is a racemic mixture at the indicated centre, unless otherwise indicated. The stereochemical configuration for centres in some compounds has been designated "R" or "S" when the mixture(s) was separated; for some compounds, the stereochemical configuration at indicated centres has been designated as "**R*" or "**S*" when the absolute stereochemistry is undetermined although the compound itself has been isolated as a single stereoisomer and is enantiomerically/diastereomerically pure. The enantiomeric excess of compounds reported herein was determined by analysis of the racemic mixture by supercritical fluid chromatography (SFC) followed by SFC comparison of the separated enantiomer(s).

The absolute configuration of chiral centres (indicated as R and/or S) can be rationalized. The synthesis of all final compounds started from intermediates of known absolute configuration in agreement with literature precedent (e.g. intermediate 20) or obtained from appropriate synthetic procedures (e.g. the formation of Ellman's sulfonamide in intermediate 3). The assignment of the absolute configuration of additional stereocentres could then be assigned by standard NMR methods.

## A. PREPARATION OF THE INTERMEDIATES

### EXAMPLE A1

### PREPARATION OF INTERMEDIATE 1

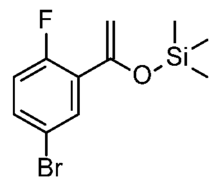

A mixture of LiHMDS (1M in THF, 719 mL, 719 mmol) in THF (2 L) was stirred at -78 °C for 30 min. 5'-bromo-2'-fluoroacetophenone (120 g, 553 mmol) was added dropwise at -78 °C over 40 min, then the r.m. was stirred at -78 °C for 10 min. TMSCl (78 g, 719 mmol) was added at -78 °C over 40 min followed by stirring of the r.m. at 25 °C for 30 min. $NH_4Cl$ in ice/water and EtOAc were added. The mixture was extracted with EtOAc, the org. layer dried, filtered and concentrated *in vacuo* to afford the crude product (155 g, 90% purity, 87%).

PREPARATION OF INTERMEDIATE 66

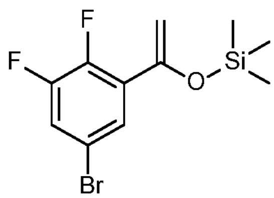

1-66 was synthesized following an analogous procedure to that described for the preparation of I-1, starting from 1- (5-bromo-2, 3-difluorophenyl) -ethanone [1600511-63-4].

EXAMPLE A2

PREPARATION OF INTERMEDIATE 2

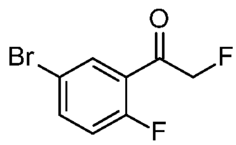

Intermediate 1 (310 g, 1.071 mol) in MeCN (1.5 L) was cooled to 5 °C. Selectfluor™ (417.4 g, 1.178 mol) was added portion wise and the r.m. was stirred at 5 °C for 5 min, then at 25 °C for 80 min. Volatiles were removed *in vacuo* and the residual partitioned between EtOAc and water. The org. layer was separated, washed with brine, dried, filtered and concentrated. The residue was purified by column chromatography (silica; petroleum ether/EtOAc 100/ 0 to 20/1) to afford the desired product (242 g, 90% purity, 85%).

Alternative method (1) for the preparation of intermediate 2

BuLi (2.5 M in hexanes, 20 mL, 50 mmol) was added dropwise to a sol. of diisopropylamine (7 mL, 50 mmol) in THF (125 mL) at - 70 °C under nitrogen. After 30 min 4-bromofluorobenzene (5 mL, 45.5 mmol) was added dropwise and the r.m. was stirred for 30 min at -70 °C. Ethyl fluoroacetate (5.3 mL, 54.6 mmol) was then added and the r.m. was stirred at -70 °C for 1 h. The reaction was quenched by pouring it onto $NH_4Cl$ sat. sol. The product was extracted with $Et_2O$, the org. layer was separated, dried ($MgSO_4$), filtered and concentrated *in vacuo.* The crude product was purified by flash column chromatography (silica; heptane/DCM 100/0 to 0/100). The desired fractions were collected and the solvents evaporated *in vacuo* to yield the product as a pale yellow solid (5.1 g, 48%).

Alternative method (2) for the preparation of intermediate 2

18-crown-6 (185 mg, 0.7 mmol) was added to a stirred mixture of KF (1.182 g, 20 mmol) in MeCN (4 mL) at r.t. The r.m. was stirred at 90 °C for 30 min and then 5-bromo-2-fluorophenacyl bromide (2.959 g, 10 mmol) was added portion wise. The r.m. was stirred at 90 °C for 18 h, then allowed to reach r.t. and the solvent was removed *in vacuo.* The residue was partitioned between water and EtOAc, the org. layer was dried ($Na_2SO_4$), filtered and concentrated *in vacuo.* The residue was purified by flash column chromatography (silica; heptane/DCM 100/0 to 60/40). The desired fractions were collected and concentrated *in vacuo* to give the desired product (1.1 g, 47%).

PREPARATION OF INTERMEDIATE 67

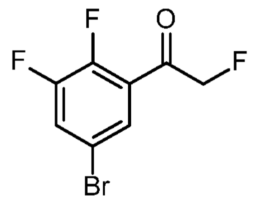

1-67 was synthesized following an analogous procedure to that described for the preparation of intermediate 2 (first method), starting from 1-66 (13.2 g, 42.94 mmol) in 43% yield.

EXAMPLE A3

PREPARATION OF INTERMEDIATE 3

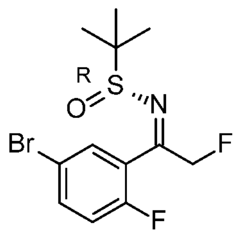

Intermediate 3 was prepared according to a procedure similar to the one described in WO 2014/134341 A1 starting from intermediate 2. The stereochemistry of the double bond was not determined at this stage.

PREPARATION OF INTERMEDIATES 68-70

Intermediates 68-70 were prepared in an analogous manner to 1-3 from the indicated starting materials:

| Intermediate | Starting material |
|---|---|
| 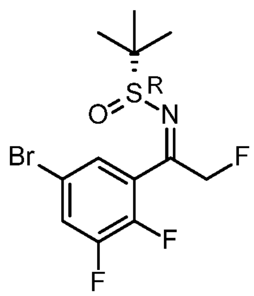 I-68 | I-67 |

(continued)

| Intermediate | Starting material |
|---|---|
| 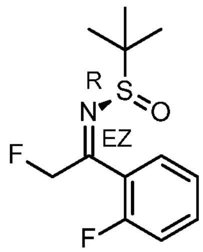 I-69 | 2-fluoro-1-(2-fluorophenyl)-ethanone |
| 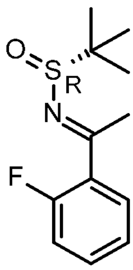 I-70 | 1-(2-fluorophenyl)-ethanone |

EXAMPLE A4

PREPARATION OF INTERMEDIATE 6

Intermediate 6 can be prepared by following either of the three-step procedures reported below.

PROCEDURE 1:

1) PREPARATION OF INTERMEDIATE 4

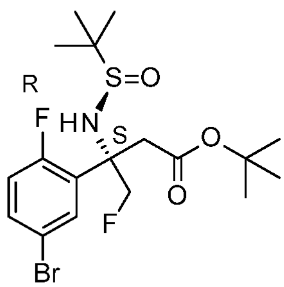

A mixture of diisopropylamine (2.69 g, 26.6 mmol) in THF (50 mL) was stirred at -78 °C under nitrogen. BuLi (2.5 M in hexanes, 10.6 mL, 26.6 mmol) was added dropwise and the r.m. was stirred at -78 °C for 15 min. Tert-butyl acetate (3.09 g, 26.6 mmol) was added dropwise and the mixture was stirred for 30 min. A sol. of intermediate 3 (5 g, 13.3 mmol) in THF (50 mL) was added dropwise and the mixture stirred at -78 °C for 3 h until TLC showed completion of the reaction. Quenching with sat. $NH_4Cl$ sat. sol. was followed by extraction with EtOAc. The org. layer was dried ($Na_2SO_4$), filtered and concentrated to give a crude material which was purified by column chromatography (silica; petroleum ether/EtOAc 50/1 to 10/1) to give the desired product as an oil (4 g, 90% purity, 60%).

2) PREPARATION OF INTERMEDIATE 5

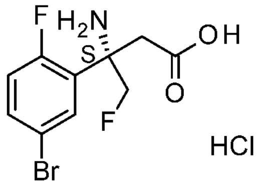

Intermediate 4 (200 g, 396.15 mmol) was dissolved in HCl in dioxane (4 M, 1.5 L). The r.m. was stirred for 5 h at 80 °C until LC-MS showed completion of the reaction, then it was cooled to r.t. The solid was collected, washed with MTBE (2x1 L) and dried *in vacuo* to give intermediate 5 as a HCl salt (130 g, 93% pure, 92%).

3) PREPARATION OF INTERMEDIATE 6

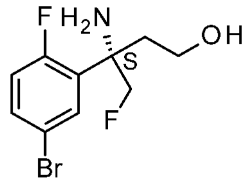

A mixture of intermediate 5 (70 g, 211.8 mmol) in THF (120 mL) was stirred at 0 °C under nitrogen. $BH_3$.THF (1 M, 800 mL, 800 mmol) was added dropwise, the reaction was stirred at r.t. for 3 h and then poured into $NaHCO_3$ (200 g) and ice (2 kg). EtOAc (1 L) was added, the mixture was stirred at r.t. and then extracted with EtOAc. The org. layer was dried ($Na_2SO_4$). Removal of the volatiles *in vacuo* afforded the desired compound (55 g). This batch was purified by column chromatography and crystallized with hydrochloric acid prior to use (see for instance, herein below).

PROCEDURE 2:

1) PREPARATION OF INTERMEDIATE 7

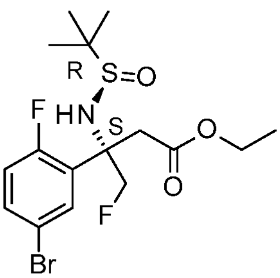

A 3-neck 250 mL round-bottom flask equipped with a reflux condenser and an addition funnel was thoroughly dried and then charged with Zn dust (11.6 g, 177.4 mmol) and CuCl (1.756 g, 17.74 mmol). The two solids were mixed under a slow stream of nitrogen. Dry THF (40 mL) was added to produce a dark slurry. The resulting r.m. was heated to reflux and stirred vigorously for 30 min. The heating bath was then removed while maintaining vigorous stirring and the addition funnel was charged with ethyl bromoacetate (4.92 mL, 44.35 mmol) in dry THF (20 mL). The sol. was slowly and carefully added dropwise until reflux of the solvent started. The addition was then continued at a rate that maintained a controllable reflux. Once addition was complete, the r.m. was stirred for additional 30 min at r.t. and then at 50 °C for 30 min. After cooling of the mixture to 0 °C, the addition funnel was charged with intermediate 3 (6 g, 17.74 mmol) and dry THF (20 mL). This sol. was then added dropwise to the r.m., which was stirred for additional 4 h at 0 °C. Subsequent filtration through a pad of Celite® and washing of the Zn and of the filter pad with $Et_2O$ (x2) gave a mixture which was washed with 0.25 M aq. HCl, then with $NaHCO_3$ sat. sol. (x2) and finally with brine. After drying ($Na_2SO_4$), and concentration *in vacuo* the residue was purified by flash column chromatography (silica; DCM) to afford the desired product as a yellow oil (7.19 g, 95%).

2) PREPARATION OF INTERMEDIATE 8

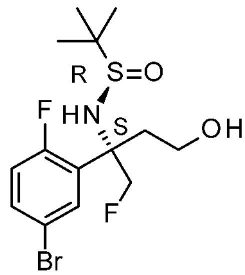

$LiBH_4$ (2M in THF, 1.81 mL, 3.626 mmol) was added dropwise to a sol. of intermediate 7 (773 mg, 1.813 mmol) in THF (8 mL) under nitrogen at 0 °C. The r.m. was stirred at 0 °C for 30 min, for 2 h at r.t and then carefully quenched with acetic acid (0.4 mL) in water (10 mL). EtOAc was added, the aq. layer extracted with EtOAc, the org. layer separated, dried ($MgSO_4$), filtered and concentrated *in vacuo* to give a crude used as such in the subsequent reaction step (696 mg, quantitative).

3) PREPARATION OF INTERMEDIATE 6

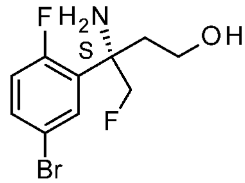

To a sol. of intermediate 8 (696 mg, 1.81 mmol) in MeOH (5.8 mL) was added HCl (4 M in dioxane, 5.8 mL) and the mixture was stirred at r.t. for 1 h, then concentrated *in vacuo* and co-evaporated with toluene to a crude which was used without further purification in the subsequent step (507 mg, quantitative).

A batch of 15 g of intermediate 6 was purified by flash column chromatography over silica gel using a gradient (DCM/7 N $NH_3$ in MeOH, 1:0 to 9:1). The product fractions were evaporated to provide a transparent glass which was taken up in 6 N HCl/ 2-propanol, evaporated to dryness and re-dissolved in 2-propanol. The resulting crystals were filtered and dried to yield intermediate 6 (13.5 g, as HCl salt).

4) PREPARATION OF INTERMEDIATE 71

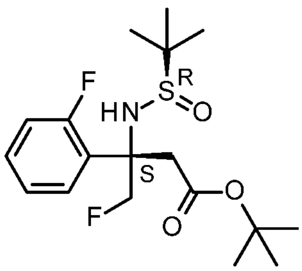

To a stirred solution of diisopropylamine (156.1 g, 1.54 mol) in THF (1 L) at -78 °C under $N_2$ was added dropwise n-BuLi (617.0 mL, 1.54 mol) and the mixture was stirred at -78 °C for 30 min, followed by dropwise addition of tert-butyl acetate 179.2 g, 1.54 mol) and the mixture was stirred for 30 min. Chlorotitanium triisopropoxide (1.54 L, 1.54 mol) was then added dropwise and the mixture was further stirred for 50 min.

A solution of I-69 (160 g, 0.62 mol) in THF was added dropwise and the mixture was stirred for 3 h. The reaction

was quenched with sat sol $NH_4Cl$ (2L) and extracted with EtOAc (3x 3 L). The combined org layers were dried ($Na_2SO_4$), filtered and evaporated to give a crude that was purified by column chromatography (silica gel; petroleum ether/EtOAc 5/1) to yield 1-71 (135 g, 53%) as a white solid.

5) PREPARATION OF INTERMEDIATE 72

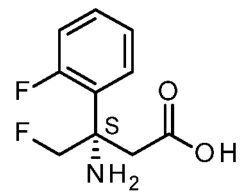

. HCl

1-72 was synthesized following an analogous procedure to that described for the preparation of I-5, starting from I-71.

6) PREPARATION OF INTERMEDIATE 73

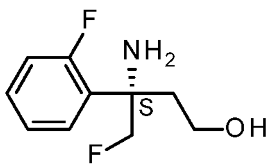

.HCl

1-73 was synthesized following an analogous procedure to that described for the preparation of I-6 (procedure 1), starting from I-72.

PREPARATION OF INTERMEDIATES 74-75

Intermediates 74-75 were prepared in an analogous manner to 1-7 from the indicated starting material:

| Intermediate | Starting material |
|---|---|
| 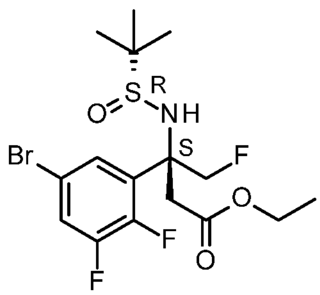 I-74 | I-68 |

(continued)

| Intermediate | Starting material |
|---|---|
| 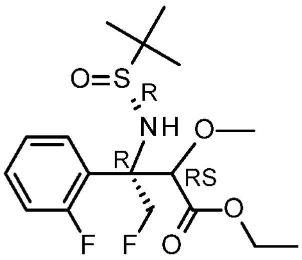 I-75 | I-69 |

Alternatively, 1-75 was synthesized as follows

To a solution of LDA (2M in THF/heptane/ethylbenzene, 100 mL, 200 mmol) in THF (450 mL) was added ethyl methoxyacetate (25 mL, 208.9 mmol) at -78 °C. The solution was stirred for 30 min, followed by dropwise addition of chlorotriisopropoxytitanium(IV) 200 mL, 200 mmol). The solution was stirred for another 30 min, followed by addition of a solution of I-69 (24.5 g, 79.5 mmol) in THF (450 mL) at -78°C. The solution was stirred at that temperature for 2 h. $NH_4Cl$ was added to the RM, which was stirred for 10 min, after which dicalite® was added and the RM was filtered and the filter cake was rinsed with EtOAc. The aq layer was separated and extracted with EtOAc, the combined org layers were dried ($MgSO_4$), concentrated and purified by flash column chromatography (filter filled with silica; heptane/EtOAc 8/2 to 3/7). The product containing fractions were concentrated and dried overnight in a vacuum oven at 55°C, yielding 1-75 (29.2 g, 97%, 78% pure) as an orange oil.

PREPARATION OF INTERMEDIATES 76-77

The following intermediates were prepared in an analogous manner to 1-8 from the indicated starting material:

| Intermediate | Starting material |
|---|---|
| 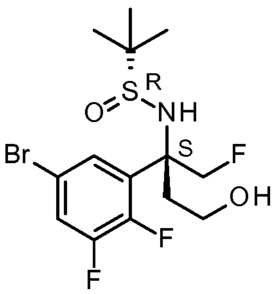 I-76 | I-74 |
| 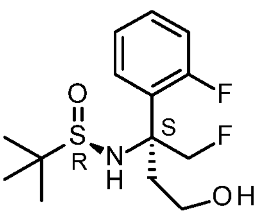 $.C_2H_4O_2$ I-77 | I-75 |

PREPARATION OF INTERMEDIATES 78 AND 73 (alternative procedure)

Intermediates 78 and 73 were prepared in an analogous manner to 1-6 (procedure 2) from the indicated starting material:

| Intermediate | Starting material |
|---|---|
| 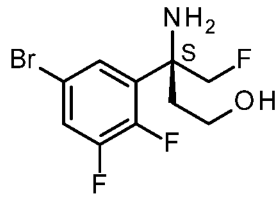 .HCl I-78 | I-76 |
| 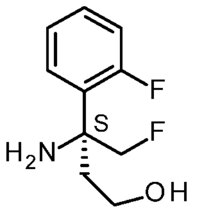 I-73 | I-77 |

PREPARATION OF INTERMEDIATE 79

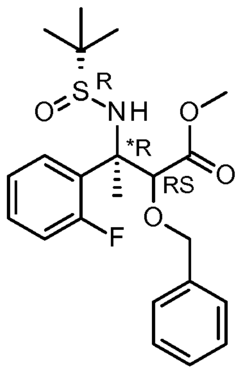

LDA (2M in THF/hexane, 8.57 g, 35.52 mmol) was added dropwise to a solution of methyl 2- (benzyloxy) acetate ([31600-43-8], 20 g, 88.79 mmol) in dry THF (on molecular sieves, 130 mL) at -78°C. The solution was stirred at -78 °C during 30 min. Chlorotitanium triisopropoxide (1M in hexane, 90 mL, 90 mmol) was added dropwise and the resulting mixture was further stirred 55 min at -78 °C. A solution of 1-70 (8.57 g, 35.52 mmol) in dry THF (60 mL) was added slowly to the solution, which was stirred at -78 °C during 10 min. Sat $NH_4Cl$ sol was added slowly. The mixture was filtered over celite® and washed with EtOAc. The org layer was separated, dried ($MgSO_4$), filtered and evaporated under reduced pressure to yield a residue that was purified by flash column chromatography (silica; EtOAc/heptane 10/90 to 60/40) to yield 1-79 (13.5 g, 90%, 26:74 mixture of diastereomers) as a yellow oil.

EXAMPLE A5

PREPARATION OF INTERMEDIATE 9

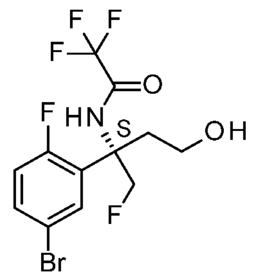

To a sol. of intermediate 6 (3.20 g, 11.42 mmol) in DCM (1.9 mL) at 0 °C was added $Et_3N$, followed by dropwise addition of trifluoroacetic anhydride. The resulting mixture was stirred at r.t. for 7 h until LC-MS showed almost full conversion. Water was added and the org. layer separated, washed with 1 N HCl and then with a mixture of brine and sat. aq. $NaHCO_3$. Drying ($MgSO_4$), filtration and evaporation of the solvent provided yellow crystals which were dried *in vacuo* (4.42 g, 96% purity, 99%).

Intermediate 9 was also prepared from intermediate 6 hydrochloride salt:

To a solution of intermediate 6.HCl (12.5 g, 40 mmol) and $Et_3N$ (11.5 mL, 83 mmol) in DCM (126.5 mL) at 0 °C was added trifluoroacetic anhydride (6.0 mL (43.4 mmol). The resulting mixture was stirred at rt overnight. 1 N HCl was added and the layers were separated, then 1N of NaOH was added and the layers were separated. The organic layer was washed with sat. aq. $NaHCO_3$ and then brine, dried ($MgSO_4$), filtered and evaporated to give intermediate 9 (11.5 g, 77%) as a white solid.

PREPARATION OF INTERMEDIATE 40

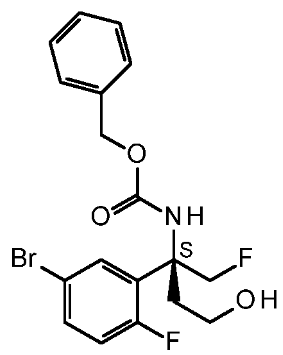

Intermediate 6 (35.5 g, 112 mmol) was dissolved in demineralized water (213 mL) in a three-neck round-bottom flask provided with mechanical stirring. Sodium carbonate (23.8 g, 224 mmol) was added and the mixture was cooled to 0 °C. Then benzyl chloroformate (24.0 mL, 168 mmol) was added and the reaction was stirred using mechanical stirring for 16 h. Next, the reaction was filtered. The aqueous layer was discarded. The filter cake was triturated with heptane, filtered and dried in vacuo to give intermediate 40 as a white solid (42.2 g, 91%).

PREPARATION OF INTERMEDIATES 80-81

Intermediates 80-81 were prepared in an analogous manner to 1-40 from the indicated starting material:

| Intermediate | Starting material |
| --- | --- |
| 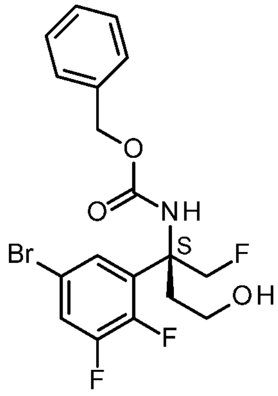 I-80 | I-78 |
| 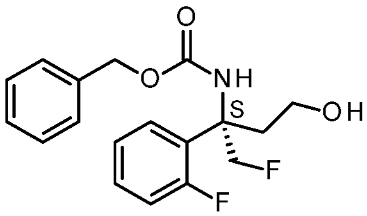 I-81 | I-73 |

EXAMPLE A6

PREPARATION OF INTERMEDIATE 10

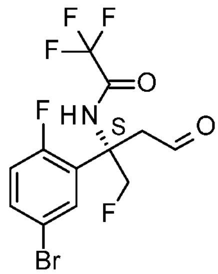

Dess-Martin periodinane (11.43 g, 26.95 mmol) was added portion wise to a sol. of intermediate 9 (4.4 g, 11.23 mmol) in DCM (80 mL). The reaction was stirred at r.t. After 4 h LC-MS showed partial conversion. Another 1.2 eq of Dess-Martin reagent were added portion wise and the mixture was stirred for 2 h. LCMS showed complete conversion. The reaction was treated with $NaHCO_3$ sat. sol. and then with a 10% sol. of $NaHSO_3$ with stirring overnight at r.t. The org. phase was then separated and the aq. phase extracted with DCM. The combined org. phases were dried ($MgSO_4$), filtered and evaporated. The residue was purified by flash column chromatography (silica; heptane/EtOAc 100/0 to 0/100). The product fractions were evaporated providing intermediate 10 as a transparent oil (3.74 g, 89%).

An additional batch of intermediate 10 was also synthesized as follows:

Dess-Martin periodinane (19.5 g, 46 mmol) was added portionwise to a solution of intermediate 9 (11.5 g, 29 mmol) in DCM (208.6 mL). The reaction was stirred at rt, then treated with sat. aq. $NaHCO_3$ and then with a 10% solution of $Na_2SO_3$ and stirred 2 h at rt. The organic phase was then separated and the aqueous phase extracted with DCM. The combined organic phases were dried ($MgSO_4$), filtered and evaporated. The residue was purified by flash column chromatography (silica gel, EtOAc in Heptane 0/100 to 40/60). The desired fractions were collected and evaporated in vacuo to yield intermediate 10 (10.7 g, 97%) as a yellowish creamy oil.

PREPARATION OF INTERMEDIATE 41

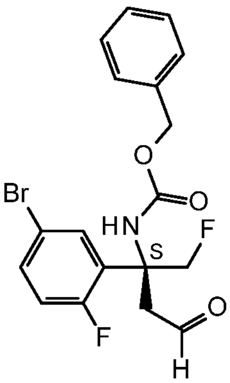

Dess-Martin periodinane (59 g, 140 mmol) was added portion wise to a solution of intermediate 40 (37 g, 89 mmol) in DCM (635 mL). The reaction was stirred at rt for 1 h 45 min. Next, the reaction was treated with aq. satd. $NaHCO_3$ (100 mL), then with aq. satd. $Na_2SO_3$ (100 mL), after which it was stirred 2 h at rt. The organic phase was then separated and the aqueous phase extracted with DCM. The combined organic phases were dried on $MgSO_4$, filtered and evaporated. The residue was purified by flash column chromatography (silica gel, EtOAc in Heptane, gradient: 0/100 to 40/60). The desired fractions were collected and evaporated in vacuo to yield intermediate 41 as a yellow creamy oil (27.5 g, 75%).

PREPARATION OF INTERMEDIATES 82-84 AND 112

Intermediates 82-84 and 112 were prepared in an analogous manner to 1-41 from the indicated starting material:

| Intermediate | Starting material |
|---|---|
| 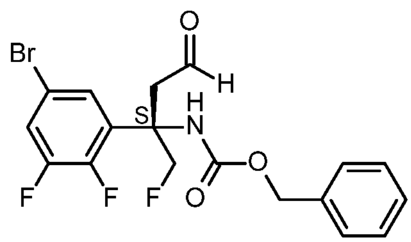 I-82 | I-80 |
| 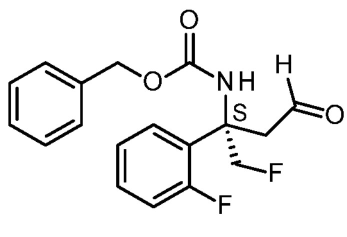 I-83 | I-81 |

(continued)

| Intermediate | Starting material |
|---|---|
| 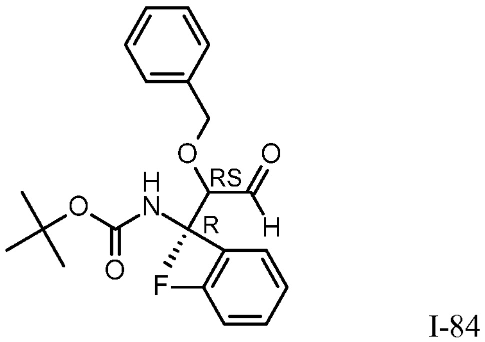 I-84 | I-97 |
| 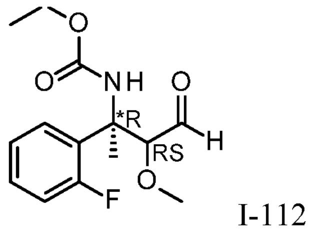 I-112 | I-111 |

EXAMPLE A7

PREPARATION OF INTERMEDIATE 11

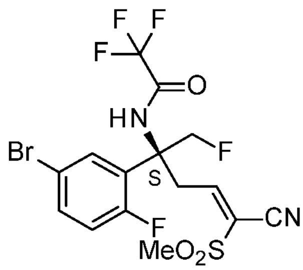

Intermediate 10 (3.73 g, 9.97 mmol) was dissolved in MeOH (56 mL). MgO (243 mg, 6.037 mmol) was added followed by 2-(methylsulfonyl)acetonitrile (1.425 g, 11.97 mmol). The r.m. was stirred for 1 h at r.t., then filtered over dicalite® and the solvent was evaporated *in vacuo*. The residued was purified by flash column chromatography (silica; heptane/EtOAc 100/0 to 0/100) to give a material used without further purification in the subsequent reaction step.

PREPARATION OF INTERMEDIATE 42

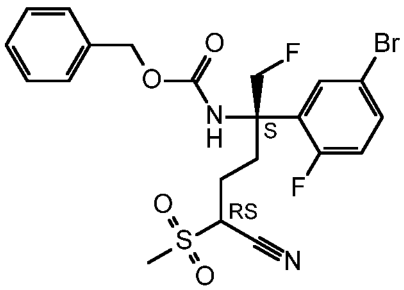

2-(Methylsulfonyl)acetonitrile (15.9 g, 133 mmol) and magnesium oxide (4.0 g, 100 mmol) were added to a solution of intermediate 41 (27.5 g, 66.7 mmol) in MeOH (344 mL). The mixture was stirred at 60 °C for 7 h. The reaction mixture was then allowed to reach rt, filtered over dicalite® and the solvent was evaporated under reduced pressure. The crude mixture was dissolved in THF (1.37 L), cooled to 0°C and $NaBH_4$ (2.52 g, 66.7 mmol) was added. The reaction mixture was stirred for 30 min after which HCl (1 N) was added until a pH of 7 was reached. Next, 10% $NaHCO_3$ solution (150 mL) and DCM (500 mL) were added. The organic layer was separated, dried with $MgSO_4$, filtered and concentrated. The residue was purified by column chromatography (silica gel, EtOAc in Heptane, gradient: 0/100 to 30/70). The desired fractions were evaporated to yield intermediate 42 as a white foam (28.0 g, 81%).

PREPARATION OF INTERMEDIATES 85-87

Intermediates 85-87 were prepared in an analogous manner to 1-42 from the indicated starting material:

| Intermediate | Starting material |
|---|---|
| 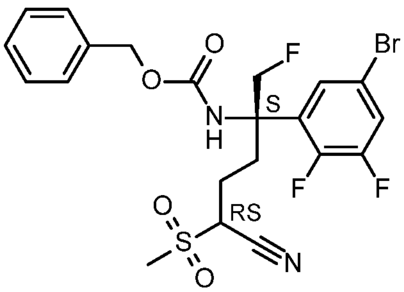 I-85 | I-82 |
| 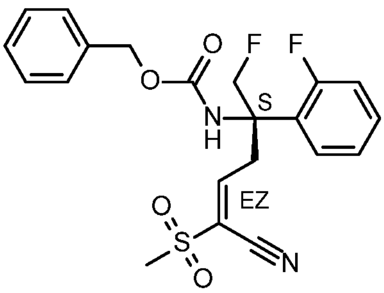 I-86 | I-83 |

(continued)

| Intermediate | Starting material |
|---|---|
| 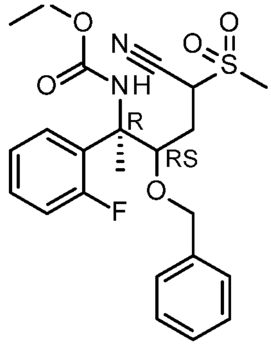 I-87 | I-84<br>N.B. reaction temperature 0 °C until addition of $NaBH_4$ (no solvent switch), then 10 °C |

EXAMPLE A8

PREPARATION OF INTERMEDIATE 12

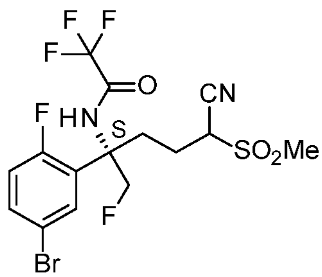

Intermediate 11 (2.63 g, 5.53 mmol) was stirred in THF (53 mL) and cooled to 0 °C. $NaBH_4$ (314 mg, 8.3 mmol) was added and the r.m. was stirred for 3 h at 0 °C. Ice, excess HCl (1 N) and water were added, followed by EtOAc. The ice-cold mixture was basified with NaOH (1 N), the org. layer was separated and the aq. layer extracted with EtOAc. The combined org. layers were dried ($MgSO_4$), filtered and the solvent removed *in vacuo.* The residue was purified by flash column chromatography (silica; DCM/(7 N $NH_3$ in MeOH) 100/0 to 90/10) to give intermediate 12 (678 mg, 57% purity, 15%).

PREPARATION OF INTERMEDIATE 88

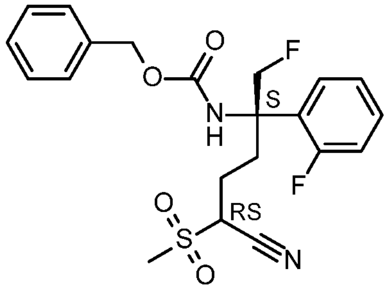

1-88 was prepared in an analogous manner to 1-12 from 1-86.

EXAMPLE A9

PREPARATION OF INTERMEDIATE 13

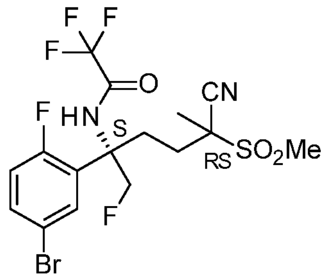

Intermediate 12 (830 mg, 1.74 mmol) was dissolved in THF (26 mL) and stirred under nitrogen atmosphere at 0 °C. NaH (60% suspension in mineral oil, 83 mg, 2.09 mmol) was added and the mixture stirred for 15 min at 0 °C. MeI (0.13 mL, 2.09 mmol) was finally added and the r.m. stirred for 2 h at 0 °C. The reaction was quenched with water and extracted with EtOAc. The org. layer was separated, dried ($MgSO_4$), filtered and the solvent removed *in vacuo.* The residue was purified by flash column chromatography (silica; DCM/MeOH 100/0 to 90/10). After evaporation of the solvent, intermediate 13 was obtained as a transparent glass (845 mg, 99%).

PREPARATION OF INTERMEDIATE 113

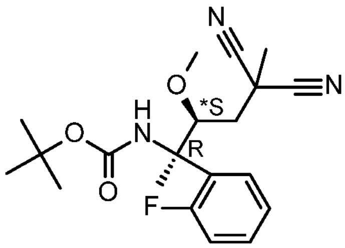

To a solution of I-121 (298 mg, 0.83 mmol) in THF (10 mL) were added $CH_3I$ (0.10 mL, 1.65 mmol) and $Cs_2CO_3$ (295.5 mg, 0.91 mmol) at 0 °C. The RM was stirred at 0 °C for 1.5 h. $NH_4Cl$ and EtOAc were added and the layers were separated. The org layer was extracted with EtOAc and the combined org layers were dried ($MgSO_4$) and concentrated to provide a residue that was purified by flash column chromatography (Redisep Gold, 24 g; heptane/EtOAc 100/0 to 60/40) to yield 1-113 (254 mg, 82%) as a colorless oil.

PREPARATION OF INTERMEDIATE 43

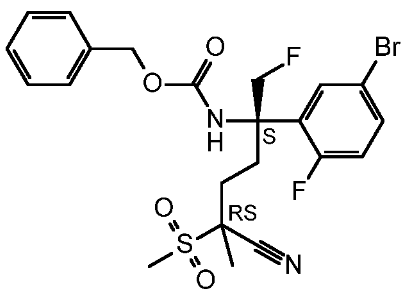

Intermediate 42 (28.0 g, 54.3 mmol) was dissolved in THF (808 mL) and stirred under $N_2$ atmosphere at 10

°C. NaH (60% dispersion in mineral oil, 3.91 g, 97.8 mmol) was added and stirring was continued for 15 min at 10 °C under $N_2$ atmosphere. Next $CH_3I$ (3.72 mL, 59.8 mmol) was added, again stirring was continued for 45 min at 10 °C. Next the reaction was quenched with $H_2O$ and extracted with EtOAc. The org layer was separated, dried with $MgSO_4$, filtered and the solvent was evaporated under reduced pressure. The crude mixture was purified by flash column chromatography (silica gel, EtOAc in Heptane, gradient: 0/100 to 30/70). The desired fractions were collected and evaporated in vacuo to yield intermediate 43 as a white foam (26.6 g, 92%).

PREPARATION OF INTERMEDIATES 89-91

Intermediates 89-91 were prepared in analogous manner to 1-43 from the indicated starting materials:

| Intermediate | Starting material |
|---|---|
| 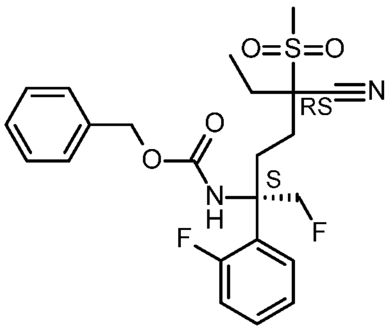 I-89 | I-88<br>$CH_3CH_2I$ (after addition, reaction mixture stirred for 20 h) |
| 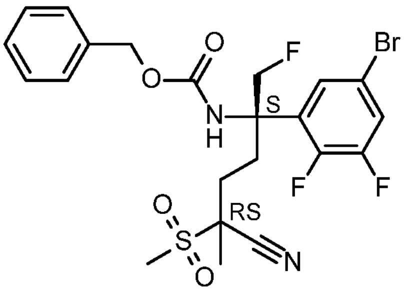 I-90 | I-85 |
| 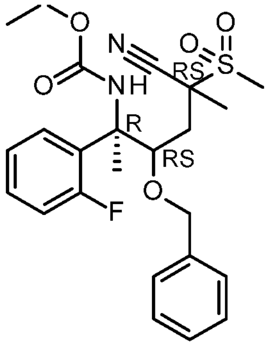 I-91 | I-87<br>N.B. reaction temperature 0 °C |

EXAMPLE A10

PREPARATION OF INTERMEDIATE 14

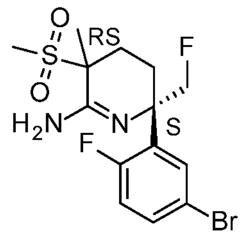

AcCl (1.5 mL, 21.1 mmol) was added to an ice-cooled sol. of MeOH (8 mL). Intermediate 13 (558 mg, 1.136 mmol) dissolved in MeOH (2 mL) was then added and the r.m. was heated for 32 h at 120 °C in a sealed Teflon® coated vessel. After cooling to r.t. the mixture was poured in an ice-cooled 1 N NaOH sol. and extracted with EtOAc (x3). The combined org. layers were dried ($MgSO_4$), filtered and the solvent evaporated *in vacuo.* The residue was purified by flash column chromatography (silica; DCM/(7 N $NH_3$ in MeOH) 100/0 to 90/10) to give a mixture of diastereoisomers (198 mg, 44%).

Alternative method for the preparation of intermediate 14

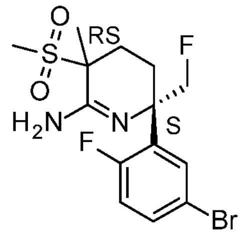

HCl (37% in water, 1.2 mL, 14.8 mmol) was added to a solution of intermediate 13 (391 mg, 0.80 mmol) in MeOH (1 mL) and the mixture was heated for 48 h at 130 °C, until LC-MS showed complete conversion. After cooling to r.t. the mixture was poured in an ice-cooled NaOH solution (1 N) and extracted with EtOAc (x3). The combined organic layers were dried ($MgSO_4$), filtered and the solvent was evaporated *in vacuo* to give a mixture of diastereoisomers.

Separation into intermediate 14a and intermediate 14b

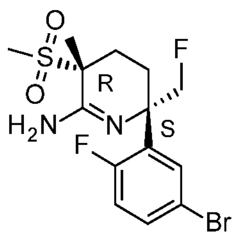

Intermediate **14a**

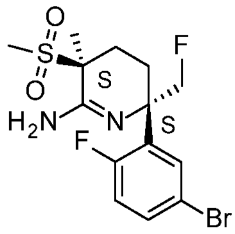

Intermediate **14b**

Intermediate 14 can be separated into diastereoisomers intermediate 14a (28-31% yield from intermediate 13) and intermediate 14b (36-37% yield from intermediate 13) by flash column chromatography (silica; heptane/EtOAc 100/0 to 0/100).

Alternative method for the preparation of intermediates 14a and 14b

HCl (9 M in $H_2O$, 44.8 mL) was added to a solution of intermediate 43 (6.4 g, 12.1 mmol) in acetic acid (34.6 mL) in a hastelloy (acid resistant) autoclave provided with mechanical stirring. The resulting slurry was heated 4 h at 140 °C internal temperature (180° jacket temperature). After cooling to rt, the reaction mixture was diluted with 1 N NaOH and EtOAc and stirred for 1 h at rt. Next the mixture was filtered and the remaining solid washed with EtOAc and water until no compound remained in the solid. The organic layer of the filtrate was separated and the aq. layer was extracted once more with EtOAc. The combined organic layers were washed with brine, dried over $MgSO_4$, filtered and evaporated. The residue was purified by column chromatography (silica gel, EtOAc in Heptane, gradient: 0/100 to 50/50). The desired fraction (first fraction) was collected and evaporated in vacuo to give intermediate 14a as a white solid (1.84 g, 38%). The second fraction was collected and evaporated to give intermediate 14b as a transparent glass (1.46 g, 31%).

PREPARATION OF INTERMEDIATES 92A AND 92B

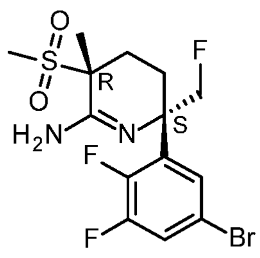

I-92a

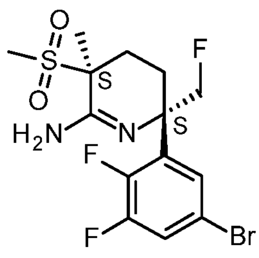

I-92b

Step 1: 1-90 (301 mg, 0.55 mmol) was dissolved in HBr (33% in AcOH, 44.5 mg, 0.55 mmol) under $N_2$ atmosphere at 0 °C and the resulting mixture was stirred for 1.5 h at 0 °C. The mixture was diluted with EtOAc and sat $NaHCO_3$ sol was added. The org layer was extracted, dried ($MgSO_4$), concentrated in vacuo and purified by column chromatography (silica gel; heptane/EtOAc 1/0 to 5/5). The product fractions were collected and evaporated yielding 207 mg (91%) of an orange oil.

Step 2: HCl (9M in $H_2O$, 1.85 mL, 16.6 mmol) was added to a solution of the oil isolated in step 1 (206 mg, 0.5 mmol) in AcOH (1.43 mL) in a microwave tube, and the mixture was stirred under microwave irradiation at 140 °C for 10 h. EtOAc and water were added to the mixture then $Na_2CO_3$ was added until pH basic. The org layer was separated and the aq layer was extracted once more with EtOAc. The combined organic layers were dried ($MgSO_4$), filtered and evaporated yielding a brown oil that was purified by via Prep HPLC (stationary phase: RP XBridge Prep C18 OBD-10μm,30x150mm; mobile phase: 0.25% $NH_4HCO_3$ solution in water, MeOH) yielding I-92a (20 mg, 10%) and I-92b (34 mg, 17%) as off-white solids.

PREPARATION OF INTERMEDIATE 93

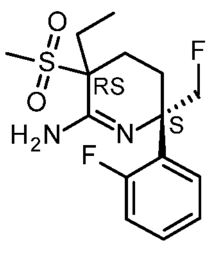

I-93

1-93 was synthesized following an analogous procedure to that described for the preparation of I-92a and b, starting from 1-89 (N.B. step 2 reaction time in microwave = 2 h).

EXAMPLE A11

PREPARATION OF INTERMEDIATE 15

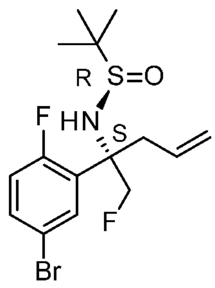

Allylmagnesium bromide (1 M in $Et_2O$, 25.1 mL, 25.1 mmol) was added dropwise to a sol. of intermediate 3 (5 g, 14.78 mmol) in DCM under nitrogen atmosphere at such a rate that the temperature was maintained at around -50 °C. After completion of the addition the reaction was stirred for 30 min at -50 °C and then allowed to reach -20 °C. $NH_4Cl$ 10% aq. sol. was added dropwise under nitrogen atmosphere. The org. layer was separated and the aq. layer was extracted with DCM.The combined org. layers were dried ($MgSO_4$), filtered and the solvent was removed *in vacuo* to give a crude purified by flash column chromatography (silica; heptane/EtOAc 100/0 to 50/50).

The product fractions were collected and the solvent was removed *in vacuo* to give intermediate 15 (3.6 g, 64%).

EXAMPLE A12

PREPARATION OF INTERMEDIATE 16

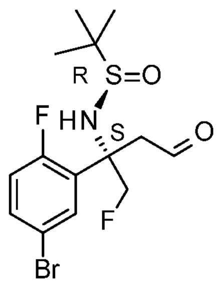

To a sol. of intermediate 15 (1.42 g, 3.734 mmol) in a mixture of acetone (150 mL) and water (7.5 mL) was added a small amount of Sudan III. The sol. was cooled to 0 °C and a mixture of $O_3/O_2$ was passed through the flask until the red color disappeared (2 h). The reaction was then purged with nitrogen for 2 min and diluted with water and DCM.

The org. layer was separated, dried ($MgSO_4$), filtered and the solvent removed *in vacuo* to yield intermediate 16 (1.2 g, 84%).

EXAMPLE A13

PREPARATION OF INTERMEDIATE 17

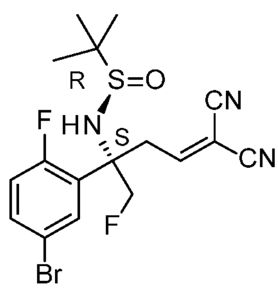

Intermediate 16 (2.05 g, 5.363 mmol) was dissolved in MeOH (30 mL). MgO (131 mg, 3.25 mmol) was added followed by methyl cyanoacetate (425 mg, 6.435 mmol) and the r.m. was stirred for 8 h at r.t. The mixture was then

filtered over dicalite®, washed with MeOH and the solvent removed *in vacuo.* The residue was purified by flash column chromatography (silica; heptane/EtOAc 100/0 to 50/50) to give intermediate 17 (2 g, 87%).

EXAMPLE A14

PREPARATION OF INTERMEDIATE 18

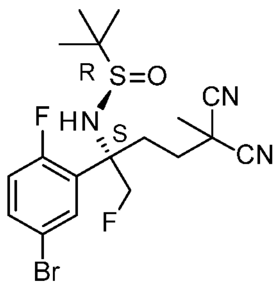

By following a synthetic route similar to the one described for the synthesis of intermediate 13, intermediate 18 was obtained starting from intermediate 17.

EXAMPLE A15

PREPARATION OF INTERMEDIATE 19

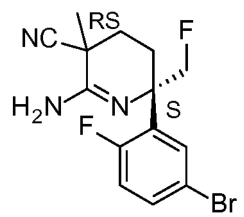

Intermediate 18 (380 mg, 0.851 mmol) was dissolved in formic acid (11.2 mL). The r.m. was heated for 12 h at 80 °C. The solvent was then removed *in vacuo* and the residue dissolved in DCM. $Na_2CO_3$ 10% aq. sol. was added until basification of the aq. layer. The org. layer was separated, dried ($MgSO_4$), filtered and the solvent evaporated. The residue was purified by flash column chromatography (silica; DCM/(7 N $NH_3$ in MeOH) 100/0 to 90/10) to yield intermediate 19 (160 mg, 55%).

PREPARATION OF INTERMEDIATES 94, 114 AND 115

Intermediates 94, 114 and 115 were prepared in analogous manner to 1-19 from the indicated starting materials:

| Intermediate | Starting material |
|---|---|
| 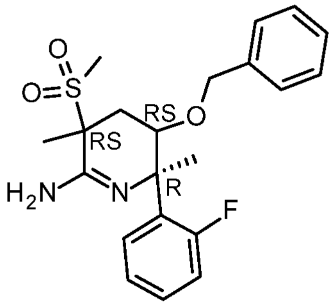 I-94 | I-91 (N.B. reaction performed at rt, 3h) |
| 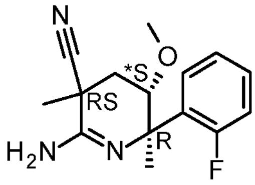 I-114 | I-113 (N.B. reaction temperature 45 °C) |
| 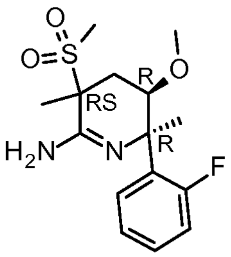 I-115 | I-117 (NB. Reaction temperature 50 °C) |

EXAMPLE A16

PREPARATION OF INTERMEDIATE 20

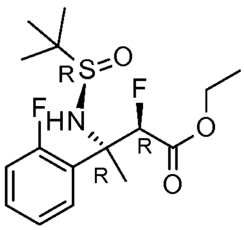

By following a synthetic route similar to the ones described in literature (see for example WO 2011138293 A1) intermediate 20 was obtained in two steps starting from commercially available 2'-fluoroacetophenone (CAS: 445-27-2). The stereochemistry was assigned based on data from literature.

EXAMPLE A17

PREPARATION OF INTERMEDIATE 21

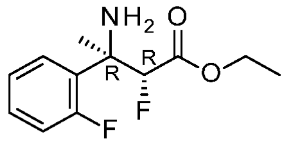

Intermediate 20 (789.43 g, 2.045 mol) was dissolved in MeOH (3.31 L) and then HCl (4 M in dioxane, 639 mL, 2.556 mol) was added over 60 min at 21 °C. The mixture was stirred for 30 min at r.t., then allowed to stand overnight. Then the solvent was evaporated and the residue taken up in 2 L of DCM. 2 L of water were added, the phases were separated and the org. layer was washed once more with HCl (1 N in water, 500 mL). The acidic aq. layers were stirred with DCM (2 L) and $NaHCO_3$ sat. sol. (3 L) was added until basicity was reached, then the pH was brought to pH 6-7 with 46 mL of concentrated HCl to break the emulsion and obtain distinct layers. The org. layer was separated, and the aqueous layer treated once more with DCM (600 mL) and $NaHCO_3$ sol. (100 mL). The combined org. layers were dried ($MgSO_4$), filtered and concentrated *in vacuo.* The crude material was used as such in the subsequent step (390 g, 78%).

PREPARATION OF INTERMEDIATE 95

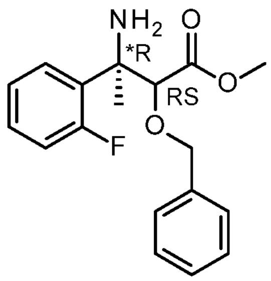

1-95 was synthesized following an analogous procedure to that described for the preparation of 1-21, starting from 1-79.

EXAMPLE A18

PREPARATION OF INTERMEDIATE 22

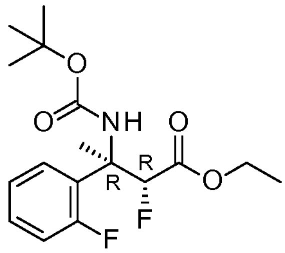

Intermediate 21 (26 g, 106.88 mmol) was dissolved in MeOH (116 mL), then BOC-anhydride (69.98 g, 320.65 mmol) was added. The mixture was stirred at 60 °C for 7 h, then the solvent was evaporated and the residue purified by flash column chromatography (silica; heptane/EtOAc 100/0 to 80/20). The product was obtained as a yellowish oil (36 g, 98%).

PREPARATION OF INTERMEDIATE 96

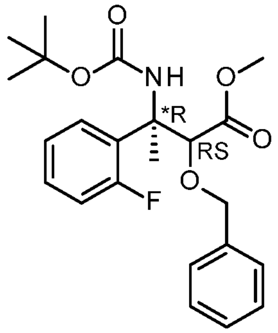

1-96 was synthesized following an analogous procedure to that described for the preparation of 1-22, starting from 1-95.

PREPARATION OF INTERMEDIATE 45

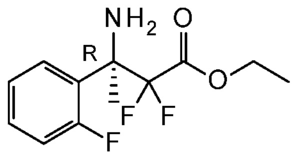

Ethyl (3*R*)-3-{[(*R*)-tert-butylsulfinyl]amino}-2,2-difluoro-3-(2-fluorophenyl)butanoate (15.1 g, 41.32 mmol, CAS: 1393802-40-8, described in US 2015/0232449 and WO 2012/110459) was dissolved in 67 mL MeOH, and then 4 M HCl in dioxane (20.7 mL, 82.65 mmol) was added. The mixture was stirred for 30 min at rt. The solvent was evaporated. The residue was taken up in water and DCM, some extra drops of HCl conc were added. The organic layer was extracted and washed once more with 1 N HCl. The organic layer was removed. The combined acidic aq. layers were diluted with DCM and $NaHCO_3$ aq. sat. solution was added until basic. The organic layer was separated, and the aq. layer was extracted once more with EtOAc. The combined organic layers were dried over $MgSO_4$, filtered and concentrated in vacuum to provide intermediate 45 (7.8 g, 29.86 mmol) as a yellow oil.

PREPARATION OF INTERMEDIATE 46

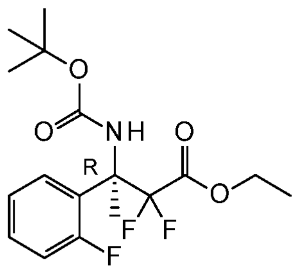

Intermediate 45 (7.6 g, 29.1 mmol) was dissolved in MeOH (33 mL) then BOC-anhydride (19.048 g, 87.3 mmol) was added. The mixture was stirred at 60 °C for 11 h then at r.t. for 11 h. The solvent was evaporated *in vacuo* and the crude product was purified by flash column chromatography (silica; heptane/EtOAc 100/0 to 80/20) to afford intermediate 46 (7.56 g, 74% purity, 71%).

EXAMPLE A19

PREPARATION OF INTERMEDIATE 23 AND INTERMEDIATE 24

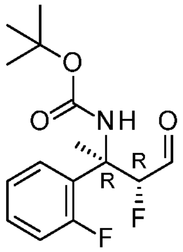

Intermediate 23

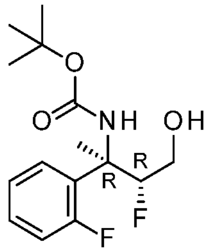

Intermediate 24

In a 5 L four-neck round-bottomed flask intermediate 22 (40 g, 116.492 mmol) was dissolved in DCM (400 mL) and the mixture was cooled to -78 °C. DIBAL (1 M in heptane, 384.4 mL, 384.4 mmol) was added dropwise. After completion of the addition LC-MS and TLC showed complete consumption of the starting material. Potassium sodium tartrate tetrahydrate (494 g, 1.75 mol) in water (1.8 L) and EtOAc (700 mL) were added and the mixture was stirred for 80 min. The phases were then separated and the aq. layer extracted once more with EtOAc. The org. layers were combined, dried ($MgSO_4$), filtered and the volatiles removed *in vacuo.* The crude was purified by flash column chromatography (silica; heptane/EtOAc 100/0 to 80/20) to afford intermediate 23 (26 g, 75%) and intermediate 24 (5 g, 14%).

Intermediate 24 could be converted into intermediate 23 by means of a procedure similar to the one reported for the synthesis of intermediate 10, in the presence of pyridine.

Alternative method for the preparation of intermediate 23

$LiBH_4$ (2 M in THF, 1.657 mL, 3.314 mmol) was added dropwise to a sol. of intermediate 22 (569 mg, 1.657 mmol) in THF (7.4 mL) under nitrogen at 0 °C. The mixture was stirred at 0 °C for 90 min until LC-MS showed complete conversion, then it was carefully quenched with acetic acid (0.4 mL in 10 mL of water). EtOAc was added, the org. layer was separated, dried ($MgSO_4$), filtered and concentrated *in vacuo* to give intermediate 23 as an off-white oil which was used as such in the subsequent step (499 mg, quantitative).

PREPARATION OF INTERMEDIATES 97 AND 116

Intermediates 97 and 116 were prepared in analogous manner to 1-23 (alternative method) from the indicated starting materials:

| Intermediate | Starting material |
|---|---|
| 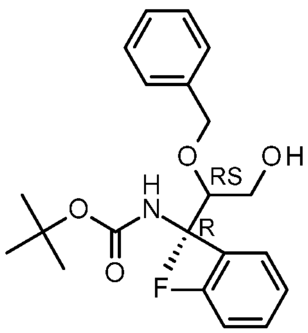 I-97 | I-96 |
| 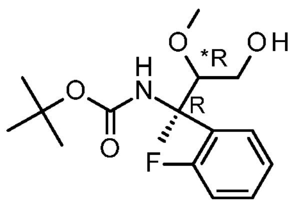 I-116 | I-106 |

PREPARATION OF INTERMEDIATE 47

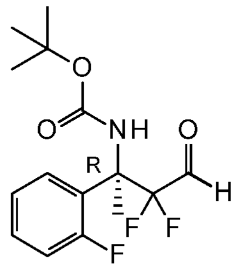

DIBAL (1 M in hexanes, 33.588 mL, 33.6 mmol) was added drop wise to a stirred solution of intermediate 46 (3.678 g, 10.2 mmol) in DCM (40 mL) at -78°C under nitrogen. The mixture was stirred for 2 h at -78 °C. The mixture was treated with a Rochelle's salt sat. sol. and diluted with EtOAc. The mixture was stirred at r.t. for 45 min. The mixture was treated with brine and extracted with EtOAc. The org. layer was separated, dried ($MgSO_4$), filtered and concentrated *in vacuo.* The crude product was purified by flash column chromatography (silica; heptane/EtOAc 100/0 to 60/40) to afford intermediate 47 (2.36 g, 100% purity, 73%).

EXAMPLE A20

PREPARATION OF INTERMEDIATE 25

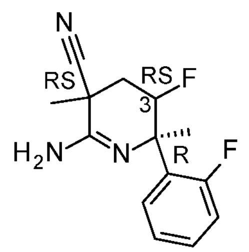

By following a synthetic route similar to the one described for the synthesis of (in the order) intermediate 17, intermediate 12, intermediate 13 and intermediate 19, intermediate 25 could be obtained starting from intermediate 23. Racemization at position 3 was observed.

EXAMPLE A21

PREPARATION OF INTERMEDIATE 26

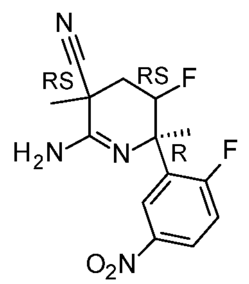

Intermediate 25 (320 mg, 1.215 mmol) was dissolved in $H_2SO_4$ (13.6 mL) and stirred at 0 °C. $KNO_3$ (135 mg, 1.337 mmol) was added, the reaction was stirred at 0 °C for 15 min and then diluted with DCM. The mixture was carefully

basified at 0 °C by using $Na_2CO_3$ aq. sol. and solid $Na_2CO_3$, the org. layer was separated, dried ($MgSO_4$), filtered and the solvent was removed *in vacuo* to give a solid used as such in the subsequent step (250 mg, 67%).

PREPARATION OF INTERMEDIATE 98

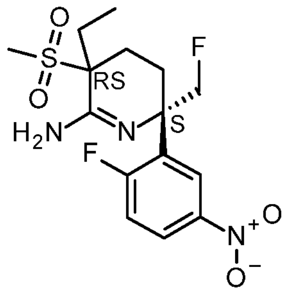

1-98 was synthesized following an analogous procedure to that described for the preparation of 1-26, starting from 1-93.

EXAMPLE A22

PREPARATION OF INTERMEDIATE 27

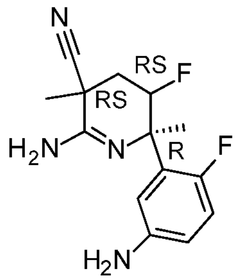

Pd/C (10% wt/wt, 86 mg, 0.08 mmol) was suspended in MeOH (30 mL) under nitrogen flow. Intermediate 26 (250 mg, 0.81 mmol) was added and the r.m. stirred for 3 h under hydrogen atmosphere, until LC-MS showed completion of the reaction.Filtration over dicalite® under nitrogen atmosphere and removal of the solvent *in vacuo* gave a crude which was purified by flash column chromatography (silica; DCM/(7 N $NH_3$ in MeOH) 100/0 to 90/10). The product fractions were collected and the solvent was removed *in vacuo* to give intermediate 27, used without further purification in the subsequent reaction (160 mg, 71%).

EXAMPLE A23

PREPARATION OF INTERMEDIATE 28

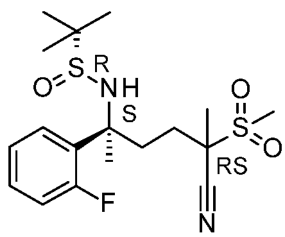

By following a synthetic route similar to the one described for the synthesis of (in the order) intermediate 15, intermediate 11, intermediate 12 and intermediate 31, intermediate 28 could be obtained starting from known (R,*E*)-N-(1-(2-fluorophenyl)ethylidene)-2-methylpropane-2-sulfinamide (CAS: 1312718-98-1).

PREPARATION OF INTERMEDIATE 50

Intermediate 50 can be prepared by following either of the three-step procedures reported below.

1) PREPARATION OF INTERMEDIATE 48

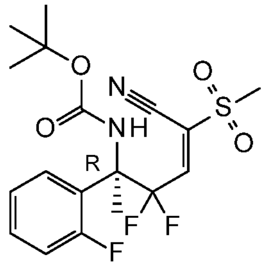

Intermediate 47 (2.36 g, 7.4 mmol) was stirred in MeOH (70 mL) at 60 °C. MgO (449.65 mg, 11.2 mmol) and $Ti(iPrO)_4$ (10.9 mL, 37.2 mmol) were added. 2-(methylsulfonyl)acetonitrile (2.658 g, 22.3 mmol) in THF (15 mL) was added. The solution was stirred at 60 °C for 2 h. The r.m. was filtered over dicalite®, rinsed with EtOAc and the filtrate was concentrated *in vacuo.* The residue was taken up in EtOAc and $H_2O$ then dicalite® was added and the suspension was filtered. The filtrate was extracted with EtOAc and the aqueous layer was extracted once more. The organic layer was dried ($MgSO_4$), filtered and concentrated *in vacuo.* Intermediate 48 was used as such in the subsequent step (3.052 g, 66% purity, 98%).

2) PREPARATION OF INTERMEDIATE 49

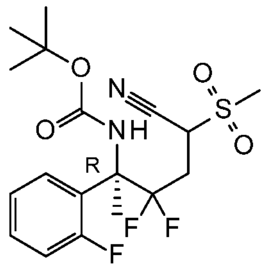

Intermediate 48 (3.052 g, 7.3 mmol) was dissolved in MeOH (10 mL) and THF (50 mL), stirring at 10 °C. Sodium cyanoborohydride (458.36 mg, 7.3 mmol) was added and stirred for 2.5 h at 10 °C. $NH_4Cl$ sat. sol. and EtOAc were added. The org. layer was separated, dried ($MgSO_4$), filtered and concentrated *in vacuo.* The crude product was purified by flash column chromatography (silica; n-heptane/EtOAc 100/0 to 60/40) to afford intermediate 49 (1.48 g, 100% purity, 48%).

3) PREPARATION OF INTERMEDIATE 50

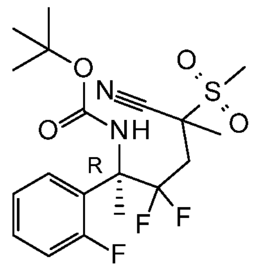

Intermediate 49 (1.48 g, 3.5 mmol) was dissolved in THF (40 mL), stirred under a $N_2$ atmosphere at 0°C. NaH (323.811 mg, 8.1 mmol) was added, stirred for 15 min at 0°C under a $N_2$ atmosphere. MeI (0.4 mL, 6.0 mmol) was added, stirred for 2.5 h at 0°C. $H_2O$ and EtOAc were added. The org. layer was separated, dried ($MgSO_4$), filtered and concentrated *in vacuo.* Intermediate 50 was used as such in the subsequent step (1.680 g, 83% purity, 110%).

PREPARATION OF INTERMEDIATE 117

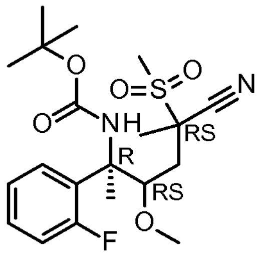

Intermediate 117 was obtained by applying a three-step procedure analogous to that reported for the synthesis of I-50 starting from 1-112.

EXAMPLE A24

PREPARATION OF INTERMEDIATE 29

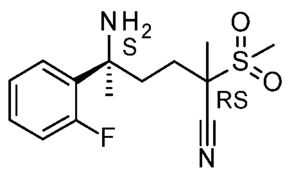

Intermediate 29 was obtained by applying to intermediate 28 the conditions reported for the synthesis of intermediate 19 (quantitative).

PREPARATION OF INTERMEDIATE 51

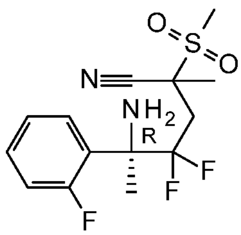

Intermediate 50 (1.680 g, 3.9 mmol) was dissolved in formic acid (21 mL) and stirred for 2 h at r.t.. The solution was brought to 80 °C for 5 h. It was stirred at r.t. overnight. The solution was concentrated *in vacuo.* The residue was dissolved in DCM and washed with $NaHCO_3$ sat. sol., the org. layer was separated, dried ($MgSO_4$), filtered and concentrated in *vacuo.* The r.m. was purified with flash column chromatography (silica; DCM/MeOH.$NH_3$ 100/0 to 96/4) to afford intermediate 51 (778 mg, 93% purity, 60%).

EXAMPLE A25

PREPARATION OF INTERMEDIATE 30

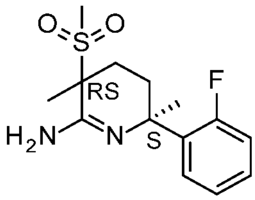

Intermediate 29 (700 mg, 2.346 mmol) was dissolved in DCM (40.6 mL) at r.t. and $AlMe_3$ (2 M in toluene, 3.52 mL, 7.038 mmol) was added. The r.m. was stirred for 16 h, then diluted with $Na_2CO_3$ sat. sol. The aqueous layer was extracted with EtOAc (x3), the combined org. layers were dried ($MgSO_4$), filtered the solvent removed *in vacuo.* The crude was purified by flash column chromatography (silica; DCM/(7 N $NH_3$ in MeOH) 100/0 to 90/10). After evaporation of the solvent intermediate 30 was obtained as a light yellow oil.

PREPARATION OF INTERMEDIATE 52

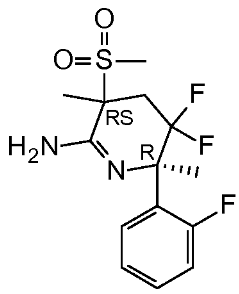

Intermediate 51 (314 mg, 0.1 mmol) was dissolved in toluene (6 mL) and the solution was stirred at 50 °C under nitrogen. Trimethyl aluminium (1.409 mL, 2 M in toluene, 2.8 mmol) was added and stirred for 1.5 h. The mixture was diluted with DCM and quenched with $Na_2CO_3$ sat. sol. The org. layer was separated and the aqueous layer was extracted with DCM. The combined org. layers were dried ($MgSO_4$), filtered and concentrated *in vacuo.* The crude product was purified by flash column chromatography (silica; DCM/7 N $NH_3$ in MeOH, 100/0 to 98/2) to afford intermediate 52 (184 mg, 100% purity, 59%).

EXAMPLE A26

PREPARATION OF INTERMEDIATE 31

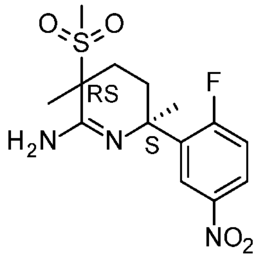

By following a synthetic procedure similar to the one described for the synthesis of intermediate 26, intermediate 31 was obtained starting from intermediate 30 (94%).

PREPARATION OF INTERMEDIATE 53

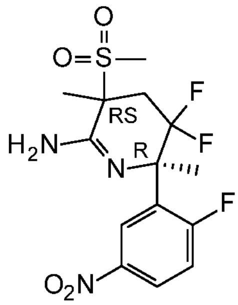

Intermediate 52 (239 mg, 0.7 mmol) was dissolved in TFA (1.6 mL) at 0 °C then cooled to -10 °C. $H_2SO_4$ (0.381 mL, 7.1 mmol) was added and the solution was stirred at -10 °C. $KNO_3$ (79.495 mg, 0.8 mmol) was added slowly and the mixture was stirred for 10 min. The r.m. was poured into an ice/$NH_3$/EtOAc emulsion (2 mL). The org. layer was separated, dried ($MgSO_4$), filtered and concentrated *in vacuo.* Intermediate 53 was used as such in the subsequent step (285 mg, 93% purity, quant.).

PREPARATION OF INTERMEDIATES 118-119

Intermediates 118-119 were prepared in analogous manner to I-53 from the indicated starting materials:

| Intermediate | Starting material |
|---|---|
| 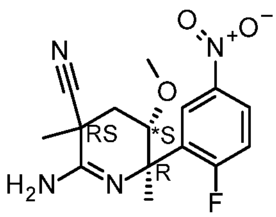 I-118 | I-114 |

(continued)

| Intermediate | Starting material |
|---|---|
| 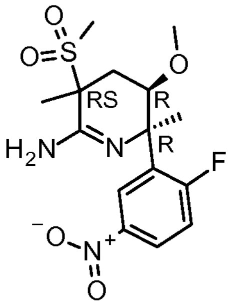 I-119 | I-115 |

EXAMPLE A27

PREPARATION OF INTERMEDIATE 32

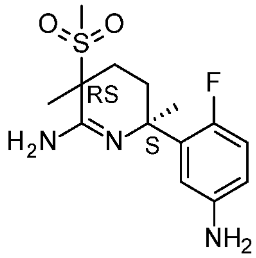

To the sol. of intermediate 31 (465 mg, 1.355 mmol) in THF (18 mL) and EtOH (3.6 mL) was added $SnCl_2$ (2.57 g, 13.55 mmol). The r.m. was stirred at 55 °C for 70 min, then the solvent was removed *in vacuo* and the crude partitioned between EtOAc and $Na_2CO_3$ sat. sol. The aq. layer was further extracted with DCM, the combined org. layers washed with brine and water, dried ($MgSO_4$) and concentrated *in vacuo* to give intermediate 32 as a white solid which was used without further purification in the subsequent step (471 mg, quantitative).

PREPARATION OF INTERMEDIATE 99

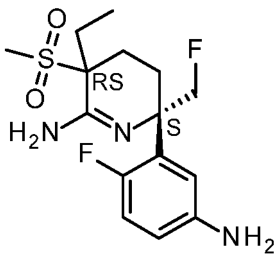

1-99 was synthesized following an analogous procedure to that described for the preparation of 1-32, starting from 1-98.

PREPARATION OF INTERMEDIATE 54

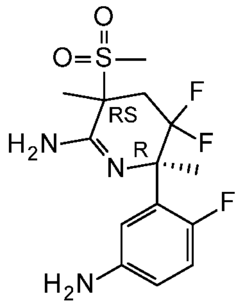

To a solution of intermediate 53 (285 mg, 0.751 mmol) in THF (9 mL) and EtOH (2 mL) was added stannous chloride (1.425 g, 7.5 mmol). The colourless solution was stirred at 55 °C for 2.5 h. The solution was treated with $Na_2CO_3$ sat. sol. The aq. layer was extracted with EtOAc. The combined org. layers were washed with brine and water, dried ($MgSO_4$), filtered and concentrated *in vacuo.* The crude product was purified by flash column chromatography (silica; DCM/7 N $NH_3$ in MeOH from 100/0 to 98/2) to afford intermediate 54 (113 mg, 98% purity, 43%).

PREPARATION OF INTERMEDIATES 100 AND 120

Intermediates 100 and 120 were prepared in analogous manner to I-54 from the indicated starting materials:

| Intermediate | Starting material |
|---|---|
| 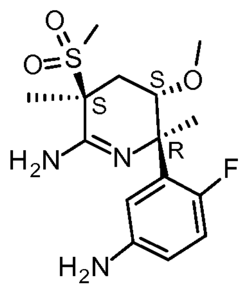 I-100 | I-101 |
| 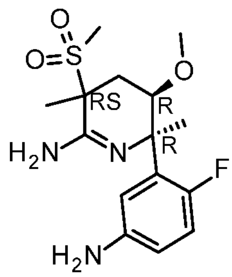 I-120 | I-119 |

EXAMPLE A28

PREPARATION OF INTERMEDIATE 33

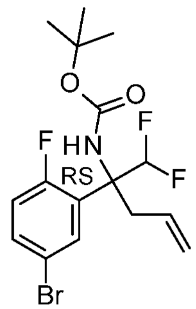

By following a synthetic procedure similar to the one described for the synthesis of intermediate 15, intermediate 33 was obtained starting from known tert-butyl N-[1-(5-bromo-2-fluoro-phenyl)-2,2-difluoro-ethylidene]carbamate (CAS: 1262858-99-0) (89%).

EXAMPLE A29

PREPARATION OF INTERMEDIATE 34

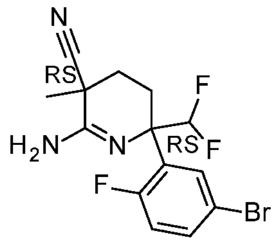

By following a synthetic route similar to the one described for the synthesis of (in the order) intermediate 16, intermediate 17, intermediate 18, and intermediate 19, intermediate 34 could be obtained starting from intermediate 33.

EXAMPLE A30

PREPARATION OF INTERMEDIATE 35

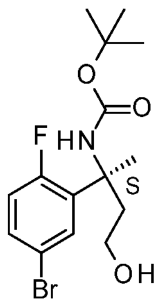

(3S)-3-Amino-3-(5-bromo-2-fluorophenyl)butan-1-ol (CAS: 1194044-45-5, 10 g, 38.15 mmol) was stirred in THF (174 mL). BOC-anhydride (15 g, 68.67 mmol) was added, together with $NaHCO_3$ aq. sol. (80 mL), and the r.m. was stirred overnight at r.t. DCM was then added, the org. layer was separated, dried ($MgSO_4$), filtered and the solvent was removed *in vacuo*. The residue was purified by column chromatography (silica; heptane/EtOAc, 100/0 to 50/50). The product fractions were collected and the solvent was removed *in vacuo* to give intermediate 35 (13.5 g, 98%).

EXAMPLE A31

PREPARATION OF INTERMEDIATE 36

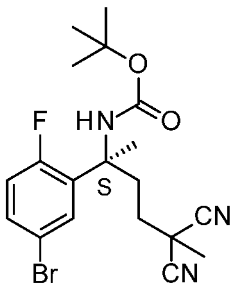

By following a synthetic route similar to the one described for the synthesis of (in the order) intermediate 10, intermediate 17, intermediate 12 and intermediate 13, intermediate 36 could be obtained starting from intermediate 35.

EXAMPLE A32

PREPARATION OF INTERMEDIATES 37 AND 38

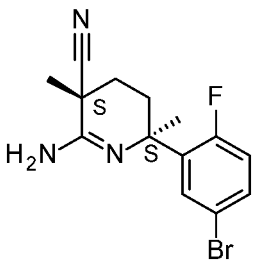

Intermediate **37**

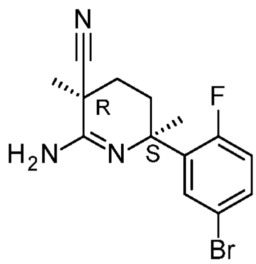

Intermediate **38**

Intermediate 36 (1.5 g, 3.535 mmol) was stirred in formic acid (25 mL) for 3 hours at r.t. After this time LC-MS analysis showed BOC-cleavage. The r.m. was evaporated *in vacuo* at 70 °C. Additional 10 mL of formic acid were added and a new evaporation of the solvent *in vacuo* at 70 °C was performed. LC-MS showed conversion to the cyclized product, with the formation of 2 diastereoisomers. DCM and $Na_2CO_3$ 10% aq. sol. were then added, the org. layer was separated, dried ($MgSO_4$), filtered and the solvent was evaporated *in vacuo.* Purification by column chromatography (silica; DCM/(7 N $NH_3$ in MeOH) 100/0 to 90/10) yielded intermediate 37 (200 mg, 17%), intermediate 38 (130 mg, 11%) and several mixed fractions, which were discarded.

EXAMPLE A33

PREPARATION OF INTERMEDIATE 39

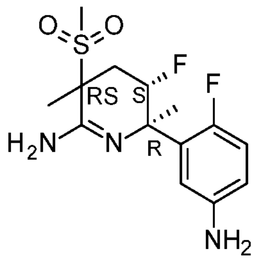

By following a synthetic route similar to the one described for the synthesis of (in the order) intermediate 11, intermediate 12, intermediate 13, intermediate 19, intermediate 26 and intermediate 32, intermediate 39 could be obtained starting from intermediate 23.

EXAMPLE A34

PREPARATION OF INTERMEDIATE 44

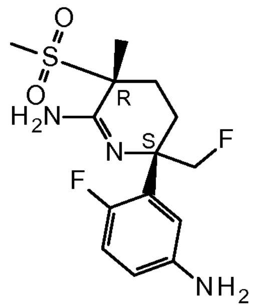

Intermediate 44 (free base)/(.HCl)

Sodium azide (1.06 g, 16.3 mmol), CuI (1.11 g, 5.82 mmol) and sodium carbonate (0.99 g 9.31 mmol) were added to a solution of intermediate 14a (1.84 g, 4.66 mmol) in ACN (55 mL). After the mixture was well purged with $N_2$, N,N'-dimethylethylenediamine (0.88 mL 8.15 mmol) was added and the mixture was stirred for 16 h at 100 °C (closed vessel). The reaction was poured into 25% aq $NH_3$ and stirred for 1 h at rt. Next the mixture was extracted with EtOAc. The combined organic layers were washed with brine and dried with $MgSO_4$, after which the solvent was evaporated under reduced pressure. The residue was purified twice by column chromatography (80 g silica gel, DCM/7 N $NH_3$ in MeOH, gradient: 1:0 to 9:1). After evaporation of the product fractions the residue was re-dissolved in DCM and 6 N HCl/2-propanol was added (2 mL). The solvent was evaporated, and the residue re-dissolved in methanol and evaporated again. The residue was dried overnight in vacuo at 50 °C providing intermediate 44.HCl as a pink solid (810 mg, 43%, 92% pure). Intermediate 44 was further purified by prep HPLC (Stationary phase: RP XBridge Prep C18 OBD-10μm,30 x 150 mm, Mobile phase: 0.25% $NH_4HCO_3$ solution in water, MeOH) yielding intermediate 44 (free base) as a white solid (289 mg, 30% from intermediate 14a).

EXAMPLE A35

PREPARATION OF INTERMEDIATE 55

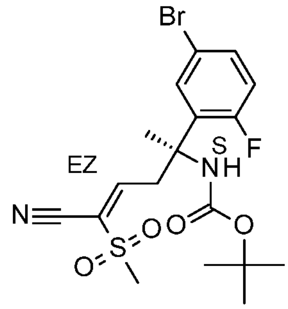

*tert*-Butyl [(2*S*)-2-(5-bromo-2-fluorophenyl)-4-oxobutan-2-yl]carbamate (10.27 g, 28.51 mmol, CAS: 1374001-20-3, described in WO 2012057247) was dissolved in 173 mL MeOH. Next MgO (696 mg, 17.26 mmol) and (methylsulfonyl)acetonitrile (6114 mg, 51.32 mmol) were added. The reaction mixture was then stirred for 2 h at rt, and filtered over dicalite®. The solvent was evaporated under reduced pressure to afford intermediate 55 as a yellow oil (13.2 g, 63% pure, quantitative). The product was used further as such.

PREPARATION OF INTERMEDIATE 56

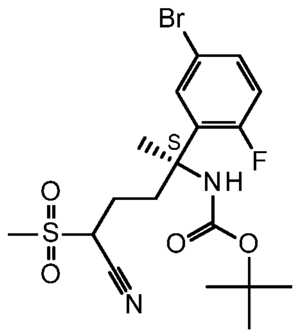

Intermediate 55 (13.2 g, 18.51 mmol) was stirred in 529 mL THF, the reaction mixture was cooled to -5°C, $NaBH_4$ (1.62 g, 42.77 mmol) was added, the reaction mixture was stirred for 30 minutes at 0 °C. Aq. $NH_4Cl$ (1 M) and DCM were added and the mixture was stirred over the weekend. The organic layer was separated, dried with $MgSO_4$, filtered and the solvent was evaporated under reduced pressure. The reaction mixture was purified twice with column chromatography over 80 g silicagel (gradient n-heptane to 100% EtOAc). The product fractions were collected and the solvent was evaporated under reduced pressure yielding intermediate 56 as a transparent glass (3.23 g, 31%).

PREPARATION OF INTERMEDIATE 121

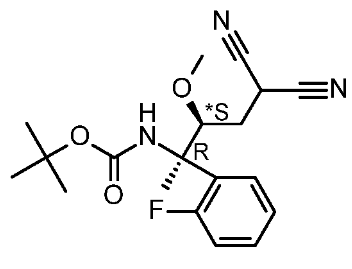

1-121 was synthesized following an analogous procedure to that described for the preparation of I-56, starting from 1-122.

PREPARATION OF INTERMEDIATE 57

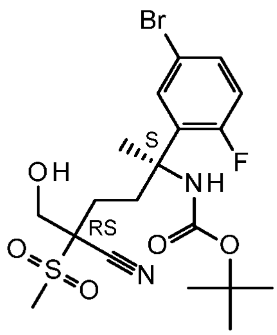

Intermediate 56 (1 g, 2.16 mmol) was dissolved in 1,4-dioxane (27 mL), formaldehyde (37% in water, 0.4mL, 5.39 mmol) and triethylamine (1 M in THF, 0.1 mL, 0.11 mmol) were added. The r.m. was stirred at 38 °C for 48h until LC-MS showed completion of the reaction, then it was cooled to r.t.. EtOAc and water were added and the organic layer was separated. The aqueous layer was extracted twice with EtOAc, the organic layers were combined, dried ($MgSO_4$) and concentrated under vacuum. The residue was purified by flash column chromatography (silica; Heptane/EtOAc 1/0 to 5/5) to afford intermediate 57 as a white solid (893 mg, 80%).

EXAMPLE A36

PREPARATION OF INTERMEDIATE 58

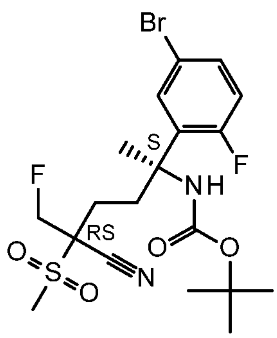

DAST (0.79 mL, 6.49 mmol) was added dropwise to a solution of intermediate 56 (400 mg, 0.81 mmol) in DCM (15 mL) under nitrogen at 0 °C. The r.m. was left to warm up to r.t. and stirred for 1 h then the temperature was increased to 50°C and the r.m. was stirred for 20h. $NaHCO_3$ sat. sol. was added and the mixture was extracted with DCM. The organic layer was separated, dried ($MgSO_4$), filtered and concentrated under vacuum to yield intermediate 58 as an orange oil which was used without further purification in the next step (440 mg, quantitative).

EXAMPLE A37

PREPARATION OF INTERMEDIATE 59

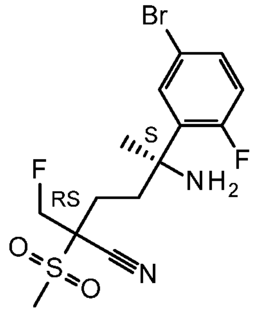

Intermediate 58 (440 mg, 0.89 mmol) was dissolved in TFA (11 mL) and the ensuing mixture was stirred for 45 min at r.t. until LCMS showed completion of the reaction. The r.m. was taken up in DCM and $NaHCO_3$ sat. solution. The organic layer was separated and the aqueous layer was extracted with DCM (3x) and EtOAc (1x). The combined organic layers were dried ($MgSO_4$), filtered and concentrated under vacuum to give a crude material which was purified by column chromatography (silica; DCM/MeOH 1/0 to 9/1) to give intermediate 59 as a brown solid (317 mg, 78%).

EXAMPLE A3 8

PREPARATION OF INTERMEDIATE 60

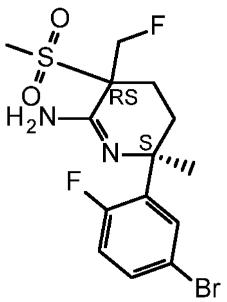

Intermediate 59 (280 mg, 0.71 mmol) was dissolved in acetic acid (14 mL) and HCl (9 M in water, 2 mL) in a microwave tube then the r.m. was stirred in the microwave at 160 °C for 12h. The r.m. was concentrated, the residue was taken up in ice and EtOAc, and then $Na_2CO_3$ was added until basic. The organic layer was separated and the aq. layer was extracted once more with EtOAc. The combined organic layers were dried ($MgSO_4$), filtered and evaporated to afford an orange oil which was purified by column chromatography (silica; Heptane/EtOAc 1/0 to 5/5) to give a mixture of diastereoisomers.

Separation into intermediate 60a and intermediate 60b:

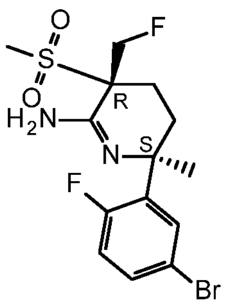

Intermediate 60a

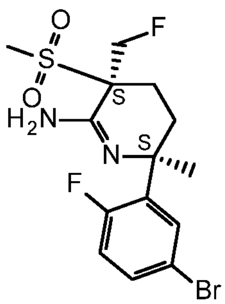

Intermediate 60b

Intermediate 60 can be separated into diastereoiomers intermediate 60a and intermediate 60b by preparative HPLC (Stationary phase: RP XBridge Prep C18 OBD-10μm,30x150mm, Mobile phase: 0.25% $NH_4HCO_3$ solution in water, MeOH) to yield intermediate 60a (70 mg, 25%) and intermediate 60b (40 mg, 14%).

EXAMPLE A39

PREPARATION OF INTERMEDIATE 61

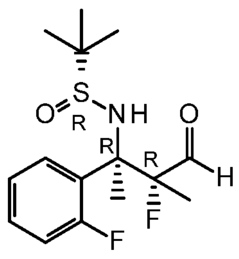

To a stirred solution of [S(R)]-N-[(1R,2R)-2-fluoro-1-(2-fluorophenyl)-3-hydroxy-1,2-dimethylpropyl]-2-methyl-2-propanesulfinamide (5g, 0.0157 mol; CAS: 1402412-96-7, described in WO2012/156284) in DCM (54 ml) were added small portions of Dess-Martin periodinane (8g, 0.0189 mol). After 5 h at r.t. the r.m. was treated with sat. $Na_2S2O_3$ and sat $NaHCO_3$ (caution: gas evolution). The biphasic mixture was vigorously stirred for 1h. The organic layer was then separated, washed with a $Na_2CO_3$ solution (x 3), and the aqueous layers were extracted with DCM. The combined organic layers were dried over $MgSO_4$ and evaporated. The residue was used without any further purification (3.4 g, 69%).

EXAMPLE A40

PREPARATION OF INTERMEDIATE 62

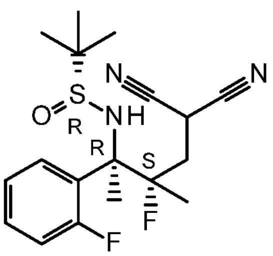

To a stirred solution of intermediate 61 in MeOH (90 ml) at 0 °C, were added magnesium oxide (590 mg, 14.6 mmol) and titanium(IV)isopropoxide (8.7 ml, 23.3mmol), followed by malononitrile (1.29 g,19.5 mmol) and sodium cyanoborohydride (614 mg, 9.8 mmol). The reaction was stirred at 0 °C for 30 min. The residue was filtered over dicalite® and washed with EtOAc, the filtrate was concentrated under reduced pressure and then taken up in EtOAc and water. Dicalite® was added to the biphasic sol. which was then filtered and washed with EtOAc. The filtrate was transfered into a separation funnel. The organic layer was separated and the aqueous layer further extracted with EtOAc. The combined organic layers were dried over $MgSO_4$, filtered, and concentrated. The residue was purified with column chromatography (silicagel gradient: n-heptane/EtOAc 100/0 to n-heptane/EtOAc 50/50). The desired product was collected and the solvent was evaporated under reduced pressure to yield intermediate 62 (1.6 g, 45%).

PREPARATION OF INTERMEDIATE 122

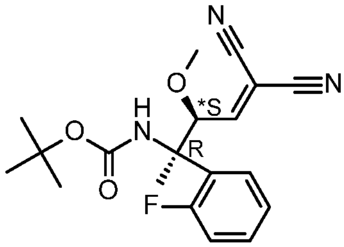

1-22 was synthesized following an analogous procedure to that described for the preparation of 1-62, starting from 1-107.

EXAMPLE A41

PREPARATION OF INTERMEDIATE 63

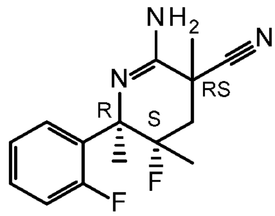

Intermediate 62 (1.4 g, 3.81 mmol) was dissolved in dry THF (100 ml), and stirred under $N_2$ atmosphere at 0°C. NaH (60% in mineral oil) (213 mg, 5.3 mmol) was added, and stirred for 15 min at 0 °C under $N_2$ atmosphere. MeI (0.474 ml, 7.62 mmol) was added, stirred for 30 min at 0°C then for 120 min at rt. $H_2O$ was added carefully under $N_2$ atmosphere and DCM was added, the organic layer was separated, dried with $MgSO_4$, filtered off and the solvent was evaporated under reduced pressure to yield intermediate 63 (0.6 g, 57%). The product was used as such without further purification

EXAMPLE A42

PREPARATION OF INTERMEDIATE 64

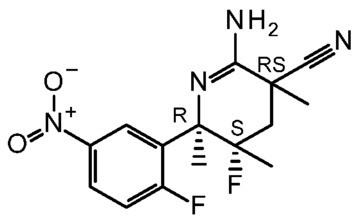

Potassium nitrate (212 mg, 2.1 mmol) was added to a mixture of intermediate 63 (530 mg, 1.9 mmol) in $H_2SO_4$ (10.2 ml, 191.1 mmol) at 0 °C, and the reaction mixture was stirred for 15 minutes at 0 °C. The reaction mixture was then poured into ice and a $Na_2CO_3$ sat solution. DCM was then added, followed by additional $H_2O$. The reaction mixture was basified to pH >8 with careful addition of $Na_2CO_3$ solid under stirring. Additional DCM was added. The organic phase was separated and the aqueous layer was extracted with DCM. The combined organic layers were dried over $MgSO_4$, filtered and evaporated to obtain a crude product (600 mg, 97%) which was used further without purification.

PREPARATION OF INTERMEDIATE 101

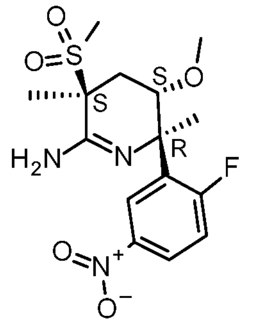

1-101 was synthesized following an analogous procedure to that described for the preparation of I-64, starting from 1-104.

EXAMPLE A43

PREPARATION OF INTERMEDIATE 65

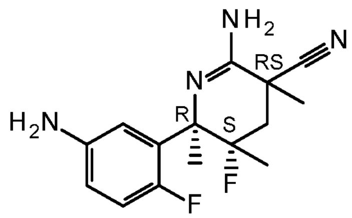

Intermediate 64 (600 mg, 1.86 mmol) was dissolved in a mixture of MeOH (9 ml) and $H_2O$ (2 ml). Iron (832 mg, 14.9 mmol) was added, followed by addition of ammonium chloride (1081 mg, 20.2 mmol). The reaction mixture was stirred at 70°C for 1 h, then allowed to cool to rt. MeOH and DCM were added and the mixture was filtered over dicalite®. The organic layer was washed with $H_2O$, the organic layer was separated and dried over $MgSO_4$, and the solvent was evaporated under reduced pressure, yielding intermediate 65 (300 mg, 55%), which was used without further purification.

PREPARATION OF INTERMEDIATE 123

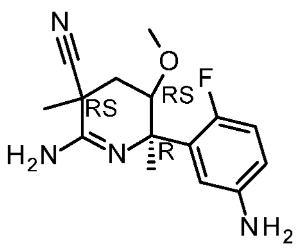

1-123 was synthesized following an analogous procedure to that described for the preparation of I-65 (NB: reaction time 23 h), starting from 1-118.

EXAMPLE A44

PREPARATION OF INTERMEDIATE 102

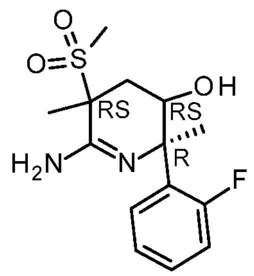

To a solution of I-94 (1.9 g, 4.70 mmol) in DCM (45 mL) at 0 °C was added boron tribromide (1 M in DCM, 12 mL, 12 mmol) and the mixture was stirred at rt for 4h. Additional boron tribromide (1 M in DCM, 4 mL, 4 mmol) was added at rt and the reaction mixture was stirred for 16 h at rt. It was then quenched with sat. aq sol of $NaHCO_3$ and extracted with 10% of 7 N $NH_3$ in MeOH in DCM twice.

The combined org layers were dried ($Na_2SO_4$), filtered and concentrated in vacuo. The residue was purified by flash column chromatography (silica; 7N $NH_3$ in MeOH in DCM 0/100 to 7/93). The desired fractions were collected and concentrated in vacuo to yield 1-102 (1.4 g, 95%).

EXAMPLE A45

PREPARATION OF INTERMEDIATE 103

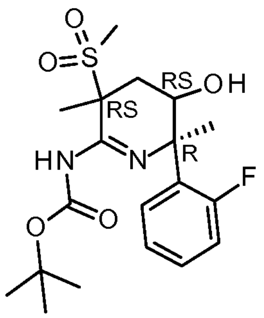

To a stirred solution of I-102 (900 mg, 2.86 mmol) in MeOH (8.2 mL) at RT was added Boc-anhydride (1.87 g, 8.59 mmol) and the reaction mixture was stirred at RT for 4h. The solvent was removed by evaporation. The residue was taken up in DCM and washed with $NaHCO_3$ sat.solution. The OL was dried ($MgSO_4$), filtered and evaporated to yield 1-103 (1.1 g, 93%) which was used without further purification.

EXAMPLE A46

PREPARATION OF INTERMEDIATE 104

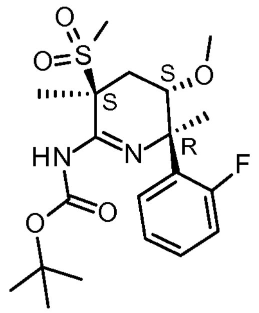

1-103 (675 mg, 1.63 mmol) was dissolved in THF (33 mL) under $N_2$, then cooled to 10 °C, NaH (60% dispersion in mineral oil, 195 mg, 4.89 mmol) was added at 10 °C and stirred at this temperature for 15 min. Then $CH_3I$ (101 μL, 1.63 mmol) was added at 10 °C for 1 h. Additional NaH (60% dispersion in mineral oil, 52.1 mg, 1.30 mmol) was added and stirred for 1 h, followed by addition of further NaH (60% dispersion in mineral oil, 65.1 mg, 1.63 mmol) and the reaction mixture was stirred for 48 h. The RM was treated with sat. $NaHCO_3$ sol. the aq layer was extracted with EtOAc and the org layers were washed with brine, dried ($MgSO_4$) and filtered. The filtrate was concentrated in vacuo and the crude product was purified by flash column chromatography (silica; DCM/MeOH 100/0 to 98/2). The product fractions were collected and concentrated in vacuo to yield two fractions, one of them requiring further purification by flash column chromatography (silica; heptane/EtOAc 100/0 to 50/50). The pure product fractions were collected, combined and concentrated in vacuo to yield 1-104 (103 mg, 15%).

EXAMPLE A47

PREPARATION OF INTERMEDIATE 105

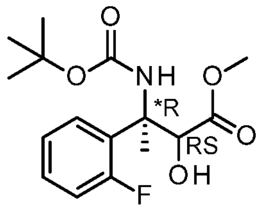

Pd/C (10%, 703.6 mg, 16.53 mmol) was added to a solution of I-96 (6.9 g, 16.53 mmol) in MeOH (413 mL) under nitrogen. The mixture was hydrogenated (atmospheric pressure) at rt overnight.

The mixture was filtered through celite® and the filtrate was evaporated in vacuo to yield 1-105 (5 g, 92%) as a colourless oil.

EXAMPLE A48

PREPARATION OF INTERMEDIATE 106

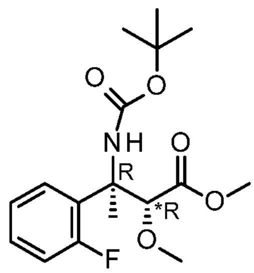

1-105 (10 g, 30.55 mmol) was dissolved in DMF (200 mL), then $Ag_2O$ (7.08 g, 30.55 mmol) and $CH_3I$ (3.80 mL, 61.10 mmol) were added. The RM was heated to 60 °C and stirred overnight. The mixture was poured out in $H_2O$ and extracted with EtOAc, dried ($MgSO_4$) and concentrated, and the residue was purified by flash column chromatography (Redisep, 120 g silica; heptane/EtOAc 100/0 to 70/30), delivering I-106 (9.01 g, 80%).

PREPARATION OF INTERMEDIATE 107

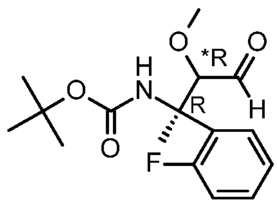

To a suspension of I-116 (483 mg, 1.54 mmol), 4-methylmorpholine N-oxide monohydrate (270.84 mg, 2.31 mmol) and molecular sieves (powdered, 750 mg) in DCM (9.5 mL) was added tetrapropylammonium perruthenate (27.1 mg, 0.08 mmol) and the RM was stirred at rt for 30 min, then it was filtered, washed with DCM and concentrated. The residue was purified by flash column chromatography (heptane/EtOAc 100/0 to 50/50), delivering 1-107 (364 mg, 76%) as a colorless oil.

PREPARATION OF INTERMEDIATE 108

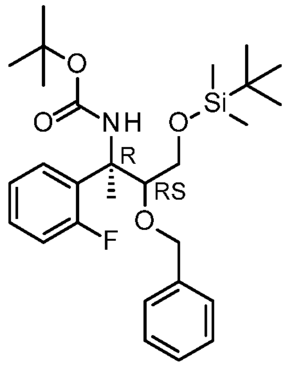

To a stirred solution of I-97 (9.5 g, 24.39 mmol) in DMF (80 mL) were added imidazole (5.81 g, 85.37 mmol) and 4-(dimethylamino)pyridine (596 mg, 4.88 mmol) and the RM was cooled to 10 °C. tert-Butyldimethylsilyl chloride (7.35 g, 48.79 mmol) was then added at 10 °C. After 24 h, tert-butyldimethylsilyl chloride, imidazole, and 4-(dimethylamino)pyridine were added again in the same amounts and the RM was stirred at rt for 48 h, then it was quenched with $H_2O$, extracted with EtOAc (3x). The org layer was dried ($MgSO_4$), filtered and concentrated by evaporation to yield a residue that was purified by flash column chromatography (silica gel, ISCO purification system, Redisep column, 330 g, 40 min; heptane/EtOAc 100/0 to 80/20).

The product fractions were collected and concentrated by evaporation to yield 1-108 (8.5 g, 69%).

PREPARATION OF INTERMEDIATE 109

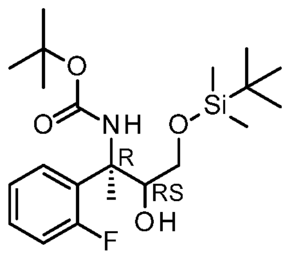

1-108 (7.2 g, 14.29 mmol) was hydrogenated at 14 °C with Pd/C (10%, 331.8 mg, 0.31 mmol) and $H_2$ in EtOAc (141 mL). After take up of $H_2$, the reaction mixture was filtered over dicalite®. The filtrate was concentrated to complete dryness and 1-109 (5.9 g, quant.) solidified upon standing.

PREPARATION OF INTERMEDIATE 110

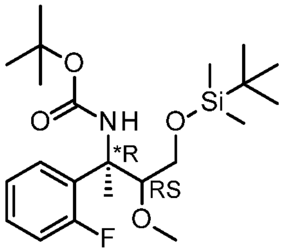

1-109 (4 g, 9.67 mmol) was dissolved in toluene (80 mL). NaOH (50% in $H_2O$, 24.05 mL, 455.5 mmol) was added, then benzyltriethylammonium chloride (220.3 mg, 0.97 mmol) and then dimethyl sulfate (1.19 mL, 12.57 mmol) was added at RT and stirred for 3.5 h. The RM was treated with sat. $NH_4Cl$ solution. The aq layer was extracted with EtOAc and the org layers were washed with brine, dried ($MgSO_4$) and filtered. The filtrate was concentrated in vacuo and the crude product was purified by flash column chromatography (silica, NP, flash purification system; heptane/EtOAc 100/0 to 90/10). The pure product fractions were collected, combined and concentrated in vacuo to yield 1-110 (3.88 g, 94%).

PREPARATION OF INTERMEDIATE 111

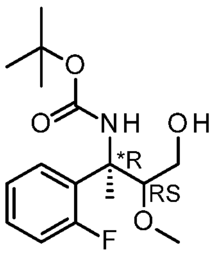

To a mixture of I-110 (3.62 g, 8.47 mmol) in THF (34 mL) was added tetrabutylammonium fluoride (1M in THF, 12.70 mL, 12.70 mmol) at 0 °C. After stirring for 1 h at 0 °C, the RM was treated with $NaHCO_3$ and the aq layer was extracted with EtOAc. The org layers were washed with $H_2O$ and brine, dried ($MgSO_4$) and filtered. The filtrate was concentrated in vacuo to yield 1-111 (3.92 g), which was used without further purification.

PREPARATION OF THE FINAL COMPOUNDS

EXAMPLE B1

PREPARATION OF COMPOUNDS 1 AND 2

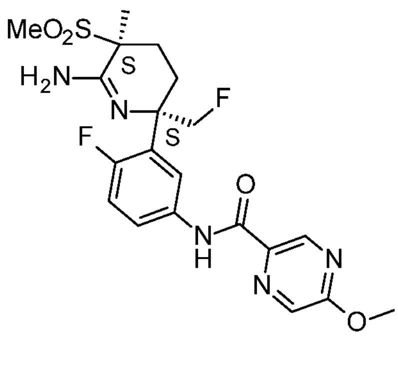

Compound **1**

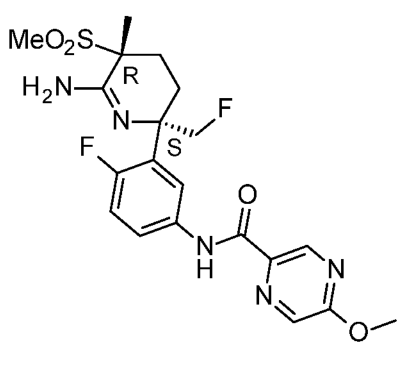

Compound **2**

A microwave tube was loaded with intermediate 14 (195 mg, 0.493 mmol), 5-methoxy-2-pyrazinecarboxamide (91 mg, 0.592 mmol), CuI (103 mg, 0.543 mmol) and $K_3PO_4$ (209 mg, 0.987 mmol) in dioxane (5.1 mL). The vial was degassed by bubbling nitrogen for a few minutes. Then, *trans-N,N'*-dimethylcyclohexane-1,2-diamine (84 mg, 0.592 mmol) was added and, after stirring for 2 min at r.t., the mixture was heated for 16 h at 100 °C. The mixture was then poured into 7 N $NH_3$ in MeOH and stirred for 1 h. Next, water and DCM were added and the org. layer was separated. The aq. layer was extracted twice with DCM. The org. layer was separated, dried ($MgSO_4$), filtered and concentrated *in vacuo* providing a crude which was purified by Prep SFC (Stationary phase: Chiralcel Diacel OD 20x250 mm; Mobile phase: $CO_2$, EtOH+0.4% $iPrNH_2$) to yield two fractions, each of them individually further purified by flash column chromatography (silica; DCM/(7 N $NH_3$ in MeOH) 100/0 to 90/10). After removal of the solvent the desired compounds were obtained as white crystals (Compound 1: 52 mg, 22%. Compound 2: 58 mg, 25%).

EXAMPLE B2

PREPARATION OF COMPOUNDS 3, 4 AND 5

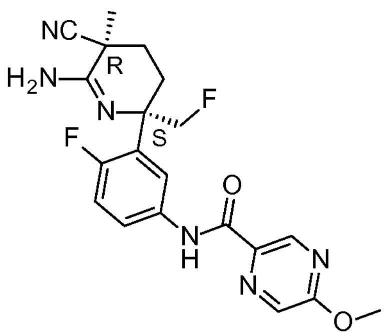

Compound **4**

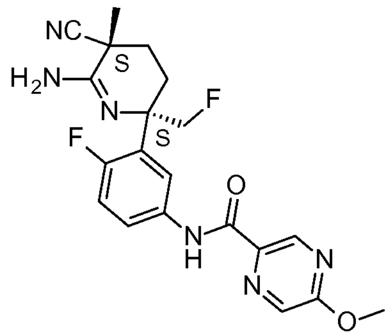

Compound **5**

5-Methoxy-2-pyrazinecarboxamide (76 mg, 0.494 mmol) was added to a stirred sol. of intermediate 19 (130 mg, 0.38 mmol) in dioxane (10 mL) under nitrogen atmosphere. $K_2CO_3$ (157 mg, 1.14 mmol), CuI (72 mg, 0.38 mmol) and *N,N'*-dimethylethylenediamine (61 μL, 0.57 mmol) were subsequently added and the resulting mixture was stirred at 100 °C for 16 h. The r.m. was then diluted with DCM and washed with $NH_3$ in water (28%). The org. layer was separated, dried ($MgSO_4$), filtered and the solvent was removed *in vacuo.* The resulting crude was purified by flash column chromatography (silica; DCM/(7 N $NH_3$ in MeOH) 100/0 to 93/7). The pure fractions were collected and the solvent removed *in* vacuo. After suspension in DIPE and drying under nitrogen stream at 50 °C Compound 3 (mixture of Compound 4 and Compound 5) was obtained. The mixed fractions were further purified by Prep HPLC (Stationary phase: RP XBridge Prep C18 ODB 5μm 30x250 mm; Mobile phase: 0.5% $NH_4Ac$ sol. in water +10% MeCN, MeCN) yielding Compound 4 (22 mg, 14%) and Compound 5 (30 mg, 19%).

EXAMPLE B3

PREPARATION OF COMPOUNDS 6, 7 AND 8

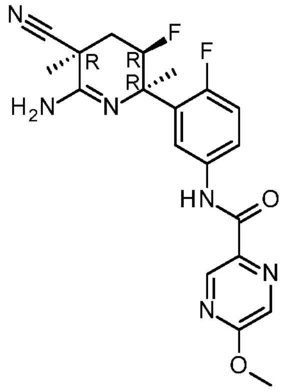

Compound **6**

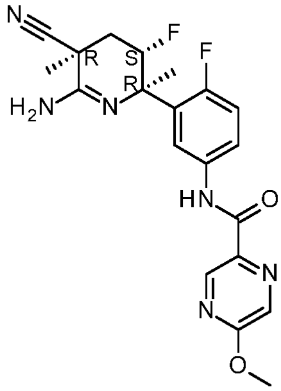

Compound **7**

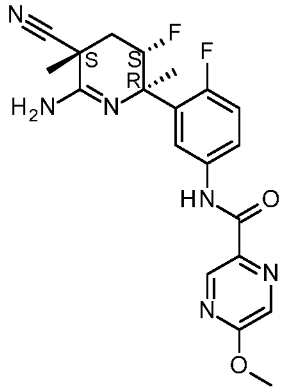

Compound **8**

Intermediate 27 (230 mg, 0.826 mmol) was stirred in MeOH (19.2 mL) at r.t. and HCl (6 M in iPrOH, 0.206 mL, 1.24 mmol) was added. After stirring for 5 min 5-methoxypyrazine-2-carboxylic acid (140 mg, 0.91 mL) was added, followed by EDCI (206 mg, 1.07 mmol) 5 min later. After 10 min reaction was complete and the solvent was removed *in vacuo.* The residue was taken up in DCM and washed with aq. $Na_2CO_3$ sol. The org. layer was dried ($MgSO_4$), filtered and evaporated. The residue was purified by flash column chromatography (silica; DCM/(7 N $NH_3$ in MeOH) 100/0 to 90/10). The fractions containing the products were collected and further purified by Prep SFC (Stationary phase: Kromasil (R,R) Whelk-O 1 20x250 mm; Mobile phase: $CO_2$, EtOH+0.4% $iPrNH_2$) yielding Compound 6 (87 mg, 25%), Compound 7 (42 mg, 12%) and Compound 8 (72 mg, 21%).

EXAMPLE B4

PREPARATION OF COMPOUNDS 9 AND 10

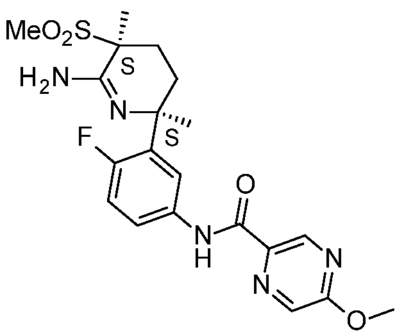

Compound **9**

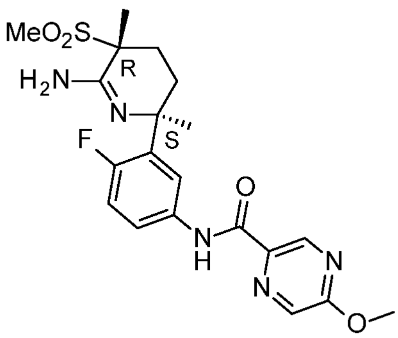

Compound **10**

By following a synthetic procedure similar to the one reported in Example B3, starting from intermediate 32 a mixture of compounds 9 and 10 was obtained. Further purification by Prep SFC (Stationary phase: Chiralpak Diacel AD 20 x 250 mm; Mobile phase: $CO_2$, EtOH) afforded Compound 9 (180 mg, 28%) and Compound 10 (112 mg, 17%) as white solids.

EXAMPLE B5

PREPARATION OF COMPOUNDS 11, 12, 13 AND 14

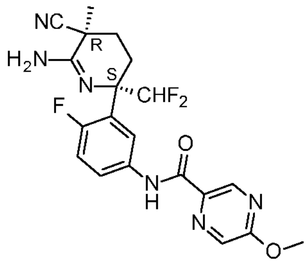

Compound **11**

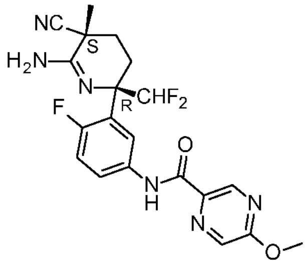

Compound **12**

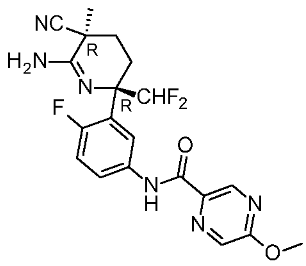

Compound **13**

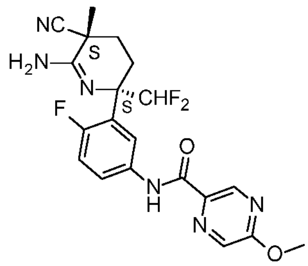

Compound **14**

By following a synthetic procedure similar to the one reported in Example B2, starting from intermediate 34 two fractions containing each a mixture of compounds 11, 12, 13 and 14 were obtained. Subsequent purification by Prep HPLC (Stationary phase: RP XBridge Prep C18 ODB- 5μm 30x250 mm; Mobile phase: 0.25% $NH_4HCO_3$ sol. in water, MeCN) and Prep SFC (Stationary phase: Chiralpak Diacel AD 20x250 mm; Mobile phase: $CO_2$, MeOH+0.4% $iPrNH_2$) yielded 4 fractions, each of them containing one compound. After removal of the solvent, concentration under nitrogen at 50 °C, trituration with DIPE and further concentration under nitrogen at 50 °C desired Compound 11 (8 mg, 2%), Compound 12 (10 mg, 3%), Compound 13 (33 mg, 9%) and Compound 14 (39 mg, 10%) were obtained.

EXAMPLE B6

PREPARATION OF COMPOUNDS 17 AND 18

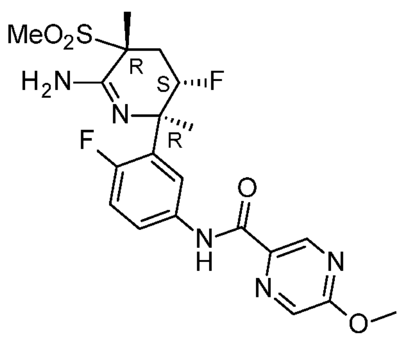

Compound **17**

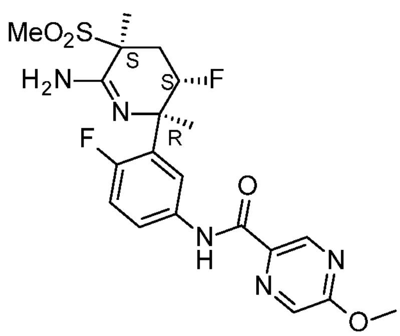

Compound **18**

By following a synthetic procedure similar to the one reported in Example B3, starting from intermediate 39 a

crude was obtained, which was purified by Prep SFC (Stationary phase: Chiralcel Diacel OJ 20x250 mm; Mobile phase: $CO_2$, EtOH-iPrOH (50-50)+0.4% $iPrNH_2$) The two fractions obtained were separately further purified by flash column chromatography (silica; DCM/(7 N $NH_3$ in MeOH) 100/0 to 98/2), dissolved in DIPE/DCM, dried in the Genevac™ and then overnight in the vacuum oven at 50 °C. Compound 17 (7.6 mg, 7%) and Compound 18 (24 mg, 21%) were finally obtained as white solids.

EXAMPLE B7

PREPARATION OF COMPOUND 19

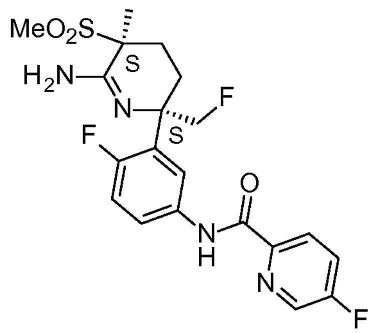

By following a synthetic procedure similar to the one reported in Example B1, starting from intermediate 14b and 5-fluoro-2-pyridinecarboxamide a crude was obtained which was further purified by Prep HPLC (RP XBridge Prep C18 OBD- 10μm 30x250 mm; Mobile phase: 0.25% $NH_4HCO_3$ sol. in water, MeOH). Compound 19 (25 mg, 25%) was obtained as a white crystalline solid.

EXAMPLE B8

PREPARATION OF COMPOUND 20

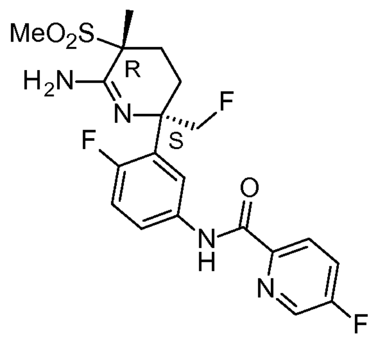

By following a synthetic procedure similar to the one reported in Example B1, starting from intermediate 14a and 5-fluoro-2-pyridinecarboxamide a crude was obtained, which was further purified by Prep HPLC (RP XBridge Prep C18 OBD- 5μm 30x250 mm; Mobile phase: 0.25% $NH_4HCO_3$ sol. in water, MeOH). Compound 20 (34 mg, 25%) was obtained as a white crystalline solid.

EXAMPLE B9

PREPARATION OF COMPOUND 21

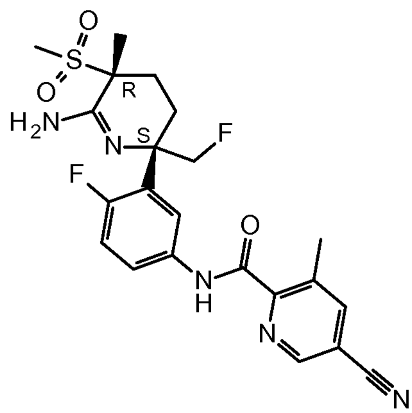

Intermediate 44 (free base) (100 mg, 0.25 mmol) was stirred in methanol (3.13 mL) at rt after which HCl (5 M in 2-propanol, 0.057 mL, 0.29 mmol) was added. The reaction mixture was stirred for 5 min. Then EDCI (94.8 mg, 0.50 mmol) was added and 5 min later, 5-cyano-3-methylpyridine-2-carboxylic acid (80.2 mg 0.50 mmol) was added. After 15 min the reaction mixture was concentrated. DCM was added followed by satd. aq. $Na_2CO_3$. The organic layer was separated and the aqueous layer extracted with DCM. The combined org layers were dried ($MgSO_4$), filtered and evaporated. The residue was purified by column chromatography (silica gel, DCM/(7 N $NH_3$ in MeOH), gradient: 100/0 to 98/2). The product fractions were collected, evaporated and dried in vacuo at 50 °C yielding compound 21 as a white solid (107 mg, 91%).

PREPARATION OF COMPOUND 41

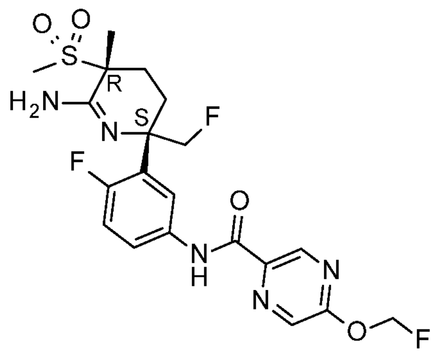

Intermediate 44 (free base) (119 mg, 0.359 mmol) was stirred in MeOH (4.5 mL) at rt. HCl (5 M in 2-propanol, 0.060 mL, 0.360 mmol) was added. The r.m. was stirred for 5 min. Then 5-(fluoromethoxy)pyrazine-2-carboxylic acid (116 mg, 0.677 mmol) was added and 5 min later, EDCI (138 mg 0.718 mmol) was added. LCMS showed complete conversion after 15 min. The reaction mixture was evaporated. DCM was added followed by aq. $Na_2CO_3$ sol. The organic layer was separated and the aqueous layer extracted with DCM. The combined org layers were dried ($MgSO_4$), filtered and evaporated. The residue was purified by column chromatography (silica gel, DCM/(7 N $NH_3$ in MeOH), gradient 100/0 to 95/5), product fractions were collected and evaporated. The residue was taken up in diethyl ether, and heptane was added. Next the ether was slowly evaporated until a white solid formed, which was filtered and washed with heptane, water and again heptane. Next the solid was dried in vacuo at 55 °C overnight, and then at 75 °C for 2 h, providing compound 41 (150 mg, 86%) as a white powder.

EXAMPLE B10

PREPARATION OF COMPOUND 46

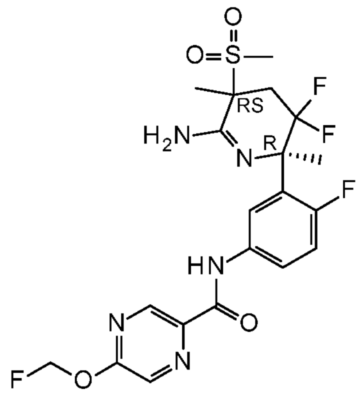

Intermediate 53 (113 mg, 0.323 mmol) was stirred in MeOH (2.5 mL) at r.t.. HCl (63.176 $\mu$L, 6M, 0.4 mmol) was added. The solution was stirred for 5 min. Then 5-(fluoromethoxy)pyrazine-2-carboxylic acid (133.603 mg, 0.8 mmol) was added and 5 min later, EDCI (161.209 mg, 0.8 mmol) was added and the solution was stirred for 1.5 h. DCM was added, followed by $Na_2CO_3$ sat. sol.. The org. layer was separated and the aq. layer was extracted with DCM. The combined org. layers were dried ($MgSO_4$), filtered and the filtrate was concentrated *in vacuo.* The crude product was purified by flash column chromatography (silica gel, gradient DCM/(7 N $NH_3$ in MeOH) from 100/0 to 98/2) to afford compound 46 (34 mg, 100% purity, 21%).

Separation into compound 23 and compound 24

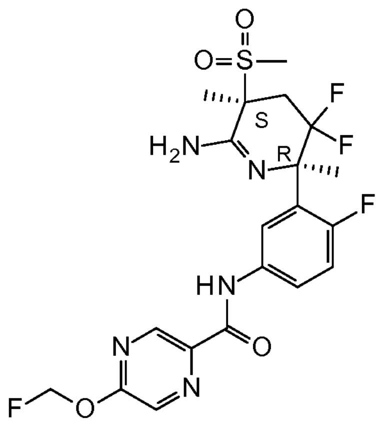

Compound 23

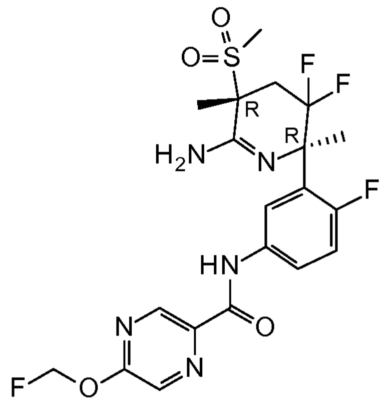

Compound 24

The diastereomers of compound 46 (34 mg) were separated via Prep HPLC (Stationary phase: RP XBridge Prep C18 OBD-10$\mu$m,30x150mm, Mobile phase: 0.25% $NH_4HCO_3$ solution in water, MeOH). The desired compound fractions were evaporated to dryness, and co-evaporated with ACN to remove residual ammonium bicarbonate. DIPE was added to the compounds and removed by a nitrogen flow overnight, yielding compound 24 (16.7 mg, 49%) and compound 23 (1.1 mg, 3.2%) as sticky powders.

EXAMPLE B11

PREPARATION OF COMPOUND 26

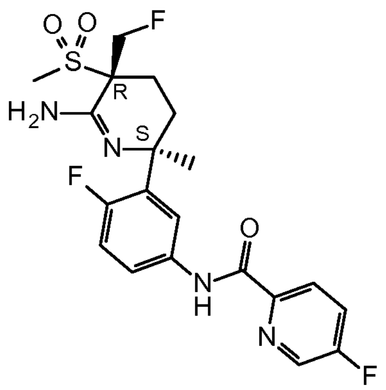

A microwave tube was loaded with intermediate 60a (70 mg, 0.18 mmol), 5-fluoropyridine-2-carboxamide (59 mg, 0.42 mmol), CuI (71 mg, 0.37 mmol) and $K_3O_4P$ (113 mg, 0.53 mmol) in 1,4-dioxane (1.8 mL). The tube was degassed by bubbling $N_2$ for few minutes. Then (1R,2R)-N,N'-dimethylcyclohexane-1,2-diamine (61 μL, 0.39 mmol) was added, after stirring for 2 min at rt, the r.m. was heated overnight at 130°C until LCMS showed completion of the reaction. The mixture was poured into 7 N $NH_3$ in MeOH and stirred for 1 h. Next, water and DCM were added and the organic layer was separated. The aqueous layer was extracted twice with DCM. The organic layer was separated, dried over $MgSO_4$, filtered and concentrated under vacuum, yielding a brown oil. A purification was performed via Prep HPLC (Stationary phase: RP XBridge Prep C18 OBD-10μm,30x150mm, Mobile phase: 0.25% $NH_4HCO_3$ solution in water, MeOH) yielding a solid which was dissolved in MeOH, evaporated and then dried in the oven at 50°C overnight to afford compound 26 (27 mg, 33%).

EXAMPLE B12

PREPARATION OF COMPOUND 27 AND COMPOUND 28

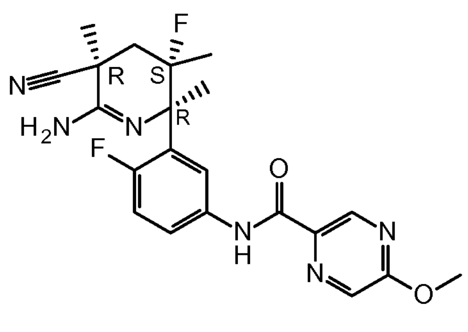

Compound 28

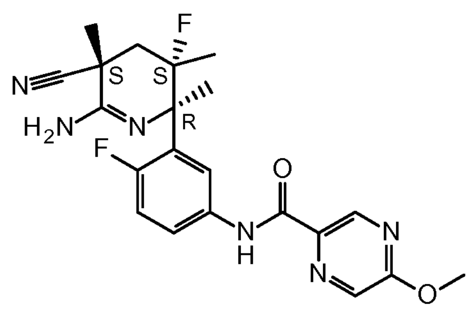

Compound 27

To a stirred solution of intermediate 65 (300 mg. 1.02 mmol) in MeOH at rt, was added HCl (6M in iPrOH) (0.257 ml, 1.54 mmol), and the reaction mixture was stirred for 5 min. Then 5-methoxypyrazine-2-carboxylic acid (175 mg, 1.1 mmol) was added and after 5 min EDCI (256 mg, 1.33 mmol) was added. The reaction was stirred for 10 min, then the solvent was removed by evaporation. The residue was taken up in DCM and washed with aq. $Na_2CO_3$ solution. The organic layer was dried ($MgSO_4$), filtered and evaporated. The residue was purified by flash chromatography (DCM:MeOH($NH_3$(7N) in DCM) 100/0 to 95/5). The pure product fractions (mixture of diastereomers) were collected and evaporated.

A purification was performed via Prep SFC (Stationary phase: Chiralpak Diacel AD 20 x 250 mm, Mobile phase: $CO_2$, EtOH + 0.4 $iPrNH_2$). The different product fractions were collected and the solvent was evaporated under reduced pressure. The products were suspended from DIPE, and dried under $N_2$ flow at 50°C, yielding compound 28 (63 mg, 14%) and compound 27 (56 mg, 13%).

EXAMPLE B13

PREPARATION OF COMPOUNDS 29A AND 29B

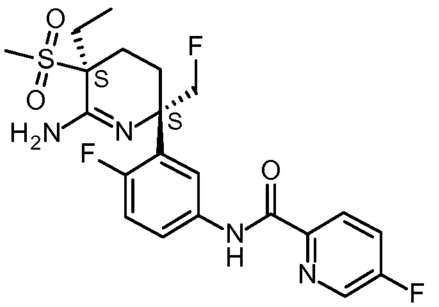

Co. No. 29a

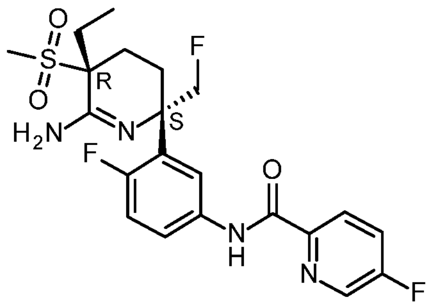

Co. No. 29b

By following a synthetic procedure similar to the one reported in Example B3, starting from I-99 (45 mg, 0.13 mmol) and 5-fluoropyridine-2-carboxylic acid ([107504-08-5], 21 mg, 0.15 mmol), a mixture of compounds 29a and b was obtained. Purification by Prep SFC (Stationary phase: Chiralpak Diacel AS 20 x 250 mm; mobile phase: $CO_2$, EtOH + 0.4 $iPrNH_2$) yielded Compound 29a (7 mg, 12%) and Compound 29b (15, 25%).

Alternatively, compound 29a/b can be prepared following a procedure similar to the one reported in Example B1.

EXAMPLE B14

PREPARATION OF COMPOUND 47

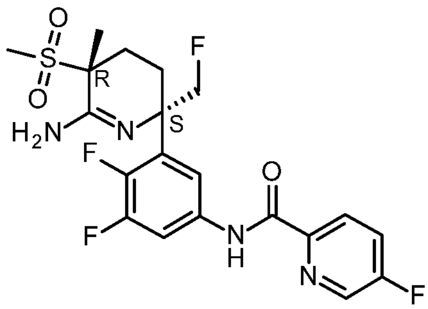

By following a synthetic procedure similar to the one reported in Example B11, starting from I-92a (47 mg, 0.11 mmol) and 5-fluoropyridine-2-carboxamide (7.3 mg, 0.05 mmol), compound 47 was obtained (53 mg, 99%). A purification was performed via Prep HPLC (Stationary phase: RP XBridge Prep C18 OBD-10μm,30x150mm, Mobile phase: 0.25% $NH_4HCO_3$ solution in water, $CH_3CN$) followed by evaporation of the eluent and co-evaporation with MeOH (x2), yielding compound 47 (5.5 mg, 26%) as a white solid.

EXAMPLE B15

PREPARATION OF COMPOUNDS 48A AND 48B

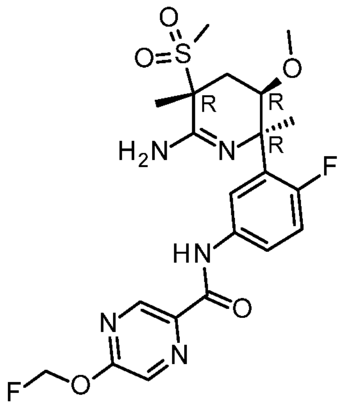

Co. No. 48a

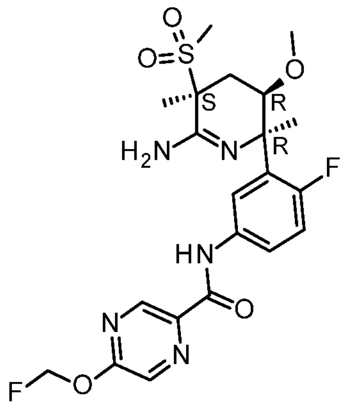

Co. No. 48b

By following a synthetic procedure similar to the one reported in Example B12, starting from 1-120 (100 mg, 0.29 mmol) and 5-(fluoromethoxy)pyrazine-2-carboxylic acid (120.28 mg, 0.70 mmol), followed by purification via Prep HPLC (Stationary phase: RP XBridge Prep C18 OBD-10μm,30x150mm, mobile phase: 0.25% $NH_4HCO_3$ solution in $H_2O$, MeOH) to yield two fractions which were concentrated. ACN was added and the solvent was removed in vacuo. The fractions were left in the oven at 55°C for 48 h. DIPE was added to the fractions and blown with $N_2$ overnight, yielding compound 48a (43.3 mg, 42%) and 48b (33.3, 32%) as white powders.

EXAMPLE B16

PREPARATION OF COMPOUNDS 49A AND 49B

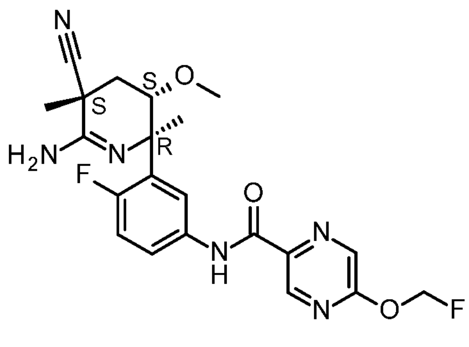

Co. No. 49a

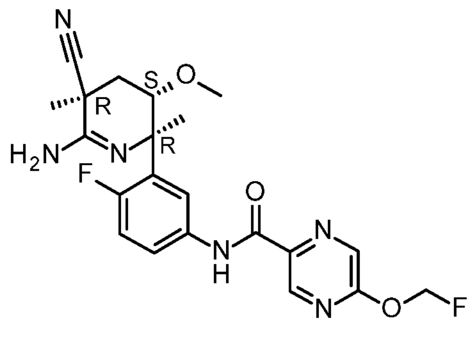

Co. No. 49b

By following a synthetic procedure similar to the one reported in Example B12, starting from 1-123 (84 mg, 0.29 mmol) and 5-(fluoromethoxy)-2-pyrazinecarboxylic acid (54.78 mg, 0.32 mmol), followed by purification by Prep HPLC (stationary phase: RP XBridge Prep C18 OBD-5μm,30x250mm; mobile phase: 0.25% $NH_4HCO_3$ solution in water, MeOH) yielding Co. No. 49a (13 mg, 10%) and Co. No. 49b (14 mg, 11%).

Compounds 1 to 49 in Tables 1a and b list the compounds that were prepared by analogy to one of the above Examples. Intermediate compounds where $R^2$ is e.g. $SO_2Et$, $SO_2{}^iPr$, $SO_2{}^cPr$ can be made by replacing 2-(methanesulfonyl)acetonitrile with commercially available 2-(ethanesulfonyl)acetonitrile, 2-[(1-methylethyl)sulfonyl]-acetonitrile and 2-(cyclopropylsulfonyl)-acetonitrile, respectively in procedures analogous e.g. to those described for the preparation of intermediates 11, 42, and 48. In case no salt form is indicated, the compound was obtained as a free base. 'Ex. No.' refers to the Example number according to which protocol the compound was synthesized. 'Co. No.' means compound number.

TABLE 1A

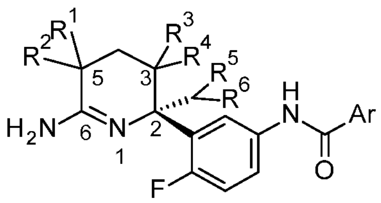

| Co. No. | Ex. No. | $R^1$ | $R^2$ | $R^3$ | $R^4$ | $R^5$ | $R^6$ | Ar | Stereochemistry |
|---|---|---|---|---|---|---|---|---|---|
| 1 | B1 | Me | $SO_2Me$ | H | H | F | H | 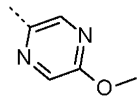 | (2S,5S) |
| 2 | B1 | Me | $SO_2Me$ | H | H | F | H | 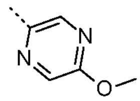 | (2S,5R) |
| 3 | B2 | Me | CN | H | H | F | H | 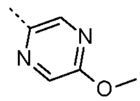 | (2S,5RS) |
| 4 | B2 | Me | CN | H | H | F | H | 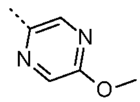 | (2S,5R) |
| 5 | B2 | Me | CN | H | H | F | H | 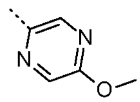 | (2S,5S) |
| 6 | B3 | Me | CN | H | F | H | H | 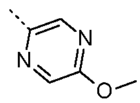 | (2R,3R,5R) |
| 7 | B3 | Me | CN | H | F | H | H | 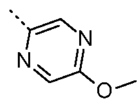 | (2R,3S,5R) |
| 8 | B3 | Me | CN | H | F | H | H | 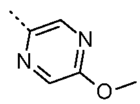 | (2R,3S,5S) |
| 9 | B4 | Me | $SO_2Me$ | H | H | H | H | 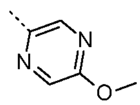 | (2S,5S) |
| 10 | B4 | Me | $SO_2Me$ | H | H | H | H | 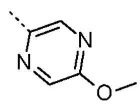 | (2S,5R) |
| 11 | B5 | Me | CN | H | H | F | F | 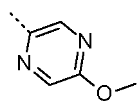 | (2S,5R) |

(continued)

| Co. No. | Ex. No. | $R^1$ | $R^2$ | $R^3$ | $R^4$ | $R^5$ | $R^6$ | Ar | Stereochemistry |
|---|---|---|---|---|---|---|---|---|---|
| 12 | B5 | Me | CN | H | H | F | F | 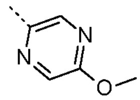 | (2R,5S) |
| 13 | B5 | Me | CN | H | H | F | F | 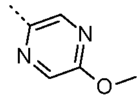 | (2R,5R) |
| 14 | B5 | Me | CN | H | H | F | F | 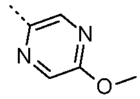 | (2S,5S) |
| 15 | B2 | Me | CN | H | H | H | H | 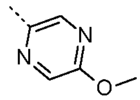 | (2S,5S) |
| 16 | B2 | Me | CN | H | H | H | H | 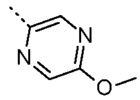 | (2S,5R) |
| 17 | B6 | Me | $SO_2Me$ | H | F | H | H | 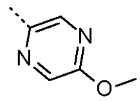 | (2R,3S,5R) |
| 18 | B6 | Me | $SO_2Me$ | H | F | H | H | 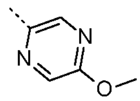 | (2R,3S,5S) |
| 19 | B7 | Me | $SO_2Me$ | H | H | F | H | 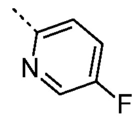 | (2S,5S) |
| 20 | B8 | Me | $SO_2Me$ | H | H | F | H | 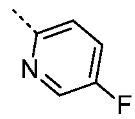 | (2S,5R) |
| 21 | B9 | Me | $SO_2Me$ | H | H | F | H | 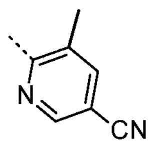 | (2S,5R) |
| 22 | B9 | Me | $SO_2Me$ | H | H | F | H | 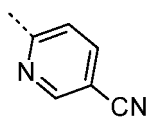 | (2S,5R) |
| 23 | B10 | Me | $SO_2Me$ | F | F | H | H | 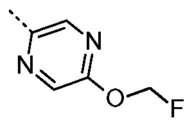 | (2R,5S) |

(continued)

| Co. No. | Ex. No. | $R^1$ | $R^2$ | $R^3$ | $R^4$ | $R^5$ | $R^6$ | Ar | Stereochemistry |
|---|---|---|---|---|---|---|---|---|---|
| 24 | B10 | Me | $SO_2Me$ | F | F | H | H | 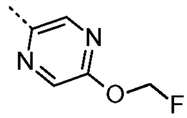 | (2R,5R) |
| 25 | B11 | Me | $SO_2{}^cPr$ | H | H | F | H | 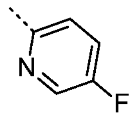 | (2S,5R) |
| 26 | B11 | $CH_2F$ | $SO_2Me$ | H | H | H | H | 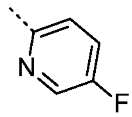 | (2S,5R) |
| 27 | B12 | Me | CN | Me | F | H | H | 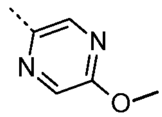 | (2R,3S,5S) |
| 28 | B12 | Me | CN | Me | F | H | H | 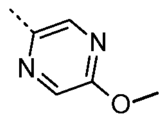 | (2R,3S,5R) |
| 29a | B1/B13 | Et | $SO_2Me$ | H | H | F | H | 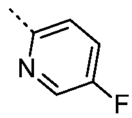 | (2S,5S) |
| 29b | B13 | Et | $SO_2Me$ | H | H | F | H | 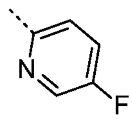 | (2S,5R) |
| 30 | B1 | Et | $SO_2Et$ | H | H | F | H | 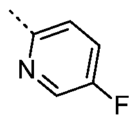 | (2S,5R) |
| 31 | B11 | Me | $SO_2Et$ | H | H | F | H | 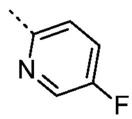 | (2S,5R) |
| 32 | B9 | Me | $SO_2Me$ | H | H | F | H | 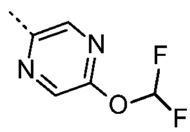 | (2S,5R) |
| 33 | B1 | Me | $SO_2{}^iPr$ | H | H | F | H | 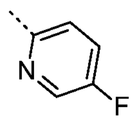 | (2S,5R) |
| 34 | B1 | Me | $SO_2{}^iPr$ | H | H | F | H | 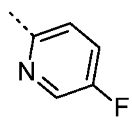 | (2S,5S) |

(continued)

| Co. No. | Ex. No. | $R^1$ | $R^2$ | $R^3$ | $R^4$ | $R^5$ | $R^6$ | Ar | Stereochemistry |
|---|---|---|---|---|---|---|---|---|---|
| 35 | B9 | Me | $SO_2Me$ | H | H | F | H | 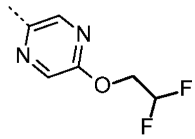 | (2S,5R) |
| 36 | B9 | Me | $SO_2Me$ | H | H | F | H | 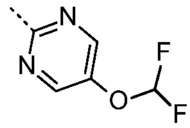 | (2S,5R) |
| 37 | B9 | Me | $SO_2Me$ | H | H | F | H | 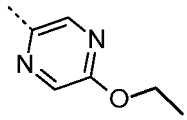 | (2S,5R) |
| 38 | B9 | Me | $SO_2Me$ | H | H | F | H | 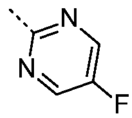 | (2S,5R) |
| 39 | B9 | Me | $SO_2Me$ | H | H | F | H | 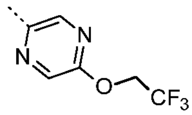 | (2S,5R) |
| 40 | B9 | Me | $SO_2Me$ | H | H | F | H | 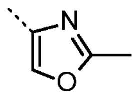 | (2S,5R) |
| 41 | B9 | Me | $SO_2Me$ | H | H | F | H | 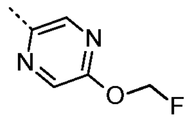 | (2S,5R) |
| 42 | B9 | Me | $SO_2Me$ | H | H | F | H | 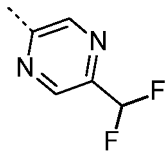 | (2S,5R) |
| 43 | B9 | Me | $SO_2Me$ | H | H | F | H | 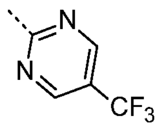 | (2S,5R) |
| 44 | B9 | Me | $SO_2Me$ | H | H | F | H | 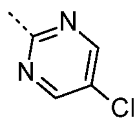 | (2S,5R) |
| 45 | B9 | Me | $SO_2Me$ | H | H | F | H | 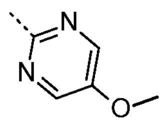 | (2S,5R) |

(continued)

| Co. No. | Ex. No. | $R^1$ | $R^2$ | $R^3$ | $R^4$ | $R^5$ | $R^6$ | Ar | Stereochemistry |
|---|---|---|---|---|---|---|---|---|---|
| 46 | B10 | Me | $SO_2Me$ | F | F | H | H | 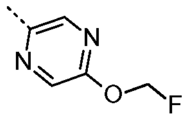 | (2R,5RS) |
| 48a | B15 | Me | $SO_2Me$ | $OCH_3$ | H | H | H | 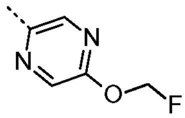 | (2R,3R,5R) |
| 48b | B15 | Me | $SO_2Me$ | $OCH_3$ | H | H | H | 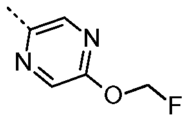 | (2R,3R,5S) |
| 49a | B12 | Me | CN | $OCH_3$ | H | H | H | 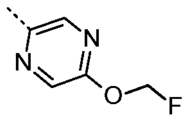 | (2R,3S,5S) |
| 49b | B12 | Me | CN | $OCH_3$ | H | H | H | 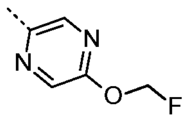 | (2R,3S,5R) |
| [c]Pr means cyclopropyl, [i]Pr means *iso*-propyl. | | | | | | | | | |

TABLE 1B

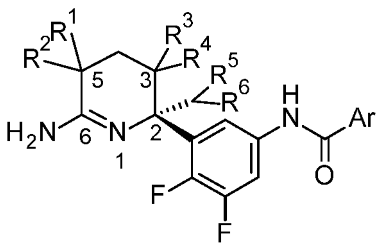

| Co. No. | Ex. No. | $R^1$ | $R^2$ | $R^3$ | $R^4$ | $R^5$ | $R^6$ | Ar | Stereochemistry |
|---|---|---|---|---|---|---|---|---|---|
| 47 | B14 | Me | $SO_2Me$ | H | H | F | H | 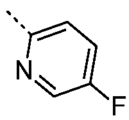 | (2S,5R) |

ANALYTICAL PART

LC-MS (LIQUID CHROMATOGRAPHY/MASS SPECTROMETRY)

LC-MS GENERAL PROCEDURE

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range,

dwell time...) in order to obtain ions allowing the identification of the compound's nominal monoisotopic molecular weight (MW) and/or exact mass monoisotopic molecular weight. Data acquisition was performed with appropriate software.

Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, $[M+CH_3COO]^-$, etc...). For molecules with multiple isotopic patterns (e.g. Br, Cl), the reported value is the one obtained for the lowest isotope mass. All results were obtained with experimental uncertainties that are commonly associated with the method used.

TABLE 2. LCMS Method codes (Flow expressed in mL/min; column temperature (T) in °C; Run time in minutes)

| Method | Instrument | Column | Mobile phase | Gradient | Flow<br>-------<br>Col T | Run time |
|---|---|---|---|---|---|---|
| 1 | Waters: Acquity® UPLC® - DAD and SQD | Waters : BEH C18 (1.7μm, 2.1*50mm) | A: 10mM $CH_3CO_2NH_4$ in 95% $H_2O$ + 5% $CH_3CN$ B: $CH_3CN$ | From 95% A to 5% A in 1.3 min, held for 0.7 min. | 0.8<br>-------<br>55 | 2 |
| 2 | Waters: Acquity® UPLC® - DAD and SQD | Waters : HSS T3 (1.8μm, 2.1*100mm) | A: 10mM $CH_3CO_2NH_4$ in 95% $H_2O$ + 5% $CH_3CN$ B: $CH_3CN$ | From 100% A to 5% A in 2.10min, to 0% A in 0.90min, to 5% A in 0.5min | 0.7<br>-------<br>55 | 3.5 |

MELTING POINTS

Values are either peak values or melt ranges, and are obtained with experimental uncertainties that are commonly associated with this analytical method.

DSC823e (indicated as DSC)

For a number of compounds, melting points were determined with a DSC823e (Mettler-Toledo). Melting points were measured with a temperature gradient of 10°C/minute. Maximum temperature was 300°C.

TABLE 3. Analytical data - melting point (m.p.) and LC/MS: $R_t$ means retention time (in minutes), $[M+H]^+$ means the protonated mass of the compound, $[M-H]^-$ means the deprotonated mass of the compound, method refers to the method used for (LC)MS. For some compounds, the exact mass was determined.

| Co. Nr. | Mp (°C) | $R_t$ | $[M+H]^+$ | $[M-H]^-$ | LCMS Method |
|---|---|---|---|---|---|
| 1 | | 0.82 | 468 | 466 | 1 |
| 2 | 122.96 (DSC) | 0.84 | 468 | 466 | 1 |
| 3 | | 1.72 + 1.74 | 415 | 413 | 2 |
| 4 | | 1.74 | 415 | 413 | 2 |
| 5 | | 1.72 | 415 | 413 | 2 |
| 6 | | 1.67 | 415 | 413 | 2 |
| 7 | | 1.72 | 415 | 413 | 2 |
| 8 | | 1.69 | 415 | 413 | 2 |
| 9 | 214.97 (DSC) | 1.5 | 450 | 448 | 2 |
| 10 | | 1.51 | 450 | 448 | 2 |
| 11 | | 1.78 | 433 | 431 | 2 |
| 12 | | 1.78 | 433 | 431 | 2 |
| 13 | | 1.79 | 433 | 431 | 2 |

(continued)

| Co. Nr. | Mp (°C) | $R_t$ | $[M+H]^+$ | $[M-H]^-$ | LCMS Method |
|---|---|---|---|---|---|
| 14 | | 1.79 | 431 | 433 | 2 |
| 15 | | 1.62 | 397 | 395 | 2 |
| 16 | | 1.57 | 397 | 395 | 2 |
| 17 | | 1.63 | 468 | 466 | 2 |
| 18 | | 1.63 | 468 | 466 | 2 |
| 19 | | 0.83 | 455 | 453 | 1 |
| 20 | 216.68 | 0.83 | 455 | 453 | 1 |
| | | 1.66 | 455 | 453 | 2 |
| 21 | | 1.7 | 476 | 474 | 2 |
| 22 | | 1.61 | 462 | 460 | 2 |
| 23 | | 0.91 | 504 | 502 | 1 |
| 24 | | 0.91 | 504 | 502 | 1 |
| 25 | | 0.89 | 481 | 379 | 1 |
| 26 | | 0.86 | 455.1 | 453.1 | 1 |
| 27 | | 1.72 | 429 | 427 | 2 |
| 28 | | 1.07 | 429 | 427 | 2 |
| 29a | | 0.88 | 469 | 467 | 1 |
| 29b | 168.45 | 1.84 | 469 | 467 | 2 |
| 30 | | 0.93 | 483 | 481 | 1 |
| 31 | | 0.88 | 469 | 467 | 1 |
| 32 | | 1.76 | 504 | 502 | 2 |
| 33 | | 1.83 | 483 | 481 | 2 |
| 34 | | 1.81 | 483 | 481 | 2 |
| 35 | | 1.75 | 518 | 516 | 2 |
| 36 | | 1.51 | 504 | 502 | 2 |
| 37 | | 1.76 | 482 | 480 | 2 |
| 38 | | 1.36 | 456 | 454 | 2 |
| 39 | | 1.86 | 536 | 534 | 2 |
| 40 | | 1.46 | 441 | 439 | 2 |
| 41 | | 1.62 | 486 | 484 | 2 |
| 42 | | 1.63 | 488 | 486 | 2 |
| 43 | | 1.57 | 506 | 504 | 2 |
| 44 | | 1.46 | 472 | 470 | 2 |
| 45 | | 1.4 | 468 | 466 | 2 |
| 46 | | 0.89 | 504 | 502 | 1 |
| 47 | | 1.75 | 473 | 471 | 2 |
| 48a | | 0.84 | 498 | 496 | 1 |
| 48b | | 0.84 | 498 | 496 | 1 |

(continued)

| Co. Nr. | Mp (°C) | $R_t$ | $[M+H]^+$ | $[M-H]^-$ | LCMS Method |
|---|---|---|---|---|---|
| 49a | | 1.65 | 445 | 443 | 2 |
| 49b | | 1.64 | 445 | 443 | 2 |

SFC-MS METHODS

The SFC measurement was performed using an Analytical Supercritical fluid chromatography (SFC) system composed by a binary pump for delivering carbon dioxide ($CO_2$) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

TABLE 4. Analytical SFC-MS Methods (Flow expressed in mL/min; column temperature (T) in °C; Run time in minutes, Backpressure (BPR) in bars.

| Method | Column | Mobile phase | Gradient | Flow<br>------------<br>Col T | Run time<br>------------<br>BPR |
|---|---|---|---|---|---|
| 1 | Daicel Chiralpak® AD-H column (5.0 μm, 250 x 4.6 mm) | A:$CO_2$ B: MeOH+0.2% $iPrNH_2$ | 20% B hold 4 min, to 50% in 1 min hold 2 min | 5<br>-------<br>40 | 7<br>-------<br>110 |
| 2 | Daicel Chiralpak® AD-H column (5.0 μm, 250 x 4.6 mm) | A:$CO_2$ B: EtOH+0.2% $iPrNH_2$ | 45% B hold 4 min, to 50% in 1 min hold 2 min | 5<br>-------<br>40 | 7<br>-------<br>110 |
| 3 | Whelk®-O-(R,R) column (5.0 μm, 250 x 4.6 mm) | A:$CO_2$ B: EtOH+0.2% $iPrNH_2$ | 20% B hold 4 min, to 50% in 1 min hold 2 min | 5<br>-------<br>40 | 7<br>-------<br>110 |
| 4 | Daicel Chiralpak® AD column (5.0 μm, 250 x 4.6 mm) | A:$CO_2$ B: EtOH+0.2% $iPrNH_2$ | 40% B hold 4 min, to 50% in 1 min, hold 2 min | 5<br>-------<br>40 | 7<br>-------<br>110 |
| 5 | Daicel Chiralpak® OD column (5.0 μm, 250 x 4.6 mm) | A:$CO_2$ B: EtOH+0.2% $iPrNH_2$ | 25% B hold 4 min, to 50% in 1 min, hold 2 min | 5<br>-------<br>40 | 7<br>-------<br>110 |
| 6 | Daicel Chiralpak® AD column (5.0 μm, 250 x 4.6 mm) | A:$CO_2$ B: EtOH+0.2% iPrNH2+3% H2O | From 10-40% B in 6 min, hold 3.5 min, to 20% in 0.1 min, hold 0.4 min | 2.5<br>-------<br>40 | 10<br>-------<br>110 |
| 7 | Daicel Chiralpak® AS3 column (3.0 μm, 150 x 4.6 mm) | A:$CO_2$ B: EtOH+0.2% iPrNH2 | 20% B hold 6 min, to 50% in 1 min hold 2.5 min | 2.5<br>-------<br>40 | 9.5<br>-------<br>110 |

(continued)

| Method | Column | Mobile phase | Gradient | Flow<br>------------<br>Col T | Run time<br>------------<br>BPR |
|---|---|---|---|---|---|
| 8 | Daicel Chiralpak® OJ-H column (5.0 μm, 250 x 4.6 mm) | A:$CO_2$ B: EtOH+0.2% $iPrNH_2$ | gradient 2: 10-50% B in 6 min, hold 3.5 min @50% | 2.5<br>------<br>40 | 9.5<br>------<br>110 |

TABLE 5. Analytical SFC data - $R_t$ means retention time (in minutes), $[M+H]^+$ means the protonated mass of the compound, method refers to the method used for (SFC)MS analysis of enantiomerically pure compounds.

| Co. Nr. | Rt | UV Area % | $[M+H]^+$ | Isomer Elution Order | SFCMS Method |
|---|---|---|---|---|---|
| 2 | 2.71 | 100.00 | 468 | | 8 |
| 6 | 2.37 | 100.00 | 415 | C | 3 |
| 8 | 1.63 | 100.00 | 415 | B | 3 |
| 7 | 1.4 | 100.00 | 415 | A | 3 |
| 10 | 2.88 | 99.40 | 450 | B | 2 |
| 9 | 1.47 | 97.58 | 450 | A | 2 |
| 14 | 4.04 | 100.00 | 433 | D | 1 |
| 13 | 2.62 | 100.00 | 433 | B | 1 |
| 12 | 3.03 | 100.00 | 433 | C | 1 |
| 11 | 2.38 | 100.00 | 433 | A | 1 |
| 20 | 1.51 | 100 | 455 | A | 5 |
| 34 | 1.35 | 100 | 483 | A | 4 |
| 33 | 2.61 | 100 | 483 | B | 4 |
| 28 | 3.4 | 100 | 429 | A | 6 |
| 27 | 4.08 | 100 | 429 | B | 6 |
| 29b | 1.57 | 100 | 469 | A | 7 |
| Isomer Elution Order: A means first eluting isomer; B means second eluting isomer; and so on. | | | | | |

NMR

For a number of compounds, $^1H$ NMR spectra were recorded on a Bruker DPX-400 spectrometer operating at 400 MHz, on a Bruker DPX-360 operating at 360 MHz, or on a Bruker Avance 600 spectrometer operating at 600 MHz, using CHLOROFORM-*d* (deuterated chloroform, $CDCl_3$) or DMSO-$d_6$ (deuterated DMSO, dimethyl-d6 sulfoxide) or BENZENE-$d_6$ (deuterated benzene, $C_6D_6$) or ACETONE-$d_6$ (deuterated acetone, $(CD_3)_2CO$) as solvents. Chemical shifts (δ) are reported in parts per million (ppm) relative to tetramethylsilane (TMS), which was used as internal standard.

TABLE 6. $^1H$ NMR results

| Co. No. | $^1H$ NMR result |
|---|---|
| 1 | $^1H$ NMR (400 MHz, DMSO-$d_6$) δ ppm 1.60 (s, 3 H) 1.70 - 1.83 (m, 2 H) 1.85 - 1.98 (m, 1 H) 2.31 (dt, *J*=13.6, 3.5 Hz, 1 H) 2.90 (s, 3 H) 4.02 (s, 3 H) 4.32 - 4.61 (m, 2 H) 6.03 (br s, 2 H) 7.17 (dd, *J*=12.0, 8.9 Hz, 1 H) 7.86 (dt, *J*=8.6, 3.5 Hz, 1 H) 7.91 (dd, *J*=7.3, 2.6 Hz, 1 H) 8.41 (d, *J*=1.3 Hz, 1 H) 8.88 (d, *J*=1.1 Hz, 1 H) 10.47 (s, 1 H) |

(continued)

| Co. No. | $^1$H NMR result |
|---|---|
| 2 | $^1$H NMR (400 MHz, DMSO-$d_6$) δ ppm 1.45 - 1.55 (m, 1 H) 1.56 (s, 3 H) 1.90 - 1.99 (m, 1 H) 2.24 (ddd, *J*=13.6, 10.6, 3.2 Hz, 1 H) 2.35 - 2.46 (m, 1 H) 3.10 (s, 3 H) 4.03 (s, 3 H) 4.55 (br d, *J*=48.0 Hz, 2 H) 5.95 (br s, 2 H) 7.15 (dd, *J*=12.1, 8.9 Hz, 1 H) 7.79 (ddd, *J*=8.8, 4.1, 2.8 Hz, 1 H) 7.84 (dd, *J*=7.3, 2.8 Hz, 1 H) 8.40 (d, *J*=1.2 Hz, 1 H) 8.88 (d, *J*=1.3 Hz, 1 H) 10.42 (s, 1 H) |
| 4 | $^1$H NMR (400 MHz, BENZENE-$d_6$) δ ppm 0.95 (s, 3 H) 1.19 - 1.30 (m, 1 H) 1.57 - 1.68 (m, 1 H) 1.68 - 1.78 (m, 1 H) 1.95 (ddd, *J*=13.8, 6.0, 2.8 Hz, 1 H) 3.44 (s, 3 H) 4.40 - 4.75 (m, 2 H) 5.10 (br s, 2 H) 6.79 (dd, *J*=11.7, 8.9 Hz, 1 H) 7.62 (d, *J*=1.2 Hz, 1 H) 7.71 - 7.84 (m, 1 H) 8.39 (dd, *J*=7.3, 2.8 Hz, 1 H) 9.10 (d, *J*=1.2 Hz, 1 H) 9.69 (s, 1 H) |
| 5 | $^1$H NMR (400 MHz, BENZENE-$d_6$) δ ppm 0.95 (s, 3 H) 1.17 (ddd, *J*=13.7, 10.5, 3.2 Hz, 1 H) 1.77 (ddd, *J*=13.4, 7.2, 3.6 Hz, 1 H) 1.88 - 1.99 (m, 1 H) 2.04 - 2.15 (m, 1 H) 3.52 (s, 3 H) 4.43 - 4.64 (m, 2 H) 6.75 (dd, *J*=11.5, 8.7 Hz, 1 H) 7.50 - 7.55 (m, 1 H) 7.63 (d, *J*=1.2 Hz, 1 H) 7.99 (dd, *J*=6.9, 2.8 Hz, 1 H) 9.01 (d, *J*=1.6 Hz, 1 H) 9.26 (br s, 1 H) |
| 6 | $^1$H NMR (600 MHz, BENZENE-$d_6$ + ACETONE- $d_6$) δ ppm 1.31 (dd, *J*=44.2, 15.1 Hz, 1 H) 1.46 (s, 3 H) 1.84 (s, 3 H) 2.22 (dt, *J*=15.0, 5.1 Hz, 1 H) 3.69 (s, 3 H) 5.26 (dd, *J*=47.4, 4.3 Hz, 1 H) 6.45 (br s, 2 H) 6.96 (dd, *J*=11.4, 8.9 Hz, 1 H) 7.83 - 7.87 (m, 1 H) 7.88 (s, 1 H) 8.20 (dd, *J*=6.8, 2.6 Hz, 1 H) 8.90 (s, 1 H) 10.02 (s, 1 H) |
| 7 | $^1$H NMR (400 MHz, CHLOROFORM-*d*) δ ppm 1.71 (dd, *J*=3.2, 2.0 Hz, 3 H) 1.83 (d, *J*=1.2 Hz, 3 H) 2.00 - 2.22 (m, 1 H) 2.32 (ddd, *J*=14.8, 8.0, 4.6 Hz, 1 H) 4.08 (s, 3 H) 4.46 (br s, 2 H) 5.20 - 5.40 (m, 1 H) 7.08 (dd, *J*= 11.7, 8.9 Hz, 1 H) 7.40 (dd, *J*=7.1, 2.6 Hz, 1 H) 7.97 (ddd, *J*=8.8, 4.3, 2.6 Hz, 1 H) 8.15 (d, *J*=1.6 Hz, 1 H) 9.01 (d, *J*=1.2 Hz, 1 H) 9.46 (br s, 1 H) |
| 8 | $^1$H NMR (600 MHz, BENZENE-$d_6$+ ACETONE- $d_6$) δ ppm 1.31 (dd, *J*=44.2, 15.1 Hz, 1 H) 1.46 (s, 3 H) 1.84 (s, 3 H) 2.22 (dt, *J*=15.0, 5.1 Hz, 1 H) 3.69 (s, 3 H) 5.26 (dd, *J*=47.4, 4.3 Hz, 1 H) 6.45 (br s, 2 H) 6.96 (dd, *J*=11.4, 8.9 Hz, 1 H) 7.83 - 7.87 (m, 1 H) 7.88 (s, 1 H) 8.20 (dd, *J*=6.8, 2.6 Hz, 1 H) 8.90 (s, 1 H) 10.02 (s, 1 H) |
| 9 | $^1$H NMR (600 MHz, BENZENE-$d_6$ + CHLOROFORM-*d*) δ ppm 1.48 (s, 3 H) 1.49 - 1.56 (m, 1 H) 1.51 - 1.56 (m, 1 H) 1.61 (d, *J*=1.2 Hz, 3 H) 1.62 - 1.68 (m, 1 H) 2.50 - 2.54 (m, 1 H) 2.56 (s, 3 H) 3.69 (s, 3 H) 6.85 (dd, *J*=11.6, 8.7 Hz, 1 H) 7.29 (dt, *J*=8.5, 3.3 Hz, 1 H) 7.78 (d, *J*=1.5 Hz, 1 H) 8.01 (dd, *J*=7.2, 2.8 Hz, 1 H) 8.92 (d, *J*=1.3 Hz, 1 H) 9.35 (s, 1 H) |
| 10 | $^1$H NMR (400 MHz, CHLOROFORM-*d*) δ ppm 1.60 (d, *J*=1.1 Hz, 3 H) 1.64 (s, 3 H) 1.78 (ddd, *J*=14.6, 7.4, 3.4 Hz, 1 H) 1.97 - 2.06 (m, 1 H) 2.23 - 2.32 (m, 1 H) 2.43 (ddd, *J*=14.4, 10.6, 3.6 Hz, 1 H) 3.02 (s, 3 H) 4.04 (s, 3 H) 5.07 (br s, 2 H) 7.03 (dd, *J*=11.7, 8.9 Hz, 1 H) 7.67 (dd, *J*=6.9, 2.8 Hz, 1 H) 7.80 (ddd, *J*=8.7, 4.0, 3.0 Hz, 1 H) 8.09 (d, *J*=1.2 Hz, 1 H) 8.97 (d, *J*=1.3 Hz, 1 H) 9.48 (br s, 1 H) |
| 11 | $^1$H NMR (600 MHz, CHLOROFORM-*d*) δ ppm 1.73 (s, 3 H) 1.95 - 2.03 (m, 2 H) 2.14 (ddd, *J*=14.7, 10.3, 4.8 Hz, 1 H) 2.30 - 2.36 (m, 1 H) 4.05 (s, 3 H) 5.34 (br s, 2 H) 6.20 (t, *J*=56.1 Hz, 1 H) 7.12 (dd, *J*=11.7, 8.9 Hz, 1 H) 7.66 (dd, *J*=6.7, 2.8 Hz, 1 H) 8.04 - 8.08 (m, 1 H) 8.07 (d, *J*=1.3 Hz, 1 H) 8.95 (d, *J*=1.3 Hz, 1 H) 9.60 (br s, 1 H) |
| 12 | $^1$H NMR (360 MHz, CHLOROFORM-*d*) δ ppm 1.73 (s, 3 H) 1.90 - 2.05 (m, 2 H) 2.07 - 2.21 (m, 1 H) 2.33 (dt, *J*=14.5, 4.5 Hz, 1 H) 4.05 (s, 3 H) 5.38 (br s, 2 H) 6.21 (t, *J*=56.2 Hz, 1 H) 7.12 (dd, *J*=11.7, 8.8 Hz, 1 H) 7.67 (dd, *J*=7.0, 2.6 Hz, 1 H) 8.04 - 8.10 (m, 2 H) 8.94 (d, *J*=1.1 Hz, 1 H) 9.62 (s, 1 H) |
| 13 | $^1$H NMR (600 MHz, BENZENE-$d_6$) δ ppm 0.79 (s, 3 H) 1.00 - 1.08 (m, 1 H) 1.67 (ddd, *J*=13.8, 6.3, 4.0 Hz, 1 H) 2.01 - 2.10 (m, 2 H) 3.47 (s, 3 H) 5.09 (br s, 2 H) 5.92 (t, *J*=56.1 Hz, 1 H) 6.73 (dd, *J*=11.5, 8.7 Hz, 1 H) 7.50 - 7.53 (m, 1 H) 7.57 (d, *J*=1.7 Hz, 1 H) 8.18 (dd, *J*=6.8, 2.7 Hz, 1 H) 8.97 (d, *J*=1.6 Hz, 1 H) 9.34 (s, 1 H) |
| 14 | $^1$H NMR (360 MHz, CHLOROFORM-*d*) δ ppm 1.58 (s, 3 H) 1.60 - 1.68 (m, 1 H) 2.24 - 2.36 (m, 3 H) 4.06 (s, 3 H) 5.30 (br s, 2 H) 6.15 (t, *J*=56.2 Hz, 1 H) 7.11 (dd, *J*=11.7, 8.8 Hz, 1 H) 7.71 (dd, *J*=6.8, 2.7 Hz, 1 H) 7.84 - 7.90 (m, 1 H) 8.06 (d, *J*=1.5 Hz, 1 H) 8.93 (d, *J*=1.1 Hz, 1 H) 9.53 (s, 1 H) |

(continued)

| Co. No. | $^1H$ NMR result |
|---|---|
| 15 | $^1H$ NMR (360 MHz, DMSO-$d_6$) δ ppm 1.30 (br t, *J*=13.0 Hz, 1 H) 1.52 (s, 3 H) 1.59 (s, 3 H) 1.71 (br t, *J*=12.8 Hz, 1 H) 2.05 (br d, *J*=14.3 Hz, 1 H) 2.21 (br d, *J*=13.5 Hz, 1 H) 4.02 (s, 3 H) 6.35 (br s, 2 H) 7.13 (dd, *J*=12.1, 8.8 Hz, 1 H) 7.65 (dd, *J*=7.3, 2.2 Hz, 1 H) 7.70 - 7.80 (m, 1 H) 8.41 (s, 1 H) 8.87 (s, 1 H) 10.55 (s, 1 H) |
| 16 | $^1H$ NMR (600 MHz, DMSO-$d_6$) δ ppm 1.46 (s, 3 H) 1.65 (s, 3 H) 1.85 - 2.03 (m, 4 H) 4.02 (s, 3 H) 6.06 (br s, 2 H) 7.14 (dd, *J*=11.8, 8.9 Hz, 1 H) 7.78 - 7.83 (m, 1 H) 7.85 (dd, *J*=7.3, 2.5 Hz, 1 H) 8.41 (d, *J*=1.3 Hz, 1 H) 8.89 (d, *J*=1.3 Hz, 1 H) 10.44 (br s, 1 H) |
| 17 | $^1H$ NMR (360 MHz, CHLOROFORM-*d*) δ ppm 1.63 (s, 3 H) 1.73 (br s, 3 H) 1.75 - 1.91 (m, 1 H) 2.96 - 3.08 (m, 1 H) 3.09 (d, *J*=1.5 Hz, 3 H) 4.07 (s, 3 H) 5.28 - 5.48 (m, 1 H) 5.65 (br s, 2 H) 7.09 (dd, *J*=11.3, 8.8 Hz, 1 H) 7.59 (dd, *J*=6.8, 2.7 Hz, 1 H) 7.67 - 7.77 (m, 1 H) 8.15 (d, *J*=1.5 Hz, 1 H) 9.01 (d, *J*=1.5 Hz, 1 H) 9.49 (br s, 1 H) |
| 18 | $^1H$ NMR (360 MHz, CHLOROFORM-*d*) δ ppm 1.69 (dd, *J*=2.9, 1.1 Hz, 3 H) 1.86 (s, 3 H) 1.92 - 2.14 (m, 1 H) 2.27 (ddd, *J*=14.9, 7.0, 4.4 Hz, 1 H) 2.80 (s, 3 H) 4.06 (s, 3 H) 5.43 - 5.61 (m, 1 H) 5.53 (br s, 2 H) 7.06 (dd, *J*=11.7, 8.8 Hz, 1 H) 7.60 (dt, *J*=8.6, 3.6 Hz, 1 H) 7.88 (dd, *J*=7.1, 2.7 Hz, 1 H) 8.11 (d, *J*=1.5 Hz, 1 H) 8.93 (d, *J*=1.5 Hz, 1 H) 9.48 (s, 1 H) |
| 19 | $^1H$ NMR (400 MHz, DMSO-$d_6$) δ ppm 1.60 (s, 3 H) 1.69 - 1.84 (m, 2 H) 1.86 - 1.97 (m, 1 H) 2.25 - 2.35 (m, 1 H) 2.91 (s, 3 H) 4.35 - 4.60 (m, 2 H) 6.04 (br s, 2 H) 7.18 (dd, *J*=12.0, 8.7 Hz, 1 H) 7.84 - 7.93 (m, 2 H) 7.97 (td, *J*=8.7, 2.9 Hz, 1 H) 8.22 (dd, *J*=8.7, 4.5 Hz, 1 H) 8.73 (d, *J*=2.9 Hz, 1 H) 10.58 (s, 1 H) |
| 20 | $^1H$ NMR (400 MHz, DMSO-$d_6$) δ ppm 1.41 - 1.53 (m, 1 H) 1.56 (s, 3 H) 1.85 - 1.99 (m, 1 H) 2.16 - 2.27 (m, 1 H) 2.34 - 2.45 (m, 1 H) 3.10 (s, 3 H) 4.44 - 4.67 (m, 2 H) 6.01 (br s, 2 H) 7.16 (br dd, *J*=11.8, 8.9 Hz, 1 H) 7.75 - 7.87 (m, 2 H) 7.97 (td, *J*=8.7, 2.9 Hz, 1 H) 8.22 (dd, *J*=8.7, 4.5 Hz, 1 H) 8.73 (d, *J*=2.9 Hz, 1 H) 10.63 (br s, 1 H) |
| 21 | $^1H$ NMR (360 MHz, DMSO-$d_6$) δ ppm 1.39 - 1.49 (m, 1 H) 1.53 (s, 3 H) 1.82 - 1.96 (m, 1 H) 2.11 - 2.25 (m, 1 H) 2.32 - 2.44 (m, 1 H) 2.53 (br s, 3 H) 3.09 (br s, 3 H) 4.39 - 4.69 (m, 2 H) 6.02 (br s, 2 H) 7.17 (dd, *J*= 11.7, 8.8 Hz, 1 H) 7.67 (dd, *J*=7.3, 2.6 Hz, 1 H) 7.75 - 7.84 (m, 1 H) 8.39 (dd, *J*=2.2, 0.7 Hz, 1 H) 8.98 (dd, *J*=1.8, 0.7 Hz, 1 H) 10.75 (br s, 1 H) |
| 22 | $^1H$ NMR (360 MHz, DMSO-$d_6$) δ ppm 1.40 - 1.52 (m, 1 H) 1.56 (s, 3 H) 1.85 - 1.97 (m, 1 H) 2.14 - 2.26 (m, 1 H) 2.33 - 2.45 (m, 1 H) 3.10 (s, 3 H) 4.42 - 4.68 (m, 2 H) 6.03 (br s, 2 H) 7.19 (dd, *J*= 11.7, 8.8 Hz, 1 H) 7.77 - 7.90 (m, 2 H) 8.28 (dd, *J*=8.4, 0.7 Hz, 1 H) 8.58 (dd, *J*=8.2, 2.0 Hz, 1 H) 9.20 (dd, *J*=2.2, 0.7 Hz, 1 H) 10.90 (s, 1 H) |
| 23 | $^1H$ NMR (360 MHz, CHLOROFORM-*d*) δ ppm 1.82 (t, *J*=2.7 Hz, 3 H) 1.90 (s, 3 H) 2.16 - 2.32 (m, 1 H) 2.72 (ddd, *J*=26.5, 14.6, 4.9 Hz, 1 H) 2.98 (s, 3 H) 5.41 (br s, 2 H) 6.16 (dq, *J*=51.2, 2.0 Hz, 2 H) 7.10 (dd, *J*=11.7, 8.8 Hz, 1 H) 7.75 (dd, *J*=7.0, 2.9 Hz, 1 H) 7.83 (dt, *J*=8.8, 3.3 Hz, 1 H) 8.31 (d, *J*=1.1 Hz, 1 H) 9.06 (d, *J*= 1.1 Hz, 1 H) 9.51 (s, 1 H) |
| 24 | $^1H$ NMR (400 MHz, CHLOROFORM-*d*) δ ppm 1.84 (s, 3 H) 1.87 (s, 3 H) 2.30 (dt, *J*=14.3, 10.4 Hz, 1 H) 2.85 - 3.02 (m, 1 H) 3.05 (s, 3 H) 5.36 (br s, 2 H) 6.16 (d, *J*=51.3 Hz, 2 H) 7.11 (dd, *J*=11.3, 8.9 Hz, 1 H) 7.70 (dd, *J*=6.7, 2.6 Hz, 1 H) 7.83 (dt, *J*=8.6, 3.4 Hz, 1 H) 8.29 (d, *J*=1.2 Hz, 1 H) 9.09 (d, *J*=1.2 Hz, 1 H) 9.49 (br s, 1 H) |
| 25 | $^1H$ NMR (400 MHz, CHLOROFORM-*d*) δ ppm 1.09 - 1.23 (m, 2 H) 1.31 - 1.41 (m, 2 H) 1.68 (s, 3 H) 1.69 - 1.77 (m, 1 H) 2.10 - 2.18 (m, 1 H) 2.49 - 2.62 (m, 2 H) 2.67 (ddd, *J*=14.7, 8.6, 3.7 Hz, 1 H) 4.51 (dd, *J*=47.5, 8.8 Hz, 1 H) 4.85 (ddd, *J*=47.8, 8.8, 1.5 Hz, 1 H) 7.08 (dd, *J*=11.4, 8.8 Hz, 1 H) 7.55 - 7.64 (m, 1 H) 7.69 (dd, *J*=6.9, 2.8 Hz, 1 H) 7.88 (ddd, *J*=8.8, 4.2, 2.9 Hz, 1 H) 8.32 (dd, *J*=8.6, 4.6 Hz, 1 H) 8.45 (d, *J*=2.6 Hz, 1 H) 9.80 (s, 1 H) |
| 26 | $^1H$ NMR (360 MHz, CHLOROFORM-*d*) δ ppm 1.65 (s, 3 H) 2.00 - 2.14 (m, 2 H) 2.27 - 2.37 (m, 1 H) 2.38 - 2.49 (m, 1 H) 3.07 (s, 3 H) 4.71 (dd, *J*=46.8, 10.6 Hz, 1 H) 5.08 - 5.33 (m, 3 H) 7.05 (dd, *J*=11.5, 8.6 Hz, 1 H) 7.59 (td, *J*=8.3, 2.7 Hz, 1 H) 7.67 (dd, *J*=7.1, 2.7 Hz, 1 H) 7.77 (dt, *J*=7.3, 4.2 Hz, 1 H) 8.31 (dd, *J*=8.6, 4.6 Hz, 1 H) 8.43 (d, *J*=2.6 Hz, 1 H) 9.78 (s, 1 H) |

(continued)

| Co. No. | $^1$H NMR result |
|---|---|
| 27 | $^1$H NMR (400 MHz, CHLOROFORM-*d*) δ ppm 1.41 (dd, *J*=22.2, 3.2 Hz, 3 H) 1.81 (t, *J*=3.4 Hz, 3 H) 1.88 (s, 3 H) 2.00 (dd, *J*=38.3, 14.5 Hz, 1 H) 2.29 (dd, *J*=14.9, 4.4 Hz, 1 H) 4.06 (s, 3 H) 4.71 (br s, 2 H) 7.02 (dd, J=12.1, 8.5 Hz, 1 H) 7.31 (dt, *J*=8.5, 3.2 Hz, 1 H) 7.75 (dd, *J*=7.3, 2.4 Hz, 1 H) 8.14 (d, *J*=1.6 Hz, 1 H) 8.98 (d, *J*=1.2 Hz, 1 H) 9.46 (s, 1 H) |
| 28 | $^1$H NMR (400 MHz, DMSO-$d_6$) δ ppm 1.13 (d, *J*=22.6 Hz, 3 H) 1.60 (br s, 3 H) 1.72 (s, 3 H) 2.22 - 2.37 (m, 2 H) 4.02 (s, 3 H) 6.19 (br s, 2 H) 7.08 - 7.14 (m, 1 H) 7.83 - 7.88 (m, 2 H) 8.40 (d, *J*=1.2 Hz, 1 H) 8.89 (d, *J*=1.2 Hz, 1 H) 10.48 (s, 1 H) |
| 29a | $^1$H NMR (400 MHz, CHLOROFORM-*d*) δ ppm 1.21 (t, *J*=7.7 Hz, 3 H) 1.85 - 1.95 (m, 1 H) 2.10 - 2.20 (m, 2 H) 2.22 - 2.37 (m, 2 H) 2.46 - 2.55 (m, 1 H) 2.84 (s, 3 H) 4.39 - 4.76 (m, 2 H) 7.08 (dd, *J*=11.5, 8.7 Hz, 1 H) 7.54 - 7.65 (m, 2 H) 7.95 (dd, *J*=6.9, 2.8 Hz, 1 H) 8.28 (dd, *J*=8.7, 4.6 Hz, 1 H) 8.44 (d, *J*=2.4 Hz, 1 H) 9.76 (s, 1 H) |
| 29b | $^1$H NMR (400 MHz, CHLOROFORM-*d*) δ ppm 0.97 (t, *J*=7.4 Hz, 3 H) 1.86 - 1.96 (m, 1 H) 2.03 (dq, *J*=14.4, 7.3 Hz, 1 H) 2.15 - 2.29 (m, 2 H) 2.38 - 2.53 (m, 2 H) 3.06 (s, 3 H) 4.44 (dd, *J*=47.4, 8.7 Hz, 1 H) 4.85 (dd, *J*=47.4, 8.0 Hz, 1 H) 5.48 (br s, 2 H) 7.08 (dd, *J*= 11.4, 8.8 Hz, 1 H) 7.59 (td, *J*=8.4, 2.9 Hz, 1 H) 7.74 (dd, *J*=6.8, 2.6 Hz, 1 H) 7.89 (dt, *J*=8.7, 3.4 Hz, 1 H) 8.31 (dd, *J*=8.7, 4.5 Hz, 1 H) 8.43 (d, *J*=2.6 Hz, 1 H) 9.79 (s, 1 H) |
| 30 | $^1$H NMR (400 MHz, CHLOROFORM-*d*) δ ppm 0.95 (t, *J*=7.5 Hz, 3 H) 1.47 (t, *J*=7.5 Hz, 3 H) 1.88 (ddd, *J*=14.6, 10.6, 3.8 Hz, 1 H) 1.99 - 2.08 (m, 1 H) 2.15 - 2.25 (m, 1 H) 2.28 (td, *J*=7.1, 4.4 Hz, 1 H) 2.35 - 2.43 (m, 1 H) 2.43 - 2.50 (m, 1 H) 3.13 - 3.30 (m, 2 H) 4.35 - 4.89 (m, 2 H) 7.07 (dd, *J*=11.5, 8.7 Hz, 1 H) 7.55 - 7.62 (m, 1 H) 7.72 (dd, *J*=6.9, 2.8 Hz, 1 H) 7.89 (ddd, *J*=8.9, 4.0, 2.8 Hz, 1 H) 8.27 - 8.32 (m, 1 H) 8.42 (d, *J*=2.8 Hz, 1 H) 9.78 (s, 1 H) |
| 31 | $^1$H NMR (400 MHz, CHLOROFORM-*d*) δ ppm 1.48 (t, *J*=7.5 Hz, 3 H) 1.68 (s, 3 H) 1.77 (br d, *J*=6.9 Hz, 1 H) 1.99 - 2.08 (m, 1 H) 2.53 - 2.60 (m, 1 H) 2.56 - 2.63 (m, 1 H) 3.20 (q, *J*=7.3 Hz, 2 H) 4.41 (br dd, *J*=47.4, 8.3 Hz, 1 H) 4.91 (br dd, *J*=48.4, 8.5 Hz, 1 H) 5.47 (br s, 2 H) 7.07 (dd, *J*=11.5, 8.7 Hz, 1 H) 7.60 (td, *J*=8.3, 2.4 Hz, 1 H) 7.73 (dd, *J*=6.9, 2.8 Hz, 1 H) 7.88 (br dt, *J*=8.4, 3.4 Hz, 1 H) 8.33 (dd, *J*=8.7, 4.6 Hz, 1 H) 8.45 (d, *J*=2.8 Hz, 1 H) 9.79 (s, 1 H) |
| 32 | $^1$H NMR (360 MHz, DMSO-$d_6$) δ ppm 1.46 (br t, *J*=11.3 Hz, 1 H) 1.56 (s, 3 H) 1.86 - 1.99 (m, 1 H) 2.12 - 2.25 (m, 1 H) 2.30 - 2.48 (m, 1 H) 3.11 (s, 3 H) 4.43 - 4.68 (m, 2 H) 6.04 (br s, 2 H) 7.18 (dd, *J*= 11.7, 8.8 Hz, 1 H) 7.54 - 8.05 (m, 3 H) 8.70 (d, *J*=1.5 Hz, 1 H) 8.96 (d, *J*=1.1 Hz, 1 H) 10.75 (s, 1 H) |
| 33 | $^1$H NMR (600 MHz, DMSO-$d_6$) δ ppm 1.31 (d, *J*=6.7 Hz, 3 H) 1.35 (d, *J*=6.7 Hz, 3 H) 1.43 - 1.50 (m, 1 H) 1.56 (s, 3 H) 1.99 - 2.04 (m, 1 H) 2.09 - 2.15 (m, 1 H) 2.37 - 2.43 (m, 1 H) 3.70 - 3.78 (m, 1 H) 4.46 - 4.66 (m, 2 H) 6.03 (br s, 2 H) 7.17 (dd, *J*=11.7, 8.7 Hz, 1 H) 7.78 - 7.83 (m, 2 H) 7.97 (td, *J*=8.7, 2.9 Hz, 1 H) 8.22 (dd, *J*=8.7, 4.6 Hz, 1 H) 8.73 (d, *J*=2.8 Hz, 1 H) 10.68 (s, 1 H) |
| 34 | $^1$H NMR (600 MHz, DMSO-$d_6$) δ ppm 1.00 (d, *J*=6.9 Hz, 3 H) 1.22 (d, *J*=6.9 Hz, 3 H) 1.63 (s, 3 H) 1.75 - 1.85 (m, 2 H) 1.89 - 1.97 (m, 1 H) 2.33 (dt, *J*=13.8, 3.8 Hz, 1 H) 3.47 - 3.56 (m, 1 H) 4.47 (br ddd, *J*=56.4, 47.7, 8.6 Hz, 2 H) 6.01 (br s, 2 H) 7.19 (dd, *J*=11.9, 8.8 Hz, 1 H) 7.74 - 7.79 (m, 1 H) 7.98 (td, *J*=8.7, 2.9 Hz, 1 H) 8.01 (dd, *J*=7.1, 2.7 Hz, 1 H) 8.22 (dd, *J*=8.7, 4.6 Hz, 1 H) 8.74 (d, *J*=2.8 Hz, 1 H) 10.59 (s, 1 H) |
| 35 | $^1$H NMR (360 MHz, DMSO-$d_6$) δ ppm 1.39 - 1.51 (m, 1 H) 1.56 (s, 3 H) 1.84 - 1.96 (m, 1 H) 2.14 - 2.24 (m, 1 H) 2.33 - 2.43 (m, 1 H) 3.10 (s, 3 H) 4.39 - 4.66 (m, 2 H) 4.74 (td, *J*=15.2, 3.3 Hz, 2 H) 6.03 (br s, 2 H) 6.28 - 6.69 (m, 1 H) 7.12 - 7.21 (m, 1 H) 7.76 - 7.90 (m, 2 H) 8.55 (s, 1 H) 8.89 (s, 1 H) 10.63 (br s, 1 H) |
| 36 | $^1$H NMR (360 MHz, DMSO-$d_6$) δ ppm 1.38 - 1.51 (m, 1 H) 1.56 (s, 3 H) 1.86 - 1.95 (m, 1 H) 2.13 - 2.25 (m, 1 H) 2.33 - 2.42 (m, 1 H) 3.10 (br s, 3 H) 4.43 - 4.68 (m, 2 H) 6.03 (br s, 2 H) 7.14 - 7.24 (m, 1 H) 7.54 (t, *J*=72.3 Hz, 1 H) 7.75 - 7.82 (m, 2 H) 8.98 (s, 2 H) 10.83 (s, 1 H) |
| 37 | $^1$H NMR (360 MHz, DMSO-$d_6$) δ ppm 1.39 (t, *J*=7.0 Hz, 3 H) 1.42 - 1.51 (m, 1 H) 1.56 (s, 3 H) 1.84 - 1.97 (m, 1 H) 2.14 - 2.27 (m, 1 H) 2.32 - 2.43 (m, 1 H) 3.10 (s, 3 H) 4.39 - 4.54 (m, 3 H) 4.55 - 4.68 (m, 1 H) 6.03 (br s, 2 H) 7.16 (dd, *J*=11.7, 8.8 Hz, 1 H) 7.75 - 7.86 (m, 2 H) 8.39 (d, *J*=1.1 Hz, 1 H) 8.86 (d, *J*=1.1 Hz, 1 H) 10.55 (s, 1 H) |

(continued)

| Co. No. | $^1H$ NMR result |
|---|---|
| 38 | $^1H$ NMR (360 MHz, DMSO-$d_6$) δ ppm 1.39 - 1.50 (m, 1 H) 1.56 (s, 3 H) 1.84 - 1.94 (m, 1 H) 2.12 - 2.24 (m, 1 H) 2.33 - 2.43 (m, 1 H) 3.10 (s, 3 H) 4.42 - 4.68 (m, 2 H) 6.03 (br s, 2 H) 7.18 (dd, *J*=11.9, 9.0 Hz, 1 H) 7.69 - 7.84 (m, 2 H) 9.11 (s, 2 H) 10.84 (br s, 1 H) |
| 39 | $^1H$ NMR (360 MHz, DMSO-$d_6$) δ ppm 1.39 - 1.51 (m, 1 H) 1.56 (s, 3 H) 1.85 - 1.96 (m, 1 H) 2.14 - 2.27 (m, 1 H) 2.31 - 2.44 (m, 1 H) 3.10 (s, 3 H) 4.41 - 4.66 (m, 2 H) 5.16 (q, *J*=8.9 Hz, 2 H) 6.04 (br s, 2 H) 7.17 (dd, *J*=11.9, 8.6 Hz, 1 H) 7.76 - 7.88 (m, 2 H) 8.62 (d, *J*=1.5 Hz, 1 H) 8.91 (d, *J*=1.5 Hz, 1 H) 10.67 (br s, 1 H) |
| 40 | $^1H$ NMR (360 MHz, DMSO-$d_6$) δ ppm 1.37 - 1.48 (m, 1 H) 1.55 (s, 3 H) 1.83 - 1.94 (m, 1 H) 2.09 - 2.21 (m, 1 H) 2.30 - 2.40 (m, 1 H) 2.51 (br s, 3 H) 3.09 (s, 3 H) 4.42 - 4.67 (m, 2 H) 6.02 (br s, 2 H) 7.13 (dd, *J*=11.7, 8.8 Hz, 1 H) 7.63 - 7.71 (m, 1 H) 7.75 (dd, *J*=7.5, 2.7 Hz, 1 H) 8.63 (s, 1 H) 10.21 (br s, 1 H) |
| 41 | $^1H$ NMR (360 MHz, DMSO-$d_6$) δ ppm 1.38 - 1.51 (m, 1 H) 1.56 (s, 3 H) 1.86 - 1.97 (m, 1 H) 2.12 - 2.25 (m, 1 H) 2.33 - 2.43 (m, 1 H) 3.10 (s, 3 H) 4.40 - 4.67 (m, 2 H) 6.04 (brs, 2 H) 6.20 (d, *J*=51.6 Hz, 2 H) 7.17 (dd, *J*=11.9, 9.0 Hz, 1 H) 7.76 - 7.89 (m, 2 H) 8.59 (d, *J*=1.5 Hz, 1 H) 8.95 (d, *J*=1.5 Hz, 1 H) 10.68 (br s, 1 H) |
| 42 | $^1H$ NMR (360 MHz, DMSO-$d_6$) δ ppm 1.40 - 1.51 (m, 1 H) 1.56 (s, 3 H) 1.85 - 1.97 (m, 1 H) 2.12 - 2.26 (m, 1 H) 2.33 - 2.43 (m, 1 H) 3.11 (s, 3 H) 4.39 - 4.69 (m, 2 H) 6.05 (br s, 2 H) 7.26 (t, *J*=53.8 Hz, 1 H) 7.20 (dd, *J*=11.9, 9.0 Hz, 1 H) 7.78 - 7.84 (m, 1 H) 7.84 - 7.89 (m, 1 H) 9.10 (s, 1 H) 9.38 (s, 1 H) 10.98 (br s, 1 H) |
| 43 | $^1H$ NMR (360 MHz, DMSO-$d_6$) δ ppm 1.38 - 1.50 (m, 1 H) 1.56 (s, 3 H) 1.82 - 1.97 (m, 1 H) 2.12 - 2.24 (m, 1 H) 2.31 - 2.45 (m, 1 H) 3.10 (s, 3 H) 4.42 - 4.68 (m, 2 H) 6.05 (br s, 2 H) 7.20 (dd, *J*=11.7, 8.8 Hz, 1 H) 7.72 - 7.86 (m, 2 H) 9.50 (s, 2 H) 11.01 (brs, 1 H) |
| 44 | $^1H$ NMR (360 MHz, DMSO-$d_6$) δ ppm 1.38 - 1.51 (m, 1 H) 1.56 (s, 3 H) 1.82 - 1.97 (m, 1 H) 2.18 (m, *J*=11.2, 11.2 Hz, 1 H) 2.30 - 2.47 (m, 1 H) 3.10 (s, 3 H) 4.43 - 4.68 (m, 2 H) 6.04 (br s, 2 H) 7.18 (dd, *J*=11.7, 8.4 Hz, 1 H) 7.74 - 7.87 (m, 2 H) 9.16 (s, 2 H) 10.88 (s, 1 H) |
| 45 | $^1H$ NMR (360 MHz, DMSO-$d_6$) δ ppm 1.38 - 1.52 (m, 1 H) 1.56 (s, 3 H) 1.82 - 1.97 (m, 1 H) 2.11 - 2.25 (m, 1 H) 2.32 - 2.43 (m, 1 H) 3.10 (s, 3 H) 4.02 (s, 3 H) 4.43 - 4.67 (m, 2 H) 6.03 (br s, 2 H) 7.16 (dd, *J*=11.9, 8.6 Hz, 1 H) 7.74 - 7.82 (m, 2 H) 8.72 (s, 2 H) 10.68 (br s, 1 H) |
| 47 | $^1H$ NMR (400 MHz, CHLOROFORM-*d*) δ ppm 1.80 - 1.95 (m, 4 H) 2.20 - 2.31 (m, 1 H) 2.60 - 2.75 (m, 2 H) 3.15 (s, 3 H) 4.83 (ddd, *J*=88.7, 46.9, 9.5 Hz, 2 H) 7.44 - 7.53 (m, 1 H) 7.59 (td, *J*=8.3, 2.2 Hz, 1 H) 8.02 (br dd, *J*=10.8, 7.0 Hz, 1 H) 8.28 (dd, *J*=8.5, 4.3 Hz, 1 H) 8.45 (br s, 1 H) 10.05 (br s, 1 H) |
| 48a | $^1H$ NMR (400 MHz, CHLOROFORM-*d*) δ ppm 1.61 (s, 3 H) 1.88 (s, 3 H) 2.32 (br dd, *J*=14.7, 3.8 Hz, 1 H) 2.54 (br d, *J*=14.1 Hz, 1 H) 3.01 (s, 3 H) 3.19 (s, 3 H) 4.16 - 4.23 (m, 1 H) 6.15 (br d, *J*=51.3 Hz, 2 H) 7.06 (dd, *J*=11.7, 8.9 Hz, 1 H) 7.76 - 7.88 (m, 1 H) 7.97 - 8.10 (m, 1 H) 8.29 (s, 1 H) 9.08 (s, 1 H) 9.52 (br s, 1 H) |
| 48b | $^1H$ NMR (360 MHz, CHLOROFORM-*d*) δ ppm 1.60 (s, 3 H) 1.79 (s, 3 H) 2.16 (dd, *J*=15.9, 3.1 Hz, 1 H) 2.93 (dd, *J*=16.1, 4.8 Hz, 1 H) 3.09 (s, 3 H) 3.22 (s, 3 H) 3.92 (m, *J*=3.3, 3.3 Hz, 1 H) 6.14 (dq, *J*=51.1, 2.1 Hz, 2 H) 7.06 (dd, *J*=12.1, 8.8 Hz, 1 H) 7.86 (dd, *J*=7.0, 2.9 Hz, 1 H) 7.88 - 7.95 (m, 1 H) 8.28 (d, *J*=1.1 Hz, 1 H) 9.06 (d, *J*=1.1 Hz, 1 H) 9.54 (br s, 1 H) |
| 49a | $^1H$ NMR (600 MHz, DMSO-$d_6$) δ ppm 1.24 (br d, *J*=14.2 Hz, 1 H) 1.49 (d, *J*=1.3 Hz, 3 H) 1.58 (s, 3 H) 2.42 (dd, *J*=14.7, 4.6 Hz, 1 H) 3.38 (s, 3 H) 3.71 (br d, *J*=4.0 Hz, 1 H) 6.12 - 6.25 (m, 4 H) 7.13 (dd, *J*= 11.8, 8.7 Hz, 1 H) 7.66 (br dd, *J*=7.0, 1.7 Hz, 1 H) 7.68 - 7.74 (m, 1 H) 8.57 (d, *J*=1.3 Hz, 1 H) 8.93 (d, *J*=1.3 Hz, 1 H) 10.69 (br s, 1 H) |
| 49b | $^1H$ NMR (600 MHz, DMSO-$d_6$) δ ppm 1.44 (s, 3 H) 1.66 (s, 3 H) 2.00 (dd, *J*=13.6, 9.7 Hz, 1 H) 2.07 - 2.13 (m, 1 H) 3.16 (s, 3 H) 3.80 (dd, *J*=9.6, 3.3 Hz, 1 H) 5.96 (br s, 2 H) 6.20 (d, *J*=51.6 Hz, 2 H) 7.11 (dd, *J*=11.9, 8.7 Hz, 1 H) 7.80 - 7.88 (m, 2 H) 8.58 (d, *J*=1.3 Hz, 1 H) 8.96 (d, *J*=1.3 Hz, 1 H) 10.60 (br s, 1 H) |

PHARMACOLOGICAL EXAMPLES

The compounds provided in the present invention are inhibitors of the beta-site APP-cleaving enzyme 1 (BACE1). Inhibition of BACE1, an aspartic protease, is believed to be relevant for treatment of Alzheimer's Disease

(AD). The production and accumulation of beta-amyloid peptides (Abeta) from the beta-amyloid precursor protein (APP) is believed to play a key role in the onset and progression of AD. Abeta is produced from the amyloid precursor protein (APP) by sequential cleavage at the N- and C-termini of the Abeta domain by beta-secretase and gamma-secretase, respectively.

Compounds of Formula (I) are expected to have their effect substantially at BACE1 by virtue of their ability to inhibit the enzymatic activity. The behaviour of such inhibitors tested using a biochemical Fluorescence Resonance Energy Transfer (FRET) based assay and a cellular αLisa assay in SKNBE2 cells described below and which are suitable for the identification of such compounds, and more particularly the compounds according to Formula (I), are shown in Table 8 and Table 9.

BACE1 BIOCHEMICAL FRET BASED ASSAY

This assay is a Fluorescence Resonance Energy Transfer Assay (FRET) based assay. The substrate for this assay is an APP derived 13 amino acids peptide that contains the 'Swedish' Lys-Met/Asn-Leu mutation of the amyloid precursor protein (APP) beta-secretase cleavage site. This substrate also contains two fluorophores: (7-methoxycoumarin-4-yl) acetic acid (Mca) is a fluorescent donor with excitation wavelength at 320 nm and emission at 405 nm and 2,4-Dinitrophenyl (Dnp) is a proprietary quencher acceptor. The distance between those two groups has been selected so that upon light excitation, the donor fluorescence energy is significantly quenched by the acceptor, through resonance energy transfer. Upon cleavage by BACE1, the fluorophore Mca is separated from the quenching group Dnp, restoring the full fluorescence yield of the donor. The increase in fluorescence is linearly related to the rate of proteolysis.

Method 1

Briefly in a 384-well format recombinant BACE1 protein in a final concentration of 1 μg/ml is incubated for 120 minutes at room temperature with 10 μm substrate in incubation buffer (40 mM Citrate buffer pH 5.0, 0.04% PEG, 4% DMSO) in the absence or presence of compound. Next the amount of proteolysis is directly measured by fluorescence measurement at T=0 and T=120 (excitation at 320 nm and emission at 405 nm). Results are expressed in RFU (Relative Fluorescence Units), as difference between T120 and T0.

A best-fit curve is fitted by a minimum sum of squares method to the plot of % Controlmin versus compound concentration. From this an $IC_{50}$ value (inhibitory concentration causing 50% inhibition of activity) can be obtained.

LC = Median of the low control values = Low control: Reaction without enzyme

HC = Median of the High control values = High Control: Reaction with enzyme

$$\%Effect = 100-[(sample-LC) / (HC-LC) *100]$$

$$\%Control = (sample /HC)*100$$

$$\%Controlmin = (sample-LC) / (HC-LC) *100$$

The following exemplified compounds were tested essentially as described above and exhibited the following the activity:

TABLE 7.

| Co. No. | Biochemical FRET based assay - Method 1 $pIC_{50}$ |
|---|---|
| 1 | 5.58 |
| 2 | 8.64 |
| 3 | 7.29 |
| 4 | 6.07 |
| 5 | 7.27 |

(continued)

| Co. No. | Biochemical FRET based assay - Method 1 $pIC_{50}$ |
|---|---|
| 6 | 6.84 |
| 7 | 7.1 |
| 8 | 7.89 |
| 9 | 7.11 |
| 10 | 8.41 |
| 11+12+ 13+14 | 6.84 |
| 11 | 6.86 |
| 12 | 5.17 |
| 13 | <5 |
| 14 | 7.93 |
| 15 | 8.01 |
| 16 | 6.59 |
| 17 | 7.51 |
| 18 | 5.24 |
| 19 | 6.42 |
| 20 | 8.52 |
| 21 | 8.86 |
| 22 | 8.95 |
| 23 | 5.78 |
| 24 | 7.04 |
| 25 | 8.62 |
| 26 | 7.83 |
| 27 | 7.38 |
| 28 | 5.94 |
| 29a | 6.06 |
| 29b | 7.3 |
| 30 | 7.8 |
| 31 | 8.77 |
| 32 | 8.98 |
| 33 | 8.37 |
| 34 | 5.44 |
| 35 | 8.43 |
| 36 | 8.37 |
| 37 | 8.43 |
| 38 | 8.41 |
| 39 | 8.89 |
| 40 | 8.34 |

(continued)

| Co. No. | Biochemical FRET based assay - Method 1 $pIC_{50}$ |
|---|---|
| 41 | 8.66 |
| 42 | 8.42 |
| 43 | 8.04 |
| 44 | 8.44 |
| 45 | 8.5 |
| 47 | 8.45 |
| 48 | <5 |
| 48a | 7.62 |
| 48b | 6.48 |
| 49a | 7.47 |
| 49b | 6.77 |

CELLULAR αLISA ASSAY IN SKNBE2 CELLS

In two αLisa assays the levels of Abeta 1-42 produced and secreted into the medium of human neuroblastoma SKNBE2 cells are quantified. The assay is based on the human neuroblastoma SKNBE2 expressing the wild type Amyloid Precursor Protein (hAPP695). The compounds are diluted and added to these cells, incubated for 18 hours and then measurements of Abeta 1-42 are taken. Abeta 1-42 are measured by sandwich αLisa. αLisa is a sandwich assay using biotinylated antibody AbN/25 attached to streptavidin coated beads and antibody cAb42/26 conjugated acceptor beads for the detection of Abeta 1-42 respectively. In the presence of Abeta 1-42, the beads come into close proximity. The excitation of the donor beads provokes the release of singlet oxygen molecules that trigger a cascade of energy transfer in the acceptor beads, resulting in light emission. Light emission is measured after 1 hour incubation (excitation at 650 nm and emission at 615 nm).

A best-fit curve is fitted by a minimum sum of squares method to the plot of %Controlmin versus compound concentration. From this an $IC_{50}$ value (inhibitory concentration causing 50% inhibition of activity) can be obtained.

LC = Median of the low control values = Low control: cells preincubated without compound, without biotinylated Ab in the αLisa

HC = Median of the High control values

= High Control: cells preincubated without compound

$$\%Effect = 100-[(sample-LC) / (HC-LC) *100]$$

$$\%Control = (sample /HC)*100$$

$$\%Controlmin = (sample-LC) / (HC-LC) *100$$

The following exemplified compounds were tested essentially as described above and exhibited the following the activity:

TABLE 8.

| Co. No. | Cellular αLisa assay in SKNBE2 cells Abeta 42 $pIC_{50}$ | Cellular αLisa assay in SKNBE2 cells Abetatotal $pIC_{50}$ |
|---|---|---|
| 1 | 5.87 | n.t. |
| 2 | 8.83 | n.t. |
| 3 | 7.25 | n.t. |
| 4 | 6.16 | n.t. |
| 5 | 7.3 | n.t. |
| 6 | 6.62 | n.t. |
| 7 | 7.18 | n.t. |
| 8 | 7.88 | n.t. |
| 9 | 7.54 | n.t. |
| 10 | 8.95 | n.t. |
| 11+12+ 13+14 | 6.2 | n.t. |
| 11 | 6.47 | n.t. |
| 12 | <5.05 | n.t. |
| 13 | <5.05 | n.t. |
| 14 | 7.31 | n.t. |
| 15 | 8.16 | n.t. |
| 16 | 7.35 | n.t. |
| 17 | 7.84 | n.t. |
| 18 | 5.45 | n.t. |
| 19 | 6.42 | n.t. |
| 20 | 8.6 | 8.59 |
| 21 | 9.09 | n.t. |
| 22 | 8.94 | n.t. |
| 23 | 5.35 | n.t. |
| 24 | 6.33 | n.t. |
| 25 | 8.43 | n.t. |
| 26 | 8.28 | 8.39 |
| 27 | 7.76 | 7.79 |
| 28 | 6.26 | 6.3 |
| 29a | 6.07 | 6.74 |
| 29b | 6.89 | n.t. |
| 30 | 7.8 | 7.87 |
| 31 | 8.72 | 8.75 |
| 32 | 9.4 | 9.51 |
| 33 | 8.32 | 8.37 |
| 34 | 5.56 | 5.72 |
| 35 | 8.51 | 8.55 |

(continued)

| Co. No. | Cellular αLisa assay in SKNBE2 cells Abeta 42 $pIC_{50}$ | Cellular αLisa assay in SKNBE2 cells Abetatotal $pIC_{50}$ |
|---|---|---|
| 36 | 8.36 | 8.36 |
| 37 | 8.46 | n.t. |
| 38 | 8.15 | n.t. |
| 39 | 8.86 | 8.67 |
| 40 | 8.73 | n.t. |
| 41 | 8.79 | n.t. |
| 42 | 8.62 | n.t. |
| 43 | 8.35 | n.t. |
| 44 | 8.94 | n.t. |
| 45 | 8.49 | n.t. |
| 47 | 7.78 | n.t. |
| 48 | <5.05 | <5.05 |
| 48a | 7.78 | n.t. |
| 48b | 6.6 | n.t. |
| 49a | 7.77 | n.t. |
| 49b | 7.09 | n.t. |

BACE2 BIOCHEMICAL FRET BASED ASSAY

This assay is a Fluorescence Resonance Energy Transfer Assay (FRET) based assay. The substrate for this assay contains the 'Swedish' Lys-Met/Asn-Leu mutation of the amyloid precursor protein (APP) beta-secretase cleavage site. This substrate also contains two fluorophores: (7-methoxycoumarin-4-yl) acetic acid (Mca) is a fluorescent donor with excitation wavelength at 320 nm and emission at 405 nm and 2,4-Dinitrophenyl (Dnp) is a proprietary quencher acceptor. The distance between those two groups has been selected so that upon light excitation, the donor fluorescence energy is significantly quenched by the acceptor, through resonance energy transfer. Upon cleavage by the beta-secretase, the fluorophore Mca is separated from the quenching group Dnp, restoring the full fluorescence yield of the donor. The increase in fluorescence is linearly related to the rate of proteolysis.

Briefly in a 384-well format recombinant BACE2 protein in a final concentration of 0.4 |ig/ml is incubated for 450 minutes at room temperature with 10 μM substrate in incubation buffer (50 mM Citrate buffer pH 5.0, 0.05% PEG, no DMSO) in the absence or presence of compound. Next the amount of proteolysis is directly measured by fluorescence measurement at T=0 and T=450 (excitation at 320 nm and emission at 405 nm). Results are expressed in RFU (Relative Fluorescence Units), as difference between T450 and TO.

A best-fit curve is fitted by a minimum sum of squares method to the plot of %Controlmin versus compound concentration. From this an $IC_{50}$ value (inhibitory concentration causing 50% inhibition of activity) can be obtained.

LC = Median of the low control values = Low control: Reaction without enzyme

HC = Median of the High control values = High Control: Reaction with enzyme

$$\%Effect = 100-[(sample-LC) / (HC-LC) *100]$$

$$\%Control = (sample /HC)*100$$

$$\%Controlmin = (sample\text{-}LC) / (HC\text{-}LC) * 100$$

The following exemplified compounds were tested essentially as described above and exhibited the following the activity:

TABLE 9.

| Co. No. | Biochemical FRET based assay $pIC_{50}$ |
|---|---|
| 1 | <5 |
| 2 | 7.86 |
| 3 | 6.27 |
| 4 | 5.24 |
| 5 | 6.38 |
| 6 | 6.27 |
| 7 | 6.25 |
| 8 | 7.15 |
| 9 | 6.29 |
| 10 | 7.66 |
| 11+12+ 13+14 | 5.75 |
| 11 | 6.05 |
| 12 | <5 |
| 13 | <5 |
| 14 | 6.94 |
| 15 | 7.18 |
| 16 | 5.87 |
| 17 | 7.03 |
| 18 | <5 |
| 19 | 6.52 |
| 20 | 8.52 |
| 21 | 8.3 |
| 22 | 8.28 |
| 23 | 5.33 |
| 24 | 6.01 |
| 25 | 8.69 |
| 26 | 7.96 |
| 27 | 6.41 |
| 28 | <5 |
| 29a | 6.11 |
| 29b | 7.53 |
| 30 | 7.61 |
| 31 | 8.58 |
| 32 | 7.94 |

(continued)

| Co. No. | Biochemical FRET based assay $pIC_{50}$ |
|---|---|
| 33 | 8.45 |
| 34 | 5.37 |
| 35 | 7.35 |
| 36 | 7.55 |
| 37 | 7.49 |
| 38 | 8.38 |
| 39 | 7.6 |
| 40 | 8.48 |
| 41 | 7.78 |
| 42 | 7.65 |
| 43 | 7.19 |
| 44 | 8.47 |
| 45 | 7.87 |
| 47 | 8.36 |
| 48 | <5 |
| 48a | 6.35 |
| 48b | 5.14 |
| 49a | 6.49 |
| 49b | 5.72 |

PHARMACOLOGY IN THE BEAGLE DOG

Test compounds were tested to evaluate the effect on the beta-amyloid profile in cerebrospinal fluid (CSF) of dogs after a single dose, in combination with pharmacokinetic (PK) follow up and limited safety evaluation.

For each of compound 20, 21, 22 or 41, four beagle dogs (2 males, 2 females) were dosed with vehicle (1 ml/kg of an aqueous solution of 20 % cyclodextrin) and 12 beagle dogs (2 males and 2 females per dosage group) were dosed with test compounds as follows:

| Compound | Dosage |
|---|---|
| 20 | 0.16, 0.63 and 1.25 mg/kg in 0.16, 0.63 and 1.25 mg/ml of an aqueous 20 % cyclodextrin solution, on an empty stomach |
| 22 | 0.02, 0.08 and 0.31 mg/kg in 0.02, 0.08 and 0.31 mg/ml of an aqueous 20% cyclodextrin solution, on an empty stomach |
| 21 | 0.08, 0.31 and 0.63 mg/kg in 0.08, 0.31 and 0.63 mg/ml of an aqueous 20 % cyclodextrin solution, on an empty stomach |
| 41 | 0.16, 0.31 and 1.25 mg/kg in 0.16, 0.31 and 1.25 mg/ml of an aqueous 20% cyclodextrin solution, on an empty stomach |

In the case of compounds 39, 33 and 31, two beagle dogs (1 male, 1 female) were dosed with vehicle (1 ml/kg of an aqueous solution of 20 % cyclodextrin) and 4 beagle dogs (2 males and 2 females) were dosed with test compound (2, 39, 33, 31 or 32) (0.31 mg/kg in 0.31 mg/ml of an aqueous 20% cyclodextrin solution) on an empty stomach.

CSF was taken in conscious animals directly from the lateral ventricle via a cannula which was screwed in the skull and covered with subcutaneous tissue and skin, before and at 4, 8, 25 and 49 hours after dosing. Eight hours after

dosing the animals got access to their regular meal for 30 minutes. Blood was taken for PK follow up (0.5, 1, 2, 4, 8, 25 and 49 hours) and serum samples for biochemistry were taken before and at 8 and 25h after dosing. The CSF samples were used for measurement of Abeta 1-37, Abeta 1-38, Abeta 1-40 and Abeta 1-42. The results are summarized in Table 10 below:

TABLE 10.

| Co. No. | % Decrease in Abeta 1-42 at 8h post dosing compared to own baseline | % Decrease in Abeta 1-42 at 24h[(a)] or 25h[(b)] post dosing compared to own baseline | % Decrease in Abeta 1-42 at 49h post dosing compared to own baseline | Dose (mg/kg) |
|---|---|---|---|---|
| 2 | 56 | | | 0.31 |
| 20 | 54 | 44[(a)] | - | 0.16 |
| | 75 | | 29 | 0.63 |
| | 77 | | 65 | 1.25 |
| 22 | No effects observed | | | 0.02 |
| | 50 | 54[(a)] | - | 0.08 |
| | 78 | | 71 | 0.31 |
| 41 | 71 | 26[(b)] | - | 0.16 |
| | 78 | 49[(b)] | - | 0.31 |
| | 89 | | 41 | 1.25 |
| 39 | 53 | 44[(b)] | - | 0.31 |
| 21 | 49 | | | 0.08 |
| | 78 | 69[(b)] | - | 0.31 |
| | 70 | | | 0.63 |
| 32 | 76 | | | 0.31 |
| 33 | 34 | | | 0.31 |
| 31 | 31 | | | 0.31 |
| % decrease indicated at 8h and at last time point at which relevant decrease (>20% decrease) was observed. | | | | |

**Claims**

**1.** A compound of Formula (I)

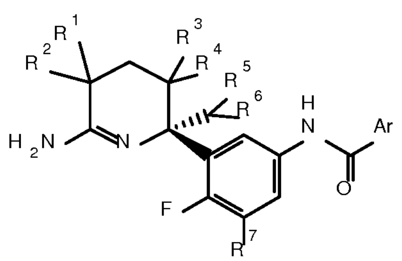

(I)

or a tautomer or a stereoisomeric form thereof, wherein

$R^1$ is selected from the group consisting of $-C_{1-3}$alkyl, $-C_{1-3}$alkyl-F and fluoro;
$R^2$ is selected from the group consisting of $-SO_2C_{1-3}$alkyl, $-SO_2$cyclopropyl, -CN, $-OC_{1-3}$alkyl, $CF_3$, and $-SO(NCH_3)CH_3$;
Ar is homoaryl or heteroaryl;

wherein homoaryl is phenyl or phenyl substituted with one, two or three substituents each independently selected from the group consisting of halo, cyano, $C_{1-3}$alkyl, cyclopropyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and $HC\equiv CCH_2O$;
heteroaryl is selected from the group consisting of pyridyl, pyrimidinyl, pyrazinyl, pyridazinyl, furanyl, thienyl, pyrrolyl, pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents each independently selected from the group consisting of halo, cyano, $C_{1-3}$alkyl, cyclopropyl, $C_{2-3}$alkynyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and $HC\equiv CCH_2O$;
$R^3$, $R^4$, $R^5$, and $R^6$ are each independently selected from the group consisting of H, fluoro, methyl and methoxy; and
$R^7$ is hydrogen or fluoro;
or a pharmaceutically acceptable addition salt or a solvate thereof.

2. The compound according to claim 1, wherein $R^2$ is $-SO_2C_{1-3}$alkyl, $-SO_2$cyclopropyl, or -CN.

3. The compound according to claim 1 or 2, wherein $>CR^3R^4$ is $>CH_2$, $>CHF$, $>CF_2$ or $>C(CH_3)F$, and $-CHR^5R^6$ is $-CH_3$, $-CH_2F$ or $-CHF_2$.

4. The compound according to any one of claims 1 to 3, having the Formula ($I^I$)

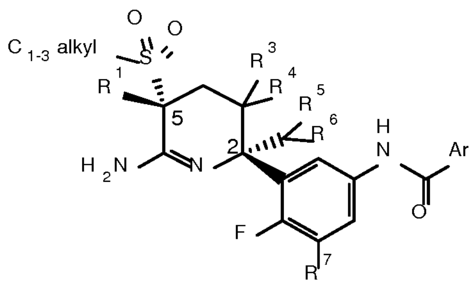

($I^I$)

wherein $R^1$, and $R^3$-$R^7$ and Ar are as defined in any one of claims 1 to 3.

5. The compound according to any one of claims 1 to 4, wherein $R^2$ is $-SO_2CH_3$, $-SO_2CH_2CH_3$, or $-SO_2CH(CH_3)_2$.

6. The compound according to any one of claims 1 to 3, having the Formula ($I^{II}$),

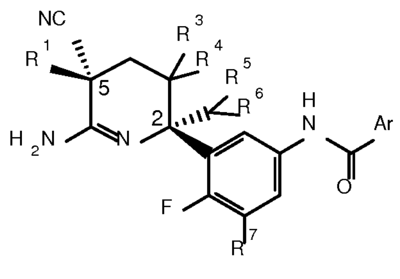

($I^{II}$)

wherein $R^1$, and $R^3$-$R^7$ and Ar are as defined in any one of claims 1 to 3.

7. The compound according to claim 1 of Formula (I) having the Formula (I-a)

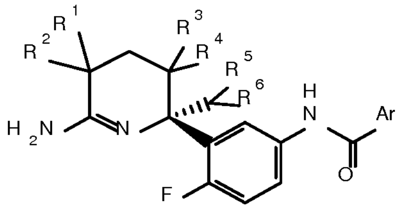

(I-a)

or a tautomer or a stereoisomeric form thereof, wherein

$R^1$ is $C_{1-2}$alkyl or fluoro;
$R^2$ is -$SO_2C_{1-3}$alkyl, -$SO_2$cyclopropyl, -CN, -$OC_{1-3}$alkyl, $CF_3$, or -$SO(NCH_3)CH_3$;
Ar is homoaryl or heteroaryl;
wherein homoaryl is phenyl or phenyl substituted with one, two or three substituents each independently selected from the group consisting of halo, cyano, $C_{1-3}$alkyl, cyclopropyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy-, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and HC≡$CCH_2O$-;
heteroaryl is selected from the group consisting of pyridyl, pyrimidinyl, pyrazinyl, pyridazinyl, furanyl, thienyl, pyrrolyl, pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents each independently selected from the group consisting of halo, cyano, $C_{1-3}$alkyl, cyclopropyl, $C_{2-3}$alkynyl, $C_{1-3}$alkyloxy, cyclopropyloxy, (cyclopropyl)$C_{1-3}$alkyloxy, monohalo-$C_{1-3}$alkyl, polyhalo-$C_{1-3}$alkyl, monohalo-cyclopropyl, polyhalo-cyclopropyl, monohalo-$C_{1-3}$alkyloxy, polyhalo-$C_{1-3}$alkyloxy, monohalo-cyclopropyloxy, polyhalo-cyclopropyloxy, ($C_{1-3}$alkyloxy)$C_{1-3}$alkyloxy, (cyclopropyloxy)$C_{1-3}$alkyloxy, and HC≡$CCH_2O$-;
$R^3$, $R^4$, $R^5$, and $R^6$ are each independently selected from H, fluoro and methyl;
or a pharmaceutically acceptable addition salt or a solvate thereof.

**8.** The compound of any one of the preceding claims, wherein $R^1$ is $CH_3$.

**9.** A pharmaceutical composition comprising a therapeutically effective amount of a compound according to any one of claims 1 to 8 and a pharmaceutically acceptable carrier.

**10.** A process for preparing a pharmaceutical composition as defined in claim 9 comprising mixing a pharmaceutically acceptable carrier with a therapeutically effective amount of a compound according to any one of claims 1 to 8.

**11.** A compound as defined in any one of claims 1 to 8 or a pharmaceutical composition as defined in claim 9 for use as a medicament.

**12.** A compound as defined in any one of claims 1 to 8 or a pharmaceutical composition as defined in claim 9 for use in the treatment or prevention of Alzheimer's disease (AD), mild cognitive impairment, senility, dementia, dementia with Lewy bodies, Down's syndrome, dementia associated with stroke, dementia associated with Parkinson's disease, or dementia associated with beta-amyloid.

**13.** Use of a compound as claimed in any one of claims 1 to 8 or a pharmaceutical composition as claimed in claim 9 for the manufacture of a medicament for treating or preventing Alzheimer's disease (AD), mild cognitive impairment, senility, dementia, dementia with Lewy bodies, Down's syndrome, dementia associated with stroke, dementia associated with Parkinson's disease, or dementia associated with beta-amyloid.

## Patentansprüche

**1.** Verbindung der Formel (I)

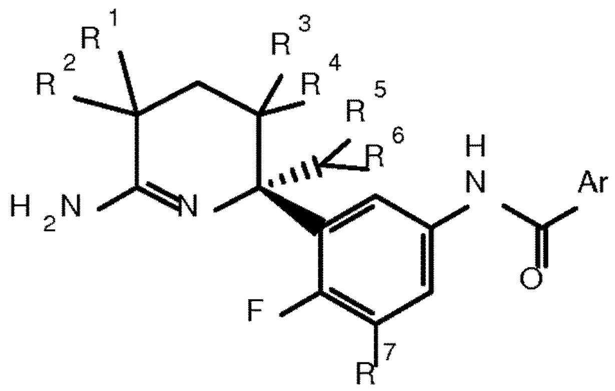

(I)

oder ein Tautomer oder eine stereoisomere Form davon, wobei

$R^1$ aus der Gruppe bestehend aus -$C_{1-3}$-Alkyl, -$C_{1-3}$-Alkyl-F und Fluor ausgewählt ist;
$R^2$ aus der Gruppe bestehend aus -$SO_2$-$C_{1-3}$-Alkyl, $SO_2$-Cyclopropyl, -CN, -O-$C_{1-3}$-Alkyl, $CF_3$ und -$SO(NCH_3)CH_3$ ausgewählt ist;
Ar für Homoaryl oder Heteroaryl steht;
wobei Homoaryl für Phenyl oder für Phenyl, das mit einem, zwei oder drei Substituenten, die jeweils unabhängig aus der Gruppe bestehend aus Halogen, Cyano, $C_{1-3}$-Alkyl, Cyclopropyl, $C_{1-3}$-Alkyloxy, Cyclopropyloxy, (Cyclopropyl) -$C_{1-3}$-alkyloxy, Monohalogen-$C_{1-3}$-alkyl, Polyhalogen-$C_{1-3}$-alkyl, Monohalogencyclopropyl, Polyhalogencyclopropyl, Monohalogen-$C_{1-3}$-alkyloxy, Polyhalogen-$C_{1-3}$-alkyloxy, Monohalogencyclopropyloxy, Polyhalogencyclopropyloxy, ($C_{1-3}$-Alkyloxy) -$C_{1-3}$-alkyloxy, (Cyclopropyloxy) -$C_{1-3}$-alkyloxy und $HC{\equiv}CCH_2O$ ausgewählt sind, substituiert ist, steht;
Heteroaryl aus der Gruppe bestehend aus Pyridyl, Pyrimidinyl, Pyrazinyl, Pyridazinyl, Furanyl, Thienyl, Pyrrolyl, Pyrazolyl, Imidazolyl, Triazolyl, Tetrazolyl, Thiazolyl, Isothiazolyl, Thiadiazolyl, Oxazolyl, Isoxazolyl und Oxadiazolyl ausgewählt ist, die jeweils gegebenenfalls mit einem, zwei oder drei Substituenten, die jeweils unabhängig aus der Gruppe bestehend aus Halogen, Cyano, $C_{1-3}$-Alkyl, Cyclopropyl, $C_{2-3}$-Alkinyl, $C_{1-3}$-Alkyloxy, Cyclopropyloxy, (Cyclopropyl) -$C_{1-3}$-alkyloxy, Monohalogen-$C_{1-3}$-alkyl, Polyhalogen-$C_{1-3}$-alkyl, Monohalogencyclopropyl, Polyhalogencyclopropyl, Monohalogen-$C_{1-3}$-alkyloxy, Polyhalogen-$C_{1-3}$-alkyloxy, Monohalogencyclopropyloxy, Polyhalogencyclopropyloxy, ($C_{1-3}$-Alkyloxy) -$C_{1-3}$-alkyloxy, (Cyclopropyloxy)-$C_{1-3}$-alkyloxy und $HC{\equiv}CCH_2O$ ausgewählt sind, substituiert ist;
$R^3$, $R^4$, $R^5$ und $R^6$ jeweils unabhängig aus der Gruppe bestehend aus H, Fluor, Methyl und Methoxy ausgewählt sind und
$R^7$ für Wasserstoff oder Fluor steht;
oder ein pharmazeutisch unbedenkliches Additionssalz oder Solvat davon.

**2.** Verbindung nach Anspruch 1, wobei $R^2$ für -$SO_2$-$C_{1-3}$-Alkyl, -$SO_2$-Cyclopropyl oder -CN steht.

**3.** Verbindung nach Anspruch 1 oder 2, wobei $>CR^3R^4$ für $>CH_2$, $>CHF$, $>CF_2$ oder $C(CH_3)F$ steht und $-CHR^5R^6$ für - $CH_3$, -$CH_2F$ oder -$CHF_2$ steht.

**4.** Verbindung nach einem der Ansprüche 1 bis 3 mit der Formel ($I^I$)

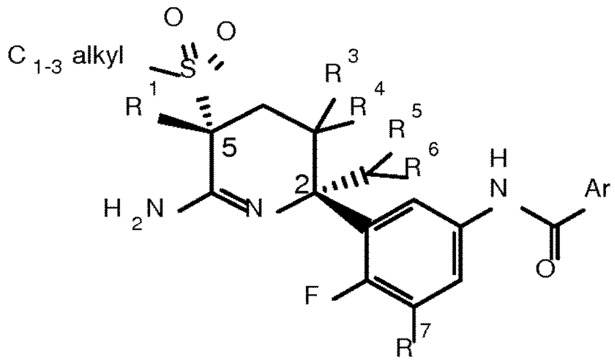

$(I^{I})$

wobei $R^1$, $R^3$-$R^7$ und Ar wie in einem der Ansprüche 1 bis 3 definiert sind.

**5.** Verbindung nach einem der Ansprüche 1 bis 4, wobei $R^2$ für $-SO_2CH_3$, $-SO_2CH_2CH_3$ oder $-SO_2CH(CH_3)_2$ steht.

**6.** Verbindung nach einem der Ansprüche 1 bis 3 mit der Formel $(I^{II})$

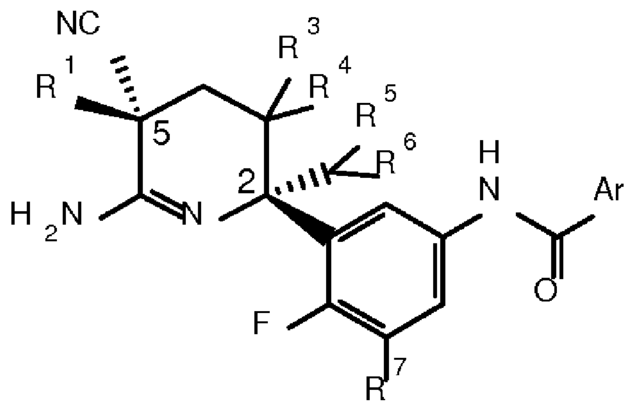

$(I^{II})$

wobei $R^1$, $R^3$-$R^7$ und Ar wie in einem der Ansprüche 1 bis 3 definiert sind.

**7.** Verbindung der Formel (I) nach Anspruch 1 mit der Formel (I-a)

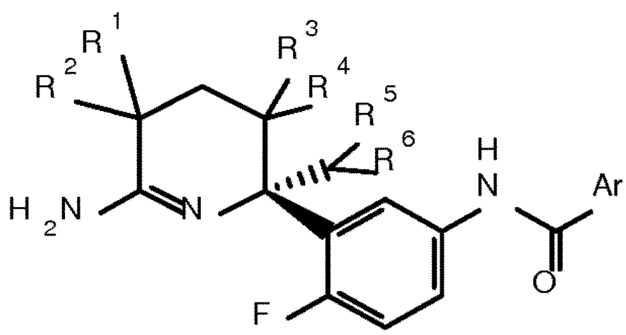

(I-a)

oder ein Tautomer oder eine stereoisomere Form davon, wobei

$R^1$ für $C_{1-2}$-Alkyl oder Fluor steht;

$R^2$ für $-SO_2-C_{1-3}$-Alkyl, $SO_2$-Cyclopropyl, -CN, $-O-C_{1-3}$-Alkyl, $CF_3$ oder $-SO(NCH_3)CH_3$ steht;
Ar für Homoaryl oder Heteroaryl steht;
wobei Homoaryl für Phenyl oder für Phenyl, das mit einem, zwei oder drei Substituenten, die jeweils unabhängig aus der Gruppe bestehend aus Halogen, Cyano, $C_{1-3}$-Alkyl, Cyclopropyl, $C_{1-3}$-Alkyloxy, Cyclopropyloxy, (Cyclopropyl) $-C_{1-3}$-alkyloxy, Monohalogen-$C_{1-3}$-alkyl, Polyhalogen-$C_{1-3}$-alkyl, Monohalogencyclopropyl, Polyhalogencyclopropyl, Monohalogen-$C_{1-3}$-alkyloxy, Polyhalogen-$C_{1-3}$-alkyloxy, Monohalogencyclopropyloxy, Polyhalogencyclopropyloxy, ($C_{1-3}$-Alkyloxy) $-C_{1-3}$-alkyloxy, (Cyclopropyloxy) $-C_{1-3}$-alkyloxy und $HC{\equiv}CCH_2O$- ausgewählt sind, substituiert ist, steht;
Heteroaryl aus der Gruppe bestehend aus Pyridyl, Pyrimidinyl, Pyrazinyl, Pyridazinyl, Furanyl, Thienyl, Pyrrolyl, Pyrazolyl, Imidazolyl, Triazolyl, Tetrazolyl, Thiazolyl, Isothiazolyl, Thiadiazolyl, Oxazolyl, Isoxazolyl und Oxadiazolyl ausgewählt ist, die jeweils gegebenenfalls mit einem, zwei oder drei Substituenten, die jeweils unabhängig aus der Gruppe bestehend aus Halogen, Cyano, $C_{1-3}$-Alkyl, Cyclopropyl, $C_{2-3}$-Alkinyl, $C_{1-3}$-Alkyloxy, Cyclopropyloxy, (Cyclopropyl) $-C_{1-3}$-alkyloxy, Monohalogen-$C_{1-3}$-alkyl, Polyhalogen-$C_{1-3}$-alkyl, Monohalogencyclopropyl, Polyhalogencyclopropyl, Monohalogen-$C_{1-3}$-alkyloxy, Polyhalogen-$C_{1-3}$-alkyloxy, Monohalogencyclopropyloxy, Polyhalogencyclopropyloxy, ($C_{1-3}$-Alkyloxy) $-C_{1-3}$-alkyloxy, (Cyclopropyloxy) $-C_{1-3}$-alkyloxy und $HC{\equiv}CCH_2O$ ausgewählt sind, substituiert ist;
$R^3$, $R^4$, $R^5$ und $R^6$ jeweils unabhängig aus H, Fluor und Methyl ausgewählt sind;
oder ein pharmazeutisch unbedenkliches Additionssalz oder Solvat davon.

**8.** Verbindung nach einem der vorhergehenden Ansprüche, wobei $R^1$ für $CH_3$ steht.

**9.** Pharmazeutische Zusammensetzung, die eine therapeutisch wirksame Menge einer Verbindung nach einem der Ansprüche 1 bis 8 und einen pharmazeutisch unbedenklichen Träger umfasst.

**10.** Verfahren zur Herstellung einer pharmazeutischen Zusammensetzung nach Anspruch 9, bei dem man einen pharmazeutisch unbedenklichen Träger mit einer therapeutisch wirksamen Menge einer Verbindung nach einem der Ansprüche 1 bis 8 mischt.

**11.** Verbindung nach einem der Ansprüche 1 bis 8 oder pharmazeutische Zusammensetzung nach Anspruch 9 zur Verwendung als Medikament.

**12.** Verbindung nach einem der Ansprüche 1 bis 8 oder pharmazeutische Zusammensetzung nach Anspruch 9 zur Verwendung bei der Behandlung oder Prävention von Alzheimer-Krankheit (Alzheimer's Disease, AD), leichter kognitiver Störung, Senilität, Demenz, Demenz mit Lewy-Körperchen, Down-Syndrom, mit Schlaganfall assoziierter Demenz, mit Parkinson-Krankheit assoziierter Demenz oder mit beta-Amyloid assoziierter Demenz.

**13.** Verwendung einer Verbindung nach einem der Ansprüche 1 bis 8 oder einer pharmazeutischen Zusammensetzung nach Anspruch 9 zur Herstellung eines Medikaments zur Behandlung oder Prävention von Alzheimer-Krankheit (Alzheimer's Disease, AD), leichter kognitiver Störung, Senilität, Demenz, Demenz mit Lewy-Körperchen, Down-Syndrom, mit Schlaganfall assoziierter Demenz, mit Parkinson-Krankheit assoziierter Demenz oder mit beta-Amyloid assoziierter Demenz.

## Revendications

**1.** Composé de formule (I)

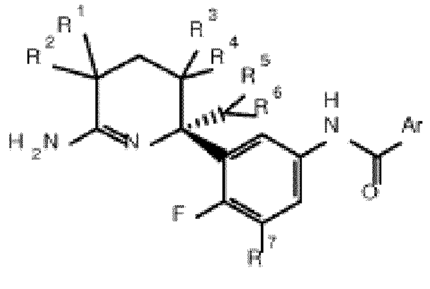

(I)

ou forme tautomère ou forme stéréoisomérique correspondante,

$R^1$ étant choisi dans le groupe constitué par $-C_{1-3}$alkyle, $-C_{1-3}$alkyl-F et fluoro ;
$R^2$ étant choisi dans le groupe constitué par $-SO_2C_{1-3}$alkyle, $-SO_2$cyclopropyle, -CN, $-OC_{1-3}$alkyle, $CF_3$, et $-SO(NCH_3)CH_3$ ;
Ar étant homoaryle ou hétéroaryle ;
homoaryle étant phényle ou phényle substitué par un, deux ou trois substituants, chacun indépendamment choisi dans le groupe constitué par halogéno, cyano, $C_{1-3}$alkyle, cyclopropyle, $C_{1-3}$alkyloxy, cyclopropyloxy, (cyclopropyl) $C_{1-3}$alkyloxy, monohalogéno-$C_{1-3}$alkyle, polyhalogéno-$C_{1-3}$alkyle, monohalogéno-cyclopropyle, polyhalogéno-cyclopropyle, monohalogéno-$C_{1-3}$alkyloxy, polyhalogéno-$C_{1-3}$alkyloxy, monohalogéno-cyclopropyloxy, polyhalogéno-cyclopropyloxy, ($C_{1-3}$alkyloxy) $C_{1-3}$alkyloxy, (cyclopropyloxy) $C_{1-3}$alkyloxy, et $HC{\equiv}CCH_2O$ ;
hétéroaryle étant choisi dans le groupe constitué par pyridinyle, pyrimidinyle, pyrazinyle, pyridazinyle, furannyle, thiényle, pyrrolyle, pyrazolyle, imidazolyle, triazolyle, tétrazolyle, thiazolyle, isothiazolyle, thiadiazolyle, oxazolyle, isoxazolyle, et oxadiazolyle, chacun éventuellement substitué par un, deux ou trois substituants chacun indépendamment choisi dans le groupe constitué par halogéno, cyano, $C_{1-3}$alkyle, cyclopropyle, $C_{2-3}$alcynyle, $C_{1-3}$alkyloxy, cyclopropyloxy, (cyclopropyl)$C_{1-3}$alkyloxy, monohalogéno-$C_{1-3}$alkyle, polyhalogéno-$C_{1-3}$alkyle, monohalogéno-cyclopropyle, polyhalogéno-cyclopropyle,
monohalogéno-$C_{1-3}$alkyloxy, polyhalogéno-$C_{1-3}$alkyloxy, monohalogéno-cyclopropyloxy, polyhalogéno-cyclopropyloxy, ($C_{1-3}$alkyloxy) $C_{1-3}$alkyloxy, (cyclopropyloxy) $C_{1-3}$alkyloxy, et $HC{\equiv}CCH_2O$ ;
$R^3$, $R^4$, $R^5$, et $R^6$ étant chacun indépendamment choisi dans le groupe constitué par H, fluoro, méthyle et méthoxy ; et
$R^7$ étant hydrogène ou fluoro ;
ou sel d'addition pharmaceutiquement acceptable ou solvate correspondant.

**2.** Composé selon la revendication 1, $R^2$ étant $-SO_2C_{1-3}$alkyle, $-SO_2$cyclopropyle, ou -CN.

**3.** Composé selon la revendication 1 ou 2, $>CR^3R^4$ étant $>CH_2$, $>CHF$, $>CF_2$ ou $>C(CH_3)F$, et $-CHR^5R^6$ étant $-CH_3$, $-CH_2F$ ou $-CHF_2$.

**4.** Composé selon l'une quelconque des revendications 1 à 3, possédant la formule ($I^I$)

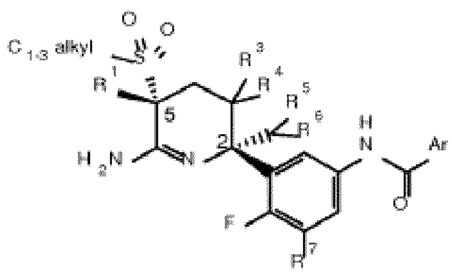

($I^I$)

$R^1$, et $R^3$ à $R^7$ et Ar étant tels que définis selon l'une quelconque des revendications 1 à 3.

**5.** Composé selon l'une quelconque des revendications 1 à 4, $R^2$ étant $-SO_2CH_3$, $-SO_2CH_2CH_3$ ou $-SO_2CH(CH_3)_2$.

**6.** Composé selon l'une quelconque des revendications 1 à 3, possédant la formule ($I^{II}$),

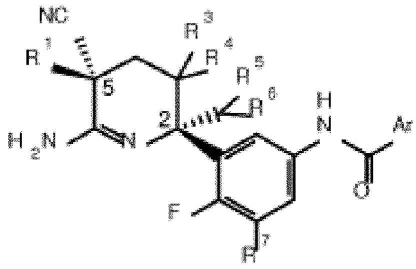

($I^{II}$)

$R^1$, et $R^3$ à $R^7$ et Ar étant tels que définis selon l'une quelconque des revendications 1 à 3.

**7.** Composé selon la revendication 1 de formule (I) possédant la formule (I-a)

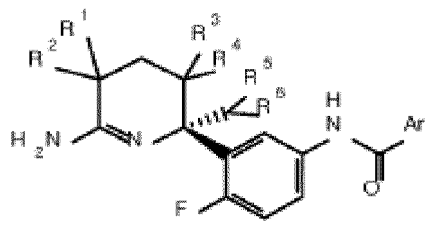

(I-a)

ou forme tautomère ou forme stéréoisomérique correspondante,

$R^1$ étant $-C_{1-2}$alkyle ou fluoro ;
$R^2$ étant $-SO_2C_{1-3}$alkyle, $-SO_2$cyclopropyle, -CN, $-OC_{1-3}$alkyle, $CF_3$, ou $-SO(NCH_3)CH_3$;
Ar étant homoaryle ou hétéroaryle ;
homoaryle étant phényle ou phényle substitué par un, deux ou trois substituants, chacun indépendamment choisi dans le groupe constitué par halogéno, cyano, $C_{1-3}$alkyle, cyclopropyle, $C_{1-3}$alkyloxy, cyclopropyloxy, (cyclopropyl) $C_1$-$_3$alkyloxy, monohalogéno-$C_{1-3}$alkyle, polyhalogéno-$C_{1-3}$alkyle, monohalogéno-cyclopropyle, polyhalogéno-cyclopropyle, monohalogéno-$C_{1-3}$alkyloxy, polyhalogéno-$C_{1-3}$alkyloxy, monohalogéno-cyclopropyloxy, polyhalogéno-cyclopropyloxy, ($C_{1-3}$alkyloxy) $C_{1-3}$alkyloxy, (cyclopropyloxy) $C_{1-3}$alkyloxy, et $HC{\equiv}CCH_2O$ ;
hétéroaryle étant choisi dans le groupe constitué par pyridinyle, pyrimidinyle, pyrazinyle, pyridazinyle, furannyle, thiényle, pyrrolyle, pyrazolyle, imidazolyle, triazolyle, tétrazolyle, thiazolyle, isothiazolyle, thiadiazolyle, oxazolyle, isoxazolyle, et oxadiazolyle, chacun éventuellement substitué par un, deux ou trois substituants, chacun indépendamment choisi dans le groupe constitué par halogéno, cyano, $C_{1-3}$alkyle, cyclopropyle, $C_{2-3}$alcynyle, $C_{1-3}$alkyloxy, cyclopropyloxy, (cyclopropyl)$C_{1-3}$alkyloxy, monohalogéno-$C_{1-3}$alkyle, polyhalogéno-$C_{1-3}$alkyle, monohalogéno-cyclopropyle, polyhalogéno-cyclopropyle, monohalogéno-$C_{1-3}$alkyloxy, polyhalogéno-$C_{1-3}$alkyloxy, monohalogéno-cyclopropyloxy, polyhalogéno-cyclopropyloxy, ($C_{1-3}$alkyloxy) $C_{1-3}$alkyloxy, (cyclopropyloxy) $C_{1-3}$alkyloxy, et $HC{\equiv}CCH_2O$- ;
$R^3$, $R^4$, $R^5$, et $R^6$ étant chacun indépendamment choisi parmi H, fluoro et méthyle ;
ou sel d'addition pharmaceutiquement acceptable ou solvate correspondant.

**8.** Composé selon l'une quelconque des revendications précédentes, $R^1$ étant $CH_3$.

**9.** Composition pharmaceutique comprenant une quantité thérapeutiquement efficace d'un composé selon l'une quelconque des revendications 1 à 8 et un support pharmaceutiquement acceptable.

**10.** Procédé pour la préparation d'une composition pharmaceutique telle que définie dans la revendication 9 comprenant le mélange d'un support pharmaceutiquement acceptable avec une quantité thérapeutiquement efficace d'un composé selon l'une quelconque des revendications 1 à 8.

**11.** Composé selon l'une quelconque des revendications 1 à 8 ou composition pharmaceutique telle que définie dans la revendication 9 pour une utilisation en tant que médicament.

**12.** Composé tel que défini selon l'une quelconque des revendications 1 à 8 ou composition pharmaceutique telle que définie dans la revendication 9 pour une utilisation dans le traitement ou la prévention de la maladie d'Alzheimer (AD), d'une déficience cognitive légère, de la sénilité, de la démence, de la démence avec des corps de Lewy, du syndrome de Down, de la démence associée à un accident vasculaire cérébrale, de la démence associée à la maladie de Parkinson, ou de la démence associée à la bêta-amyloïde.

**13.** Utilisation d'un composé selon l'une quelconque des revendications 1 à 8 ou d'une composition pharmaceutique telle que définie dans la revendication 9 pour la fabrication d'un médicament pour le traitement ou la prévention de la maladie d'Alzheimer (AD), d'une déficience cognitive légère, de la sénilité, de la démence, de la démence avec des corps de Lewy, du syndrome de Down, de la démence associée à un accident vasculaire cérébrale, de la démence associée à la maladie de Parkinson, ou de la démence associée à la bêta-amyloïde.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015124576 A
- WO 2011009943 A
- WO 2014059185 A
- WO 2014134341 A1
- WO 2011138293 A1
- US 20150232449 A
- WO 2012110459 A
- WO 2012057247 A
- WO 2012156284 A


### Non-patent literature cited in the description


- *CHEMICAL ABSTRACTS,* 1892-57-5
- Diagnostic & Statistical Manual of Mental Disorders (DSM-5™). American Psychiatric Association
- *CHEMICAL ABSTRACTS,* 445-27-2
- *CHEMICAL ABSTRACTS,* 1393802-40-8
- *CHEMICAL ABSTRACTS,* 1312718-98-1
- *CHEMICAL ABSTRACTS,* 1262858-99-0
- *CHEMICAL ABSTRACTS,* 1194044-45-5
- *CHEMICAL ABSTRACTS,* 1374001-20-3
- *CHEMICAL ABSTRACTS,* 1402412-96-7